(19) Europäisches Patentamt
European Patent Office
Office européen des brevets

(11) **EP 3 035 846 B1**

(12) **EUROPEAN PATENT SPECIFICATION**

(45) Date of publication and mention
of the grant of the patent:
**06.11.2019 Bulletin 2019/45**

(21) Application number: **13817949.4**

(22) Date of filing: **23.12.2013**

(51) Int Cl.:
*A61B 5/053* [(2006.01)]    *A61B 5/107* [(2006.01)]

(86) International application number:
**PCT/GB2013/053412**

(87) International publication number:
**WO 2015/025113 (26.02.2015 Gazette 2015/08)**

(54) **APPARATUS AND METHOD FOR ESTIMATING SHAPE**

KANALSCHÄTZUNGSVORRICHTUNG UND -VERFAHREN

APPAREIL ET PROCÉDÉ POUR ESTIMER UNE FORME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2013 US 201361867904 P**

(43) Date of publication of application:
**29.06.2016 Bulletin 2016/26**

(73) Proprietors:
• **Middlesex University Higher Education Corporation**
**London NW4 4BT (GB)**
• **Demosthenous, Andreas**
**Berkhamstead, Herts HP4 3JJ (GB)**

(72) Inventors:
• **KHOR, Joo Moy**
**Bow**
**London E3 3EU (GB)**
• **BAYFORD, Richard**
**Sutton, Surrey SM2 5LG (GB)**
• **TIZZARD, Gary Andrew Lionel**
**Ilford, Essex, IG3 9SH (GB)**

• **DEMOSTHENOUS, Andreas**
**Berkhamstead, Herts, HP4 3JJ (GB)**

(74) Representative: **Lucas, Phillip Brian**
**Lucas & Co.**
**135 Westhall Road**
**Warlingham, Surrey CR6 9HJ (GB)**

(56) References cited:
**EP-A1- 1 974 663    WO-A2-2012/168836**

• **DATABASE INSPEC [Online] THE INSTITUTION OF ELECTRICAL ENGINEERS, STEVENAGE, GB; 2009, KHOR J M ET AL: "Development of a Sensor Network for Dynamic Boundary Measurement in Neonatal Electrical Impedance Tomography (EIT)", XP002722742, Database accession no. 11271536 & WORLD CONGRESS ON MEDICAL PHYSICS AND BIOMEDICAL ENGINEERING 7-12 SEPT. 2009 MUNICH, GERMANY, 12 September 2009 (2009-09-12), pages 386-389, World Congress on Medical Physics and Biomedical Engineering Springer Verlag Berlin, Germany DOI: 10.1007/978-3-642-03879-2_110 ISBN: 978-3-642-03878-5**

Description

CROSS REFERENCE TO RELATED APPLICATIONS

This application claims priority to U.S. provisional patent application serial no. 61/867,904 filed August 20th, 2013.

FIELD OF THE INVENTION

This invention relates to an apparatus for use in estimating the shape of a body part of a subject; to a method of generating an estimated shape of a body part using the apparatus; to a method of generating an EIT image of a body part; and to various methods of manufacturing an apparatus. The invention is of particular value, but not exclusively, for the monitoring of lung-gestation in preterm neonates. However the principles are transferable and applicable to a range of patient types where continuous monitoring of lung function would be of clinical importance. Examples would be in any intensive care or ambulatory environment where particular conditions arising from injury or artificial ventilation would require lung monitoring, such as oedema and over-distension. The invention also finds use in the fields of EIT imaging of the breast, brain and limbs.

BACKGROUND TO THE INVENTION

Biomedical Electrical Impedance Tomography (hereinafter 'EIT') is a non-invasive medical imaging technique in which an image of the conductivity or permittivity of an interior part of the human or animal body is inferred from electrical measurements made by electrodes placed on the skin. In humans, EIT may be used on all patient groups, but has particular promise for those patient groups where other medical imaging techniques (e.g. X-ray, CT scan, MRI scan) are not ethical and/or not possible. One example of the latter is pre-term or full term neonates.

EIT relies upon determination of biological tissue transfer impedance; measurement of the transfer impedance requires placement of electrodes in direct contact with tissues or skin, which enables the injection of a known current from which the resultant voltage can be measured, or vice-versa. Current injection and resultant voltage measurement can be performed through a variety of methods, the most common of which are the two-electrode (bipolar) and four-electrode (tetrapolar) methods. The bipolar method uses the same pair of electrodes to apply current and to measure the resulting voltage. As both current injection and voltage measurement are carried out on the same pair of electrodes, the impedance measured includes not only tissue impedance but also electrode impedance. This can cause inaccuracy if calibration is not performed to find the electrode impedance beforehand. In the tetrapolar method an outer pair of electrodes is used for current injection and an inner pair of electrodes is used to measure voltage. In this way the electrode impedance is eliminated, giving more accurate results. A short introduction to bioimpedance can be found in Holder 2005a to which reference is specifically made in this respect.

Different tissue types exhibit different electrical properties (conductivity, resistivity, permittivity, capacitive and impedance) depending on their dimensions, water content, ion concentration, and tissue structure. Measuring bioimpedance can therefore be used to monitor physiological processes, tissue abnormality, tissue characterisation, and can be used to generate a series of images of tissue impedance contrast (Grimnes & Martinsen 2000; Bayford 2006). The latter can be achieved through taking sets of measurements using a large number of electrodes over a range of frequencies. The images can either be cross-sectional spatial images or a three-dimensional model. The spatial resolution of the images produced is dependent upon the number of measurements taken and the type of electrodes used.

The various methods of generating images based on measurement of tissue electrical properties, such as tissue impedance distribution contrast, are known by different terms: bio-impedance imaging, resistance imaging, impedance tomography, applied potential tomography (APT) and electrical impedance tomography (EIT), the latter including both single-frequency and multi-frequency EIT. EIT is the most commonly used terminology today in the literature and in practice, and will be used throughout this document.

The basic principle of EIT is the mapping of interior impedivity/resistivity distribution of a body region by injecting a small current and measuring the resultant voltage, or vice-versa, through one or more arrays of electrodes attached equidistantly around the surface of the subject. One known method (sometimes called the 'neighbouring' method) for obtaining an impedance image is by injecting a small known current to an adjacent pair of electrodes and measuring the voltage differences generated between other adjacent pairs of electrodes all the way around the subject. A.C. current is used instead of D.C. current because the latter would cause the polarization of ions within the tissues and hence may cause burns on the skin. Typically, the injected A.C. current is very small: international standards limit A.C. current to below 0.1mA rms and 10mA rms for frequencies up to 1 kHz and above 100 kHz respectively, to ensure the safety of subject. Once all possible measurements have been take for the first pair of current injection electrodes, current is injected at the next adjacent pair of electrodes around the subject. Voltage differences are then taken for each adjacent pair of electrodes around the subject. This process is repeated until current has been injected through every adjacent pairs of electrodes. Other methods are known, including the 'opposite' method (see for example (Baysal & Eyüboğlu 2000; Cherepenin *et al.* 2002; Otten *et al.* 2004; Zlochiver *et al.* 2004; Soleimani 2006; McEwan *et al.* 2007; Zlochiver *et al.* 2007; Nissinen *et al.* 2009) and the 'adaptive' method (see for example(Gisser *et al.* 1988; Breckon & Pidcock 1988; Riu, Rosell & Pallás-Areny 1992; Zhu *et al.* 1994; Isaacson *et al.* 1999; Borsic *et al.* 2001; Babaeizadeh *et al.* 2004).

Whichever method is used, a set of current and voltage data is produced which represents the transfer impedance of the subject. This data is processed in a computer-implemented image reconstruction algorithm to estimate the interior impedance of each of a set of small volumes, or 'voxels', inside the subject. This is an inverse problem that is both non-linear and ill-posed.

In order to solve the inverse problem, two approaches have been applied: analytical methods which solve the inverse problem directly, and numerical methods which require iterative solutions of a forward problem. The difficulty with analytical methods is they are only practical when very simple assumptions are made, for example that the cross section of the subject is a circle or ellipse.

In the numerical methods a forward model is used to simulate the subject, electrodes, current distribution, etc. The forward model is solved by assuming certain boundary conditions and a certain resistivity distribution, in order to determine expected voltage differences to be measured between electrode pairs. These are compared with the original measured voltage data. The resistivity distribution is adjusted, and the procedure repeated until the error between the calculated and recorded voltages is minimised to an acceptable level.

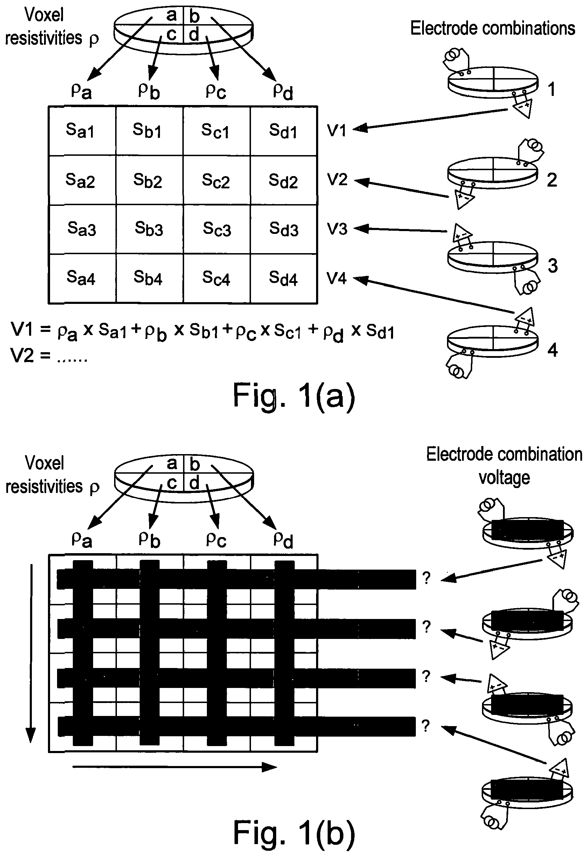

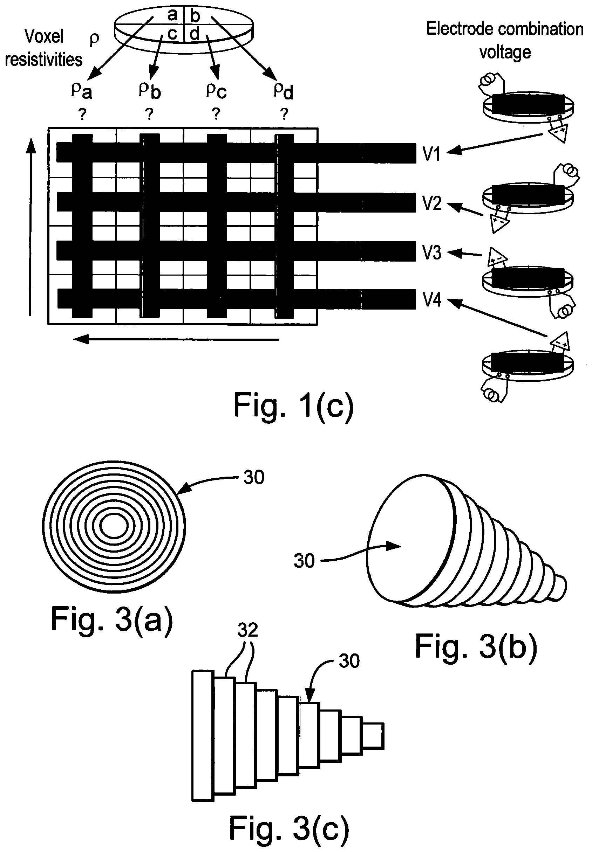

Referring to Fig. 1 the forward model assumes that the volume of the subject surrounded by the electrodes is divided into a number of voxels 6, 7, 8, 9 (in practice there may be tens of thousands of such voxels). A sensitivity matrix 1 relates resistivity to measured voltage: one column 2 of the matrix represents the resistivity of one voxel in the subject, and each row 4 represents the voltage difference measured between a particular pair of electrodes (in practice there is likely to be several hundred rows). The total voltage between each particular pair of electrodes is the sum of the resistivity in each voxel 6, 7, 8, 9 in any particular row weighted by a factor S for each voxel. The factor S indicates how much effect any particular voxel has on the total voltage measured between any pair of electrodes. In the forward model, the resistivity and factor S of each voxel is estimated in advance. From this, it is comparatively straightforward to calculate the total voltage difference measured between each electrode pair (see the formula in Fig. 1 (a) and Fig. 1 (b)).

However in reality, the problem is the reversed: voltage differences are measured and the factors S are estimated in the forward model (see Fig. 1 (c)), but the resistivity is unknown. Therefore to determine the resistivity the forward solution is reversed by inverting the sensitivity matrix. This is theoretically possible using certain assumptions, e.g. linearity between resistivity and the effect on measured voltage, that the shape of the subject is 'simple' (such as a circle or ellipse), and that there is the same number of voxels and electrode combinations. In reality none of these assumptions is true. For example, if the body part is the human thorax, its cross sectional shape does not approximate very well to a circle or an ellipse. Furthermore, many voxels will contribute a very small or zero voltage; dividing a voltage value by a small or zero S value can generate instabilities in the solution.

In order to address these problems the sensitivity matrix is 'regularized'. The process of regularization involves using constraints and *a priori* information such as anatomical data, resistivity distribution assumptions, etc. in order to obtain a stable solution. In the field EIT, known regularization techniques include TSVD (Bayford *et al.* 2008); Tikhonov (Adler *et al.* 2009; Vauhkonen *et al.* 1998); or a combination of different methods - for example Kao *et al.* (2006) combined the Tikhonov regularization and the NOSER method in a single algorithm to produce satisfactory images detecting breast cancer using EIT. Other regularization methods e.g. using total variation (Borsic 2002) have also been suggested.

As mentioned above, one of the boundary conditions in the forward model is the shape of the surface, which determines the electrode positions. It is important to have an accurate subject-specific shape model and to know the electrode locations on the shape model. That way, when the solution provided by the forward model is reversed, the resistivity distribution of the forward model will more closely reflect the real resistivity distribution in the subject. Therefore, images generated from the resistivity distribution will be more accurate.

The effects of inaccurate boundary shape and electrode positions on reconstructed EIT images have been studied in the literature and were found to be significant, even at the very early stages of EIT development. In their study of the possibilities and problems of real-time EIT imaging, Brown and Barber (1988) demonstrated the importance of accurate body shape in determining potential gradient profiles. They used a finite element model to determine the surface potential profiles in two scenarios: a circular and ellipsoidal homogeneous medium. A 16-electrode system was used and they showed that the potential gradient profiles were greatly affected by the cross-sectional shape of the boundary (Brown & Barber 1988).

Barber and Brown have also shown that error due to assumed boundary shape and electrode positions is interconnected. The images produced from their study have indirectly shown that high accuracy was required for the boundary shape and electrode position. The average error calculated from their results showed that for a body circumference of 100 mm, an electrode positioning error of less than 3.3 mm was required for a useful image (Barber & Brown 1988).

In another study, Breckon & Pidcock (1988) performed a simple analytical calculation on the effect of boundary shape and electrode position error and found that in certain cases, an approximately linear relationship exists between the error of boundary shape/electrode positions and the voltage error.

Kolehmainen (2008) demonstrated the significant effect of inexact forward model geometry and contact impedances on the reconstructed images through numerical and experimental EIT data. Bayford (2008) showed the importance of boundary shape information in the context of neonatal EIT lung imaging, as the accuracy of voltage profiles is strongly dependent on the conformance of the forward model geometry with that of the subject undergoing monitoring. Apart from the research on the importance of accurate boundary model for the thorax, Bayford's group (Bayford *et al.* 2001; Tizzard *et al.* 2005; Tizzard & Bayford 2007; Bagshaw *et al.* 2003) has carried out studies related to the importance of accurate boundary models for the human head in EIT.

In a recent study, Grychtol *et al.* (2012) quantified the errors resulting from boundary shape mismatch between the actual body shapes (human and healthy pig) and a series of progressively more inaccurate FEM models (described as a function of number of Fourier coefficients retained) for a number of EIT reconstruction algorithms using real and simulated data. They found that shape mismatch (area of symmetric difference or non-overlapping area between actual shape and shape model) of greater than 4% showed significant errors on images generated from all tested reconstruction algorithms (GREIT, NOSER, TSVD and Laplace). Hence, the study recommended that: "The EIT data be reconstructed on models closely resembling the actual shape of the body and reflecting the true positions of the electrodes. "

Various attempts have been made to obtain a more accurate boundary shape of the subject for use in the forward model. The known methods are summarised in the table below:

| | | | Research papers | Boundary Measurement/ compensation methods | Problem(s) |
|---|---|---|---|---|---|
| (Cuardo *et al.* 1990) | Produced a rigid electrode frame to achieve accurate electrode placement and object geometry | This method is not practical for neonatal EIT applications in the ICU environment and settings. | | | |
| (Kiber 1991) | Estimation of boundary shape and electrode positions through voltage gradients when known currents injected through the electrodes. An algorithm estimates dipole distances from the voltage data and from the set of dipole distances and dipole positions, shape can be estimated using iterative algorithms. | Accuracy of dipole distances dependent on the accuracy of voltage data set measured from the electrodes which is subject to movement artefact and noise | (Brown & Barber 1988) | Possibility of determining body shape from surface potential profiles | Accuracy of surface potential profile dependent on limitation of hardware |

(continued)

| (Cuardo *et al.* 1990) | Produced a rigid electrode frame to achieve accurate electrode placement and object geometry | This method is not practical for neonatal EIT applications in the ICU environment and settings. | Research papers | Boundary Measurement/ compensation methods | Problem(s) |
|---|---|---|---|---|---|
| (Ider *et al.* 1992) | Suggested that by inserting object into a water-filled cylinder with the electrodes fixed at known positions, boundary can be determined. | This method is not practical for neonatal EIT applications in the ICU environment and settings. | (Breckon & Pidcock 1988) | Suggested that by approximating the boundary by a Fourier series, the boundary shape can be determined through voltage measurement | Involves a non-linear inverse problem. They have also suggested that this can be combined with an electromechanical or optical method, but no specifics were presented for measuring the boundary shape in this way. The shape was determined from boundary data, which is still subject to measurement errors. |
| (Seydnejad & Fahimi 1994) | Suggested a computer electronic system using a circle with a sliding linear optical encoder and photo optical diodes placed at the electrode to calculate the distance between the body and circle hence provide information of body perimeter and location of electrode. | This method is not practical for neonatal EIT applications in the ICU environment and settings. | | | |

The known methods for measuring boundary shape of the subject can be categorised as numerical, mechanical or computational. However these known methods are either too complicated mathematically, or not practical for the clinical environment.

In the clinical environment, many patients who might benefit from EIT monitoring, for example monitoring of lung function, may not be able to manoeuvre themselves into an upright sitting position to enable individual electrodes to be placed around the thorax. Furthermore some patients may not be able to remain sitting upright and relatively still during EIT monitoring. One example of such a patient group is pre-term (or premature) neonates. Patients in this group are invariably in a Neonatal Intensive Care Unit (NICU), and are often in an incubator. Accordingly access to the patient is constricted, and the patient is lying down almost all of the time.

Babies born at less than 37 gestational weeks are deemed as premature or preterm (Division 2007). According to the Office of National Statistic (ONS), more than 7% of births in the UK are premature each year (UK Office of National Statistics 2012). These infants often suffer immaturity related medical complications, especially for those extremely preterm at less than 28 weeks gestational ages. Lung diseases due to respiratory system immaturity are the most prominent (Fraser et al. 2004). Of the many medical problems experienced by these preterm infants, lung diseases such as Respiratory Distress Syndrome (RDS), Chronic Lung Disease of Infancy (CLDI) and apnoea are the most common sources of morbidity and mortality which usually require mechanical ventilation at birth.

RDS is a clinical term for surfactant deficiency in an infant's lungs due to lack of mature type II pneumocyte cells, which are responsible for the generation and secretion of alveolar surfactant (Agrons et al. 2005). Since the surfactant is only produced after about 30-32 weeks of gestation, neonates born before that time are at higher risk of developing RDS. Alveolar surfactant plays an important role in reducing alveolar tension and helping the alveoli open. A deficiency can cause collapse of the alveoli leading to a decrease in functional residual capacity and an increase in dead space. If not treated immediately, it will lead to respiratory insufficiency and failure frequently resulting in fatality of the premature infant. Mechanical ventilation and treatment such as surfactant replacement are often required for premature neonates diagnosed with RDS (Fraser et al. 2004; Agrons et al. 2005). However, it is well known that prolonged mechanical ventilation to the inflexible and non-compliant lungs, if not monitored properly can increase the risk of short and long term acute respiratory complications.

CLDI commonly known as bronchopulmonary dysplasia (BPD), is a form of a long term respiratory disorder that develops as a result of lung injury due to oxygen toxicity by prolonged mechanical ventilation and oxygen administration in severe RDS (Fraser, Walls & McGuire 2004). The preterm lung is not fully developed and therefore vulnerable to injury and infection.

Apnoea is another type of respiratory complication resulting from difficulty in controlling breathing activity or upper airway obstruction at the larynx and pharynx (Ruggins & Milner 1991). This condition causes the preterm infant to stop breathing temporarily (Committee on Understanding Premature Birth and Assuring Healthy Outcomes 2007), hence potentially leading to a sudden death. Preterm infants with apnoea problem require constant monitoring and care, such as repositioning of the body. Mechanical ventilation such as nasal positive airway pressure is required in some cases (Committee on Understanding Premature Birth and Assuring Healthy Outcomes 2007).

As described above, respiratory disorders arising from premature birth commonly requires mechanical ventilation and constant monitoring. The development of an imaging system to help ensure the lungs of premature infants are properly ventilated offers an opportunity to prevent further lung injuries and to improve survival rate.

In addition, close monitoring and lung function tests are often required on infants born prematurely with lung disease before discharge from specialist neonatal units. Traditional lung function tests such as peak flow and spirometry tests are not appropriate for infants because they require patient cooperation. Although other techniques like impedance pneumography (Fenner et al. 1973; Ganong 2005; Heimler et al. 1992) and plethysmography (Coates et al. 1997; Stocks et al. 2001) have been widely used for monitoring of respiratory function, these can only provide information across a brief physiological window and cannot provide regional quantitative information (Brown et al. 1985).

Different medical imaging modalities have been used for examination, diagnosis and monitoring of regional lung disease. The most commonly used are chest X-ray, computed tomography (CT) and nuclear medicine based imaging such as positron emission tomography (PET). X-ray and CT provide anatomical information, while functional CT and PET can provide three-dimensional functional images with the aid of radioactive isotopes. However, the subjects will be exposed to high ionising radiation dose if continuous imaging is performed. The uses of Magnetic Resonance Angiography (MRA) for diagnosis of embolism as well as for monitoring of pulmonary ventilation and perfusion have been documented and evaluated by Maki (1999). Although MRA does not involve ionizing radiation and has higher spatial resolution than PET, it does require use of contrast agents via intravenous injection. Furthermore, it is expensive, not feasible for continuous monitoring, requires patient cooperation, and is not portable for use at the bedside.

Although the existence of advanced imaging systems: in both anatomical and functional imaging, have significantly improved the quality of health care in terms of diagnosis and treatments, most of these imaging modalities are unsuitable for premature infants owing to a number of issues such as high dosage of radiation (Downie & Marshall 2007), the need for patient cooperation, and the lack of portability. To the best of the applicant's knowledge and belief there is, at present, no system in routine clinical use for continuous monitoring of neonate lung function in the NICU. Despite recent developments of lung ultrasound imaging tool (Caiulo et al. 2011) for use in respiratory disorders in the ICU, it is mostly used as diagnostic tool and has its limitations owing to the difficult interpretation technique and a need for special training of physicians using the tool (Gillman & Kirkpatrick 2012; Bouhemad et al. 2007).

Although EIT suffers from poor spatial resolution compared to other imaging modalities, it has excellent temporal resolution, e.g. up to 1000 frames per second (Dai *et al.* 2008), making it possible to record rapid impedance changes in the body and thus making it a potential functional imaging device. Extensive work carried out by several research groups (Smallwood *et al.* 1999; Hampshire *et al.* 1995; Marven *et al.* 1996; Taktak *et al.* 1996; Yerworth & Bayford 2007; Taktak *et al.* 1995; Brown *et al.* 2002b; Brown *et al.* 2002a; Frerichs *et al.* 2001; Murphy *et al.* 1987; Bayford *et al.* 2007) have shown that EIT has great potential for continuous monitoring of regional lung function of small, un-sedated infants in the NICU.

The development of a clinical EIT system for premature infants pulmonary monitoring has been relatively slow owing to the limitations such as the physical chest size, electrode size, skin condition of premature infants and electrode contact impedance that affect EIT measurements (Taktak *et al.* 1995; Taktak *et al.* 1996). The practical aspects of EIT measurements in neonatal lung function applications present a challenge to clinicians in terms of electrode attachment difficulties, both in applying and positioning the electrodes correctly to the un-sedated, ventilated infants. Since skin preparation or abrasion is inadvisable with premature neonates owing to the sensitivity and fragility of their skin, selection and properties of the electrodes are very important. When skin preparation or abrasion cannot be performed, contact impedance between electrode and skin, which affects the accuracy of voltage measurements, can become critically high. As such, the impedance and the size of various clinical electrodes were investigated in this thesis in order to identify the optimum electrode type for neonatal lung function monitoring using EIT (see Chapter 3).

In Rahal 2009 we investigated the impedance of clinical electrodes as a function of frequency with the aim of identifying the optimum electrode type for use with neonates. Six different types of self-adhesive electrodes commonly used in general and neonatal cardiology were investigated. These electrodes were Ag/AgCl electrodes from the Ambu® Cardiology Blue sensors range (BR, NF and BRS), Kendall (KittyCat™ and ARBO®) and Philips 13953D electrodes. In addition, a textile electrode without gel from Textronics was tested on two subjects to allow comparison with the hydrogel-based electrodes. Two- and four-electrode measurements were made to determine the electrode-interface and tissue impedances, respectively. The measurements were made on the back of the forearm of six healthy adult volunteers without skin preparation with 2.5 cm electrode spacing. Impedance measurements were carried out using a Solartron SI 1260 impedance/gain-phase analyser with a frequency range from 10 Hz to 1 MHz. For the electrode-interface impedance, the average magnitude decreased with frequency, with an average value of 5 k$\Omega$ at 10 kHz and 337$\Omega$ at 1 MHz; for the tissue impedance, the respective values were 987$\Omega$ and 29$\Omega$. Overall, the Ambu BRS, Kendall ARBO® and Textronics textile electrodes gave the lowest electrode contact impedance at 1 MHz suggesting that they are good candidates for multi-frequency EIT systems.

As described above, the issue of acquiring subject-specific boundary shape has been a subject of study in the EIT image reconstruction owing to its important role for accurate solving of the forward problem during the image reconstruction process. Many research groups have emphasised the importance of this external boundary shape information for accurate generation of EIT images (Adler *et al.* 1996; Lionheart 1998; Bayford *et al.* 2008); and have demonstrated the significant effect of inexact or unknown forward model geometry on the reconstructed images (Kolehmainen *et al.* 2008; Gersing *et al.* 1996).

In Khor 2009 we described the boundary shape problem and suggested a wearable device for measurement of boundary shape of the thorax of a neonate. We also suggested the possibility that bend and stretch sensors might be integrated into the wearable device to provide curvature and perimeter data of the boundary of thorax region of the neonate. To that end the paper presented results of an investigation into three different conductive ink type bend sensors, and in particular the repeatability and accuracy of measurements with such sensors. The sensors were manufactured by Abrams Gentile Entertainment Inc., Flexpoint Sensor Systems, Inc. and Images SI, Inc. The conclusions in our paper were as follows: (1) all of the sensors increase resistance with bending radius; and (2) the Abrams Gentile Entertainment sensors perform better compared to the other sensors, though their length raised concerns regarding suitability within the size constraints of pre-term neonates. In the paper we reported that a sensor network was under construction using bend sensors combined with stretch sensors to evaluate their effectiveness for real-time measurement of boundary shape.

## SUMMARY

Aspects of the invention are driven by the need for an EIT system for monitoring/imaging the lung function of small and un-sedated infants in the intensive care units (ICUs, also known as neonatal ICUSs), which system provides data to clinicians tasked with diagnosing and treat lung problems associated with premature birth. This is in response to the difficulty in obtaining subject-specific boundary shape models for use in EIT image reconstruction. This problem is particularly acute in the ICU setting, especially infants in an ICU. At present, the boundary shape is often assumed or guessed incorrectly (as described above), which contributes to image distortion and noise. It is to be noted that the invention is not limited to neonatal EIT applications, but can be adapted for use in other applications and patient groups with similar environmental constraints. Other fields of application for the invention include: EIT imaging of the breast, the brain or the limbs.

In one aspect the invention provides an apparatus for use in estimating the shape of a body part of a subject, which apparatus comprises:

a string of sensors for positioning adjacent the body part so that the string of sensors substantially conforms with the shape of the body part, or at least a part of it, the string of sensors comprising at least one bend sensor and at least one stretch sensor arranged end to end such that, in use, the at least one bend sensor lies adjacent a first region of the body part and the at least one stretch sensor lies adjacent a second region of the body part, wherein the magnitude of a curvature of the first region of the body part is greater than the magnitude of a curvature of the second region of the body part. A particular advantage of such an apparatus is that line-of-sight is not needed to estimate the shape of the body part.

In use data can be obtained from the bend sensors in real-time without the need for patient co-operation and without restricting access to the patient, or the patient's movement.

In some embodiments said string of sensors comprises a closed loop of sensors (which may be openable to permit placement on and removal from the body part), whereby in use, the loop of sensors circumscribes the perimeter of the body part whose shape is to be estimated. In this way the shape of the entire perimeter of the body part may be estimated. For example, the loop of sensors may be adapted to circumscribe the thorax, the breast, the head or a limb. One particular advantage of the apparatus when adapted for use on the breast is that the apparatus more or less conforms to the shape of the breast tissue in contrast to other EIT imaging methods that require a squeezing or pressing of the breast tissue to a particular shape.

In other embodiments said string of sensors has a length which is insufficient to circumscribe a body part such as the thorax, the breast, the head or a limb. In such embodiments, the string of sensors adopts an arc-like shape when held against the body part. In this way a shape of part of the body part may be estimated. The string of sensors may be incorporated into a belt or the like so that the apparatus may be held against the body part. In such embodiments, there is a first portion of the belt that comprises the string of sensors and a second portion of the belt which comprises a fastening device, which may be elasticated or adjustable in some way to hold the string of sensors against the body part.

The presence of the stretch sensor(s) brings about a number of advantages, including: a degree of movement is permitted whilst the apparatus is positioned about the body part, the apparatus can be used on a range of body parts of different sizes and, when used around the thorax, the device can accommodate changes in size with breathing. By positioning the or each stretch sensor at point(s) in the string where there is low curvature of the body part, we have realised that these advantages can be achieved, but without adversely affecting estimate of shape.

In some embodiments, the at least one stretch sensor is arranged in the string of sensors so that, in use, it lies adjacent a substantially flat region of the body part. This is preferred since it is difficult to obtain curvature data from stretch sensors. One example of a substantially flat region of a body part is the anterior or posterior of the thorax.

In one embodiment, there is a single stretch sensor in the string of sensors, with the remainder of the string comprising at least one bend sensor. In another embodiment, there may be more than one stretch sensor in the string of sensors. It is preferred that each stretch sensor is separated from other stretch sensors in the string by one or more bend sensor. However, it may be possible to position two stretch sensors next to one another in the string, provided they are positioned over a substantially flat region of the body part.

In one embodiment, there is a first stretch sensor positioned in the string so that, in use, it is positioned over the posterior of the thorax, and a second stretch sensor positioned in the string so that, in use, it is positioned over the anterior of the thorax. Between the first and second stretch sensors there is a first plurality of bend sensors and a second plurality of bend sensors.

In certain aspects the apparatus is adapted for use in estimating the shape of part of the thorax of a human subject. The particular shape of interest may be that of the perimeter of the thorax at, or just below, the nipple line, in the transverse plane. In this particular case, the second region of the body part may be the posterior and/or anterior portion of the thorax. Shape estimates of a part of or the whole of the perimeter of the thorax in the transverse plane are useful for EIT image reconstruction for lung monitoring.

In other embodiments, the apparatus is adapted for use in estimating the shape of a part of the breast, the head or a limb of a subject.

In some embodiments, the number of bend sensors is greater than the number of stretch sensors.

As mentioned above, when in use the string of sensors is positioned adjacent the body part (for example in contact with the skin) so as to conform to its shape. In preferred embodiments, the bend sensors are not directly in contact with the body part. In particular, the string of sensors may be covered (partially or completely) by a thin fabric or textile to increase comfort for the subject. In some embodiments the apparatus may be in the form of a belt that can be wrapped around the body part and releasably secured thereto, for example using VELCRO (RTM) tabs positioned at either end of the belt.

By shape of a body part we mean the shape formed by a part or the whole of the perimeter of the body part at its surface, for example the cross sectional shape of the thorax of a human subject at the nipple line in the transverse plane. The apparatus can be designed to be used with other body parts as described elsewhere herein.

In some embodiments said apparatus has an open position whereby it may be placed around said body part, and a closed position in which said apparatus is secured around the body part. In other embodiments, the apparatus is a closed loop that cannot be opened; in that case, the apparatus is slid over and around the body part.

We have found that the use of at least one stretch sensor enables the apparatus to accommodate a range of sizes of body part (for example between minimum and maximum sizes, where size can be defined length of a perimeter around the body part where the apparatus is to be used), allowing it to be used on more than one subject. Furthermore the stretch sensor permits some degree of movement when in position on a subject, so that subject movement is not unduly restricted. When the wearable device is sized and adapted for use on the human thorax the stretch sensor may accommodate change in thorax size and shape with breathing. If data is gathered from the sensors and processed quickly enough, there is the possibility of substantially real-time shape estimates of the thorax to be produced.

In some embodiments said at least one bend sensor comprises a single sensor divided into different sensing portions along its length in order to provide an effect similar to that of a plurality of separate and distinct bend sensors. In this way there may be just one or a few bend sensors, each of which extends around some portion of the body part. The division may be provided by a number of taps. For example, the bend sensor may be a conductive-ink type sensor, and each tap may comprise conductive glue by which contacts are adhered to the surface of the bend sensor so as to divide it into the different portions.

In other embodiments said at least one bend sensor comprises plurality of separate and distinct bend sensors.

In some embodiments the plurality of bend sensors numbers between 7 and 12, and preferably 8.

Preferably, each bend sensor has a length such that it bends approximately into a circular arc shape when the apparatus is positioned on the body part. In one particular embodiment, the length is about 30mm. We have found that using bend sensors of such a length greatly assists in constructing an estimated shape from measurements of all of the bend sensors. The length is chosen so that that stiffness of the sensor does not become too great (when the sensor would not conform well to the shape of the body part), but not so long that the bend sensor can adopt a non-circular shape when conforming to the shape of the body part. In one embodiment, an apparatus for estimating the shape of the thorax of a pre-term neonate comprises eight bend sensors, each of approximately 30mm length, and one stretch sensor of approximately 20mm length; the stretch sensor can double its length and therefore the apparatus can accommodate a range of thorax perimeters between about 260 mm and about 280mm. Apparatus for older and/or larger subjects are envisaged: the number of bend sensors is likely to be about eight (greater or fewer numbers are not excluded however), but the length of each bend sensor will be longer, on condition that the bent shape remains closely similar to a circular arc.

In some embodiments the length of each bend sensor is such that it bends approximately into a shape comprising a plurality of circular arcs of positive and/or negative curvature when the apparatus is positioned on the body part. A negative curvature corresponds to an inflection or 'dip' in the shape of the body part. A measurement of bending taken between either end of such a bend sensor will provide an indication of mean curvature along its length.

The bend sensor can be any device that measures strain. Accordingly other types of sensor may take the place of the bend sensors in the apparatus, such as a wire strain gauge or an aluminium foil strain gauge, etc. Elastomer skin curvature sensors could be used.

In some aspects each bend sensor comprises a conductive ink which changes resistance upon bending. In use, measurement of this resistance permits a radius of the bend sensor to be determined. In particular embodiments, conductive ink bend sensors manufactured by Abrams Gentile Entertainment, Inc. are adapted and used in the apparatus.

The bend sensors may be woven into a fabric or textile. An array of openings may be incorporated into the fabric or textile to permit quick and easy attachment of the electrodes to the skin of the subject at predefined locations. A particular advantage of such an arrangement is that the electrodes are held in known positions relative to the bend sensors. Accurate electrode positions are also useful for improving the forward model used in EIT image reconstruction. For example the apparatus may be made from a number of layers of flexible, stretchable textile material (for example any textile or fabric which has some elasticity) in which the bend sensors are sewn, woven or otherwise incorporated.

Preferably the stretch sensors are flexible so as to conform to the shape of the body part, and exhibit linearity, accuracy and repeatability when stretched. For example, it is preferred that the or each stretch sensor has an accuracy better than 2% when repeatedly stretched by the same length. In some embodiments, the stretch sensor may comprise a conductive rubber cord, or a conductive knitted yarn. In other embodiments the stretch sensor could be an elastic capacitor made of a laminated polymer structure, an example of which is available from StretchSense (www.stretchsense.com).

The apparatus may be so arranged as to allow electrode sites to be well-defined, permitting the application and removal of the electrode array to and from the patient with speed, accuracy of placement and reliability. Such an apparatus can be easily applied to the patient and fastened securely (for example using VELCRO) to ensure that there is no movement of the electrodes, but firm conformance to the body shape without adversely hindering breathing or causing discomfort. Optionally miniaturised electronic circuitry could be incorporated into the apparatus. Such circuitry could acquire measurements from the stretch and bend sensors, reducing the need for a dedicated cable per sensor running from patient to the bed-side processing and display equipment. Alternatively, the electronic circuitry could incorporate a means of wireless communication of the acquired data.

The apparatus may further comprise a plurality of strings of sensors as aforesaid, each string of sensors adjacent one another so as to provide shape estimates in different planes of the body part. For example, the strings of sensors may be arranged so that different planes of shape estimation are given along the length of the thorax of a human subject. In this way, the apparatus is useful in generating a three dimensional shape estimate of the body part. When estimating the shape of the thorax at the nipple line, there may be between two and five strings of sensors depending on size of the subject.

When the stretch sensor comprises a plurality of strings, inter-string bend and/or stretch sensors may be used between one string and another. For example, inter-string bend sensors may be placed diagonally between one string and another. Diagonal means that the longitudinal axis of the inter-string bend sensor is at some oblique angle to the longitudinal axis of the bend sensors. In this way it is possible to estimate displacement between strings in the apparatus, which helps with construction of a three dimensional shape.

The apparatus is suitable for use in various types of EIT imaging techniques including multi-frequency EIT and single frequency EIT. The apparatus may also be suitable for use when EIT is combined with other imaging modalities such as ultrasound or MRI.

In another aspect the invention provides a computer-implemented method of generating an estimated shape of a body part using an apparatus as set out above, which method comprises the steps of:

(a) reading at least one bend sensor output from each bend sensor, wherein each bend sensor output represents a curvature value of the curvature of the respective bend sensor;

(b) determining an angle subtended by the or each bend sensor using the at least one bend sensor output and a known length of the or each bend sensor;

(c) determining an angle subtended by the or each stretch sensor using an estimated curvature for the or each stretch sensor, which estimated curvature is determined based on a curvature value measured for a bend sensor adjacent the stretch sensor;

(d) adding all angles determined in in steps (b) and (c) and comparing to a predetermined angle value; and

(e) adjusting each angle determined in steps (b) and (c) if the result of comparison in step (d) is that the sum of the angles is greater than or less than said predetermined angle value; and

(f) using said adjusted angles to generate an estimated shape of the body part.

In some embodiments said apparatus circumscribes the body part and said predetermined angle value is $+2\pi$ radians considered in an anti-clockwise direction and $-2\pi$ considered in a clockwise direction. Ideally, the angular outputs from the sensors should add to make a complete circle. We have found that performing this check is a very useful way to correct some sources of error in the sensors.

In certain aspects the step of adjusting comprises either multiplying all estimated included angles by the ratio of their actual sum and $-2\pi$ or $+2\pi$ radians, or by subtracting from each subtended angle the mean angular error being the difference of the sum of the angles and $-2\pi$ or $+2\pi$, divided by the total number of bend and stretch sensors.

In some embodiments step (f) comprises:

(a) using the adjusted angle values to define a curve with i points, the gradient of the ith point in the curve given by

$$\left[ \cos\left( \sum_{1}^{i} \theta_i \right) \quad \sin\left( \sum_{1}^{i} \theta_i \right) \right]$$

where i is the number of bend and stretch sensors and $\theta_i$ is the adjusted angle value for the ith bend or stretch sensor;

(b) interpolating a spline through the *i* points;

(c) integrating the curve over its length, where the length is given by the sum of the lengths of each bend sensor and the measured length of each stretch sensor; and

(d) multiplying the integrated curve by the length to provide an estimated shape of the body part. In certain embodiments in this disclosure this is referred to as the derivative method.

In certain aspects step (b) comprises the step of interpolating a degree $\underline{n}$ B-spline through the *i* points, and the output of step (c) is degree $n+1$ B-spline.

The method may further comprise the step of using the adjusted angles to calculate respective adjusted curvature values for each bend sensor curvature value in step (a).

In some embodiments step (f) comprises generating an estimated shape of the body part by adding arcs end-to-end in a co-ordinate space, each arc corresponding to a respective one of the adjusted curvature values.

In certain aspects the first arc is generated by setting an initial point in the co-ordinate space at the end of a vector having a length equal to the radius of the corresponding adjusted curvature value, performing a plane rotation on the vector so that end of the vector sweeps through an arc to an end point, the arc having a length equal to the length of the corresponding bend sensor, and fitting the first arc between the initial point and the end point along the arc; and repeating this process for the next sensor in the string, but with the initial point set to the end point of the previous vector in the co-ordinate space;

whereby arcs are added end-to-end so as to generated said estimated shape.

The process of adding arcs end-to-end may proceed unidirectionally around the string of sensors from a start point to an end point, the end point being close to the start point. In some embodiments herein this is referred to as the unidirectional method.

In other aspects the process of adding arcs end-to-end proceeds bidirectionally around the string of sensors in opposite senses from a start point toward an end point or points, whereby respective portions of the estimated shape are generated separately. In some embodiments herein this is referred to as the bidirectional method.

In some embodiments said end point or points in the co-ordinate space corresponds to the ends of a stretch sensor in the string of sensors.

The method may comprise the step of adjusting each measured radius value by a compensation factor, or by a total compensation factor, for each bend sensor in the string of sensors. The compensation factor may be determined during manufacture for example, and compensates for measurement inaccuracy of the bend sensors.

In certain aspects the apparatus comprises a plurality of strings of sensors of known separation between each string, wherein the method further comprises the steps of generating a two dimensional estimated shape for each string of sensors, stacking the two dimensional estimated shapes above or below one another in co-ordinate space, and blending the stacked two-dimensional estimated shapes into a three dimensional estimated shape.

In some embodiments, the apparatus comprises one or more axial bend sensor between the strings of sensors, and the method further comprises the step of using the output from the or each axial bend sensor to shift each two dimensional estimated shapes in the x-y plane. In this way the three-dimensional shape has improved accuracy.

The step of blending said two-dimensional estimated shapes may comprise a contour stitching method or interpolating a spline, such as a cubic spline.

In another aspect the invention provides a computer-implemented method of generating an estimated shape of a body part using an apparatus as set out above, which method comprises the steps of:

> (a) reading a bend sensor output from each bend sensor and a stretch sensor output from the stretch sensor, wherein each bend sensor output represents a curvature value of the curvature of the respective bend sensor, and the stretch sensor output represents a length value of the length of the stretch sensor; and
> (b) generating two portions of the estimated shape, a first portion generated by adding arcs end-to-end corresponding to the appropriate radius values toward or away from one end of the stretch sensor, and a second portion generated by adding arcs corresponding to the appropriate radius values toward or away from the other end of the stretch sensor.

In certain embodiments herein this method is referred to as the bi-directional sensor co-ordinate generation algorithm. 'Bi-directional' refers to the fact that the estimated shape is built up in opposite directions starting from either end of the stretch sensor. We have found that working toward either end of the stretch sensor greatly increases the accuracy of the estimated shape. However, it is possible to work away from either end of the stretch sensor.

In some embodiments, the first and second portions are approximately a first half and a second half of the apparatus respectively.

The stretch sensor is assumed to be positioned over a substantially flat region of the body part, such as the anterior or posterior of the thorax of a human subject. In the estimated shape the stretch sensor can be assumed to be a straight line in the estimated shape. This has been found to substantially improve the accuracy of the estimated shape.

The method may further comprise the step of interpolating a curve or spline through all co-ordinate points to generate said estimated shape of the body part.

It will be appreciated that the co-ordinate points do not have to be calculated completely for one portion before commencing the next portion. For example it would be possible for both portions to be determined substantially simultaneously, increasing speed of shape estimation. Accordingly the method may comprise performing steps (ii), (iii) and (iv) at substantially the same time as step (v), whereby bend sensor co-ordinates for both portions of the apparatus are generated substantially at the same time.

In any shape estimation method described herein the radius of each bend sensor may be determined by inputting the bend sensor output into a bespoke model for that bend sensor, such as a linear regression model, in order to determine the corresponding radius value. The length of the stretch sensor may also be determined by inputting the stretch sensor output into a bespoke model for that stretch sensor, such as a linear regression model, in order to determine the corresponding radius value. In certain aspects the linear regression models take the form of an equation of a line relating sensor output to radius. The bespoke models may be stored in memory on the computing device that performs the method.

The last two end co-ordinate points of the bend sensors in each half do not always meet in the co-ordinate space. The method may further comprise the step of calculating a final co-ordinate point midway between the last two end co-ordinate points. We have found that the fit of the curve or spline with the real shape can be improved in this way.

In certain aspects the apparatus comprises a plurality of strings of sensors, optionally with a fixed vertical separation. In that case, the method described above is used to generate an estimated shape for each string. Following that the strings can be 'stacked' on top of one another in co-ordinate space (for example in the z direction, where the two dimensional shape of the string is described in the x-y plane). In that way, a three dimensional shape of the body part may be generated.

In some aspects the apparatus comprises a one or more axial bend and/or stretch sensor positioned between different strings of sensors. Such axial bend sensors enable measurement of curvature in the z direction. Accordingly the method may further comprises the step of reading an output from the or each axial bend sensor representing curvature between strings of sensors, and adjusting co-ordinates of the strings of sensors to reflect that curvature. In this way strings of sensors are not simply stacked on top of one another (in the z direction): the curvature in the axial direction enables a more accurate representation of the shape of the body part to be generated.

After that, a three dimensional boundary/surface can be fitted to the three dimensional shape using known techniques, for example by interpolating a spline , or generating a 3D co-ordinate point cloud (scattered or organized). Alternatively the estimated three dimensional shape can be in the form of a B-spline surface to which a predefined finite element model can be warped that is based on simpler geometry such as a cylinder. It would be possible to use other types of spline, such as a flat plate spline.

The method may further comprise the step of using the estimated shape (two dimensional or three dimensional) in a forward model representing the body part of the patient. Following that the method may comprise the step of solving the forward model as part of a process of reconstructing EIT images. The shape estimation may proceed substantially continuously, whereby shape estimates may be input into the forward model substantially in real-time.

According to other aspects of the invention, there is provided a computer-readable storing medium comprising executable instructions that when executed perform any of the method steps described above. The executable instructions may be in the form of a computer program, which may be provided on a CDROM, DVD, mass storage device, etc. Alternatively the computer program may be stored on a remote server for download via a website. For example the apparatus may be supplied with directions to visit a website to obtain the computer program for extracting and analysing data from the apparatus. The computer program may be stored and executed locally on a computing device that is brought near the subject during monitoring. In other aspects, the computer program may be stored on a device (e.g. PLC) at point of manufacture, and supplied as a device adapted to interface with the apparatus, and to send data to another computing device (such as a PC with a display).

In one aspect the invention provides a computer-implemented method of generating an EIT image of a body part of a subject, which method comprises the steps of:

(i) placing an apparatus as described above onto a body part of a subject;
(ii) generating an estimated shape of the body part of a patient using a method as described above;
(iii) using the estimated shape in a forward model of the body part of the subject; and
(iv) generating an EIT image by solving the forward model, and displaying the EIT image.

Steps (ii) - (iv) of the method may be repeated substantially continuously whereby EIT images are generated in real-time using real-time shape estimates of the body part of the subject.

In another aspect the invention provides a method of manufacturing an apparatus for use in estimating the shape of a body part of a subject, which apparatus comprises a plurality of bend sensors (which may be one or more bend sensor each divided into different sensing portions, or a plurality of separate and distinct bend sensors), the method comprising the steps of:

(a) for each bend sensor:

(i) bending the bend sensor by different amounts;
(ii) for each amount of bending, storing data representing a response of the bend sensor associated with the amount of bending;
(iii) using the data generated in step (ii) to determine a model of the response bend sensor; and
(iv) storing the model of the bend sensor in a computer memory associated with an identity of the bend sensor;

(b) repeating steps (i)-(iv) for each other bend sensor, whereby the computer memory stores a model for each bend sensor; and
(c) arranging each of the bend sensors in a string of sensors, which may comprise at least one stretch sensor.

In some embodiments, the bend sensors are of the conductive ink type and have a resistance that varies with amount of bending. To achieve different amounts of bending, a mandrel may be used that has a shape with a stepped profile that defines circular surfaces of differing radii. One example of such a mandrel is a cone-like shape with a stepped profile which forms the surfaces having different radii. By bending each bend sensor on each of the circular surfaces of the mandrel, it is possible to generate and store a model which represents the characteristics of the bend sensor.

Once models have been generated for all of the bend sensors (or sensing sections thereof), the method may further comprise the step of placing the apparatus around a known shape, for example a cylinder of known radius. The output (e.g. resistance or voltage) from each bend sensor may be measured, and the model of the bend sensor used to convert the measurement to an estimated radius. Once this is done for all bend sensors, the estimated radii are compared to the known radius. The comparison can be used either to generate and store an error correction factor for each individual bend sensor, or to generate and store an error correction factor for all of the bend sensors. For example, in one particular embodiment, we found than an error correction factor of 0.85 (or -15%) was needed when using conductive ink type bend sensors manufactured by Abrams Gentile Entertainment, Inc. The error correction factor(s) can be stored in the computer memory and applied to estimated radii in the field.

In one aspect the invention provides a method of manufacturing an apparatus for use in estimating the shape of a body part of a subject, which method comprises the steps of:

(a) dividing into a first number of equal length segments a 2D shape that approximates the shape of the body part to be estimated;

(b) fitting one or more circular arc to each segment of said first number of segments to generate an estimated shape that approximates said 2D shape;

(c) determining an error between said estimated shape and said 2D shape;

(d) repeating steps (a) to (c) with a second number of segments different to said first until a plurality of errors is generated;

(e) comparing one or more of said plurality of errors to identify the fewest number of segments required to reduce said error below a predetermined threshold; and

(f) constructing the apparatus with a number of bend sensors equal to the fewest number of segments in step (e).

The 2D shape could be an ellipse for example. There are various ways that the predetermined threshold can be set. For example, in step (c) the error between the end of each circular and the 2D shape can be determined as a distance. One possible way to do that is to determine the square root of the sum of the squares of the differences between the x and y estimated and true co-ordinates respectively. This generates a set of distance differences. Using these values, the maximum distance difference and the mean distance difference can be determined. Once the maximum and mean distance differences are known, either one or both of these values can be compared against some parameter of the 2D shape to determine how well the estimate fits, and therefore whether or not the error is below the predetermined threshold.

In one embodiment, the mean distance difference is compared with the average radius of the 2D shape, which may be expressed as a percentage. This process can be repeated for the estimated shapes with different numbers of segments. When defined in that way, the predetermined threshold should be 6% or lower, and preferably 2% or lower, for EIT image reconstruction. The fewest number of bend sensors is then used in step (f) which is expected to reduce the percentage error just below the predetermined threshold.

The invention is defined by the appended claims, the description being for illustrative purposes.

## BRIEF DESCRIPTION OF THE FIGURES

For a better understanding of the present invention reference will now be made, by way of example only, to the accompanying drawings in which: -

Fig. 1(a), (b) and (c) show a subject divided into voxels and how a sensitivity matrix is used in a forward model to obtain values for resistivity in each voxel;

Fig. 2 is a schematic block diagram a known EIT apparatus in use on a subj ect;

Fig. 2A is schematic diagram of electrodes positioned around the thorax of a subject with some positions of current source and voltmeter shown to illustrate how data is generated;

Fig. 3(a), (b) and (c) shows a plan view, a perspective view and a side view respectively of a mandrel used in measuring the outputs provided by bend sensors bent to different radii;

Fig. 4 is a schematic circuit diagram of a Wheatstone bridge used to convert bend sensor resistance to voltage;

Fig. 5 is a schematic block diagram of an apparatus for measuring bend sensor output using the mandrel of Fig. 3 and the Wheatstone bridge of Fig. 4;

Fig. 6 is a graph of radius versus resistance measured for the Abrams Gentile bend sensors;

Fig. 7 is a graph of curvature versus resistance measured for the Abrams Gentile bend sensors;

Fig. 8 is a graph of radius versus resistance measured for the Flexpoint bend sensors;

Fig. 9 is a graph of curvature versus resistance measured for the Flexpoint bend sensors;

Fig. 10 is a table showing percentage resistance drift for the Abram Gentile and Flexpoint bend sensors after 30s at each radius on the mandrel of Fig. 3;

Fig. 11 is a table showing percentage resistance drift of the bend sensors in a flat position before and after an entire resistance drift test;

Fig. 12 is a table showing calculated radius from resistance measurements of the bend sensors on the mandrel of Fig. 4, together with the percentage error;

Fig. 13 (a) is a diagram of a stretch sensor used in a belt;

Fig. 13 (b) is a diagram of the stretch sensor of Fig. 14 (a) being calibrated using Vernier callipers;

Fig. 14 is a schematic diagram showing how three bend sensors may be combined to form a shape;

Fig. 15 is a graph of curvature versus resistance for three different Abrams Gentile bend sensors;

Fig. 16 is a graph of stretched length versus resistance for the stretch sensor of Fig. 14(a);

Fig. 17 (a) is side view of a first belt laid in a flat position;

Fig. 17 (b) is a plan view of the first belt of Fig. 17 (a) in closed position around a circular shape; and

Fig. 17 (c) is a perspective view of an experiment using the first belt to estimate the shape of a beaker;

Fig. 18 is graph showing the estimated shape from the experiment of Fig. 17 and the true shape of the beaker;

Fig. 19 (a) is a plan view of a modified stretch sensor;

Fig. 19 (b) is a plan view of a modified bend sensor;

Fig. 20 is a table showing results obtained in testing a second belt fabricated using the modified bend and stretch sensors of Figs. 19 (a) and (b);

Fig. 21 is a graph showing the estimated shape from an experiment using the second belt of Fig. 20;

Fig. 22 is a graph of points versus position error for different total sensor error compensation factors;

Fig. 23 is a graph showing the estimated shape from an experiment using the second belt of Fig. 20, with a compensation factor applied;

Fig. 24 is a graph of points versus position error for different total sensor error compensation factors;

Fig. 25 (a) shows the process of scanning an anatomically correct baby doll using a 3D scanner;

Fig. 25 (b) shows the 3D computer model produced by the 3D scanner in Fig. 25 (a);

Fig. 26 (a) shows a cross section through the 3D computer model of Fig. 25 (b);

Fig. 26 (b) shows a closed cubic spline fitted to the cross section in Fig. 26 (a);

Fig. 27 is a 2D shape generated using a third belt;

Fig. 28A is a 2D shape generated using the third belt, but with an adapated coordinate generation algorithm;

Fig. 28B is a closed splined interpolation of the 2D shape of Fig. 28A;

Fig. 29 is a 2D generated shape using the third belt, which includes an additional coordinate point to correct mismatch between ends of the bend sensors;

Fig. 30 is a table of mean, maximum and minimum, error correction factor for individual bend and stretch sensors;

Fig. 31A, 31B and 31C are 2D generated shapes using the third belt, illustrating the difference between minimum, mean and maximum sensor error correction factor respectively;

Fig. 31D is a schematic diagram representing a method of estimating shape using derivatives;

Fig. 32 is a diagram illustrating various methods for determining curvature;

Fig. 33 is diagram of a circle constructed using a coordinate generation algorithm;

Fig. 34 is a graph of maximum error factor versus b/a ratio for different numbers of segments;

Fig. 35 is a graph of mean error factor versus b/a ratio for different numbers of segments;

Fig. 36 is a graph of maximum error factor versus b/a ratio for 12 segments;

Fig. 37 is a graph of maximum error factor for an ellipse with a semi-major axis of 50mm and a semi-minor axis of 40mm;

Fig. 38 is a table showing values from the graph in Fig. 37;

Fig. 39A is a graph of mean error versus number of segments for an ellipse with a semi-major axis of 45mm and a semi-minor axis of 35mm;

Fig. 39B is the graph of Fig. 39A to which an inverse cubic relationship is shown between number of segments and mean error;

Figs. 40A, 40B and 40C show the ellipse of Fig. 39 with shapes estimated with 4, 8 and 12 segments respectively;

Fig. 41 is a spline fitted to CT scan data of an infant;

Fig. 42A is a boundary shape estimation generated using a uni-directional coordinate generation algorithm;

Fig. 42B is a boundary shape estimation generated using a bi-directional coordinate generation algorithm;

Fig. 42C is a boundary shape estimation generated with eight circular arcs using a uni-directional coordinate generation algorithm and an angle error correction method;

Fig. 42D is a boundary shape estimation generated with sixteen circular arcs using a uni-directional coordinate generation algorithm and an angle error correction method;

Fig. 42E is a boundary shape estimation generated with eight circular arcs using a bi-directional coordinate generation algorithm and an angle error correction method;

Fig. 42F is a boundary shape estimation generated with sixteen circular arcs using a bi-directional coordinate generation algorithm and an angle error correction method;

Fig. 43A - C show EIT images reconstructed using three different thorax shapes in a forward model;

Fig. 44A shows four known FEM meshes;

Fig. 44B shows a known neonatal FEM mesh;

Fig. 45 is a table of various data about premature infants gathered by Great Ormond Street Hospital;

Fig. 46 is a table showing possible electrode numbers, electrode spacing and number of electrode rings for the patient age groups in Fig. 45;

Fig. 47A is a graph illustrating construction of a 3D shape by stacking 2D boundary shape estimates;

Fig. 47B is the graph of Fig. 47A on which curves have been fitted to join the 2D boundary shape estimates together; and

Fig. 48 is a schematic plan view of a fourth sensor belt.

Fig. 49 is a graph illustrating co-ordinate determination for bend sensors in the belt of Fig. 48.

## DETAILED DECRIPTION OF THE PREFERRED EMBODIMENTS

The description comprises the following sections:

1. EIT systems;
2. Bend sensors
3. Stretch sensors
4. 2D Boundary Shape Estimation
5. Bend sensor modelling
6. 3D Boundary Shape Estimation
7. 3D Data Measurement

## 1. <u>EIT Systems</u>

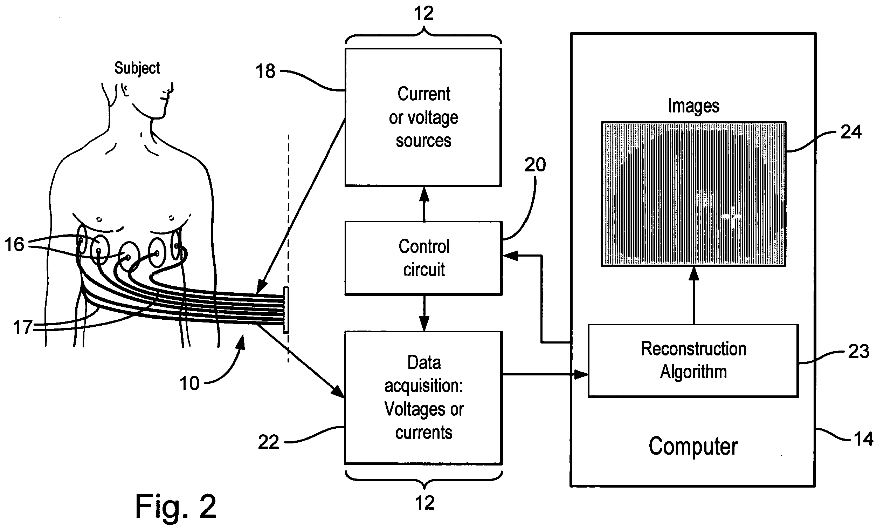

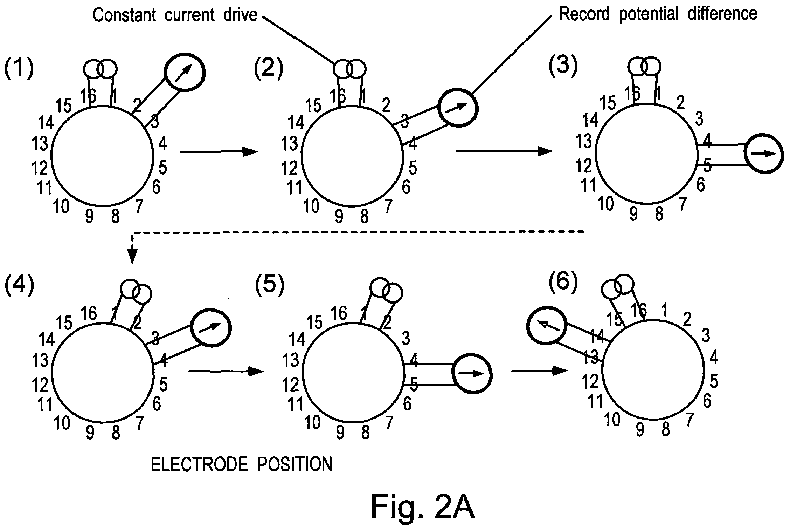

Referring to Fig. 2 a known EIT imaging system 10 comprises system instrumentation 12, a computer 14 for data acquisition and data processing, and an array of electrodes 16. In use, the array of electrodes 16 is placed on the subject around the body part of interest, and each electrode is connected to the system instrumentation 12 via a respective wire lead 17 (such as a co-axial cable). The array of electrodes 16 usually comprises between 8 and 64 individual electrodes, although other numbers are possible. Aspects of invention are concerned with monitoring of lung function; in that case the array of electrodes would be placed around the thorax of the patient, typically just below the nipple line.

The system instrumentation 12 comprises one or more current source 18 controlled by control circuitry 20, and data acquisition hardware 22 which includes digital voltmeters with operational amplifiers. An electronic switch (such as a multiplexer) is positioned between the array of electrodes 16 on the one hand and the digital voltmeters and the current source 18 on the other. In this way the current source 18 and digital voltmeters can be switched between the electrodes under control of the control circuitry 20. This enables different current injections and measurement methods be performed according to the particular EIT application. The computer 14 comprises a memory storing computer-executable instructions that enable collection and storage of data from the array of electrodes 16. The computer-executable instructions further comprise an image reconstruction algorithm 23 that is able to process data gathered from the electrodes and to generate an image or images therefrom, and to output the image(s) on a display 24.

Fig. 1A illustrates the principle of EIT data acquisition using the EIT imaging system 10. This procedure was employed by a standard early prototype, known as the 'Sheffield mark 1' system (Brown and Seagar 1987). Sixteen electrodes 16 are applied to a subject, roughly approximating a ring as shown. In step (1) a single measurement is made with four electrodes: a current of up to 5 mA at 50 kHz is applied between an adjacent pair of electrodes (numbered 1 and 16), and the voltage difference is recorded by a voltmeter between two other adjacent electrodes (numbered 2 and 3 in this step). This yields a single transfer impedance measurement which is stored by the computer in memory. Only the in-phase component of the voltage is recorded, so this is a recording of resistance, rather than impedance. Voltage differences are measured between all other adjacent electrode pairs in turn (see steps (2) and (3)), until the entire ring of electrodes has been measured. In step (4), the current is applied to the next adjacent pair of electrodes, and voltage differences measured between adjacent pairs of electrodes (see step (5)). Sequential pairs of electrodes are then successively used for injecting current until all possible combinations have been measured (see step (6)). Each individual measurement takes less than a millisecond, so a complete data set of 208 combinations is collected in 80 ms, and 10 images/s can be acquired. This can be increased to 25 frames/s if reciprocal electrode combinations are not used, and each data set comprises 104 measurements. Some modern EIT systems are reported to produce up to 1000 frames/s (Dai *et al.* 2008)

As the data is gathered from the electrodes, it is processed by the reconstruction algorithm 23 to produce images of the resistivity in the part of the subject under examination. As explained in the background section above, this involves reversing the forward model to obtain the resistivity in each voxel from voltages measured between different pairs of electrodes. In solving the forward problem one of the important boundary conditions is the shape of the surface of the subject and the position of electrodes on that surface: it has been established that the surface and electrodes positions in the forward model should conform to the shape of the patient and real electrode position as closely as possible.

As mentioned in our paper Khor 2009, we have investigated bend sensors and stretch sensors with the aim of designing a sensor network to enable real-time measurement of boundary shape.

## 2. Bend Sensors

At the priority date hereof, the common types of bend sensors (also known as flexion sensors) were conductive-ink based, fibre-optic and conductive fabric, or textile. The conductive-ink based bend sensors were commercially available and widely used in many different applications.

For convenience, the term 'bend sensor' in the following refers to conductive-ink based flex sensors, but the invention is not limited to that kind of bend sensor as explained elsewhere herein.

In order to choose the right type of bend sensor for proof of principle, a test was carried out to obtain regression model of each sensor type by relating two variables: bend sensor output and curvature.

Electrical conductive bend sensors comprise a thin plastic film or substrate coated with a proprietary conductive ink. The commonly used substrates are of polyester or polyimide film such as Mylar® or Kapton® with thickness of less than one millimetre and have high durability. The conductive ink is a type of resistive element, which typically used for making potentiometers, but is usually modified during manufacturing according to the manufacturer's desired specifications e.g. resolution and durability. The conductive ink usually comprises carbon particles in a binder. The conductive ink coated on the substrate forms a strong bond such that it will continue to strongly attach to the substrate when it is bent at different angles and maintain its integrity in shape.

By way of example, US-A-5 086 785 describes bend sensor sold by Abrams Gentile Entertainment, Inc. That document explains how the electrical resistance of the conductive ink changes with bending (see col. 7 1.18-65). In particular, when the substrate is bent such that the printed ink is on the outside of the bend, two physical reactions within the ink cause a change in its electrical resistance. In the first physical reaction, the ink stretches causing the distances between the conductive carbon particles to increase. This causes a very steady, predictable increase in electrical resistivity. This physical reaction also occurs when the conductive ink is on the inside of the bend, with the distances between particles decreasing, thus decreasing resistivity.

By a second physical reaction, micro cracks form transversely to the longitudinal extent of the sensor when the conductive ink is on the outside of the bend. As the bend increases, the width of these cracks increases causing a more dramatic increase in electrical resistance.

US-A-5 086 785 also explains one way these effects can be achieved. In particular, two ink formulas may be mixed to create the conductive ink, depending on the particular characteristics desired. A more durable stretchable ink formula embodies the characteristics of the first physical reaction and a second ink formula is more brittle and embodies the characteristics of the second physical reaction. These two inks may be mixed to customize the performance of the bend sensor. US-A-5 086 785 explains that a suitable ink comprising a combination of both ink types can be obtained from Amtech International, Lot 92349. These inks are typically used in making potentiometers. When a greater percentage of the second ink is used, the rise of resistance versus bend is much sharper and higher. US-A-5 086 785 says that it was found that for use where a high amount of resolution is desired, a higher percentage of the second ink should be used. For durability and average resolution, e.g., subdivision of the resistance range into approximately 4 levels, a higher percentage of the first ink should be used. In particular, for use in a bend sensor to be affixed to a glove for determining hand positions for inputting data into a video game controller with four levels of resolution, the conductive ink identified above Amtech Lot 92349 comprising a selected percentage of the first ink and the remainder the second ink is preferable.

In order to select a bend sensor for further investigation, three off-the-shelf bend sensors were compared. The important criteria we were searching for are: linear relationship between sensor outputs versus geometric characteristics, stability and accuracy. The three bend sensors were the Abrams Gentile Entertainment sensor (http://www.ageinc.com/tech/index.html, a sensor from Flexpoint Inc. (www.flexpoint.com) and a sensor from Images SI Inc. (www.imagesco.com). The Table below provides a summary of the bend sensors' specifications.

|  | Abrams Gentile -AG | Images Co-IC | Flexpoint-FP |
|---|---|---|---|
| **Dimensions L x W x T (mm)** | 114x6x0.5 | 114x6x0.5 | 50x6x0.1 |
| **Flat Resistance (k$\Omega$)** | ~ 10 | ~ 20-50 | ~ 3 - 5 |
| **Excitation voltage (V)** | ≤ 5 | - | 5 - 10 |

*-Measuring bend sensor output*

In order select a suitable bend sensor for incorporation into a belt, a test was carried out to obtain regression model for each sensor by relating two variables: bend sensor output and curvature when bent.

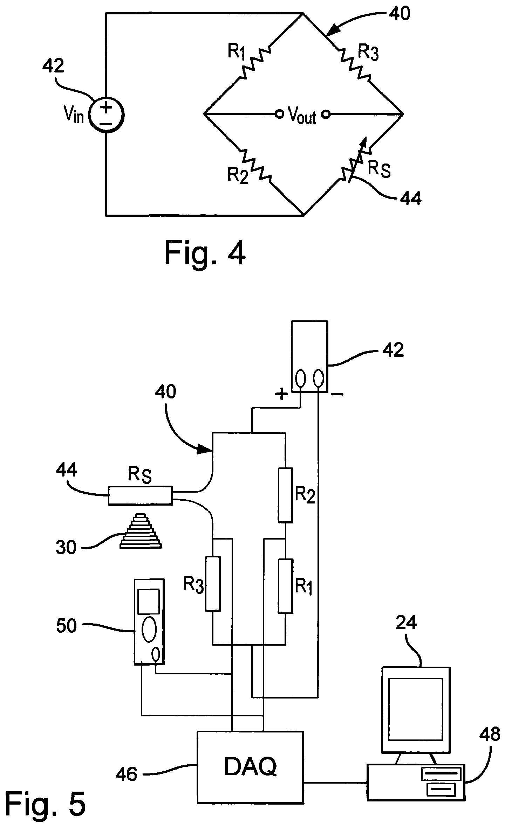

To do that each bend sensor was bent around different parts of a custom-built mandrel, developed in CAD, and machined from hardwood (other materials could be used of course). Referring to Fig. 3 the mandrel 30 comprises a cone-like shape having a stepped edge 32. The stepped edge 32 defines various radii ranging from 10 mm to 50 mm, with a 5 mm interval. The output of each bend sensor was recorded for each radius on the mandrel. To do that the output of the bend sensor 44 was fed to a custom-built Wheatstone bridge 40 (see Fig. 5) driven by a constant excitation voltage of $\pm 5$ V from a power supply 42. The value of $R_1$, $R_2$ and $R_3$ were set to balance the Wheatstone bridge with reference to the particular bend sensor. Referring to Fig. 4 the Wheatstone bridge converted the output resistance of the bend sensor 44 into a voltage $V_{out}$ which was sampled at 1 kHz using a National Instruments USB 6212 data acquisition system 46 with the aid of Labview 2011 programming software on a computer 48 in order to convert the voltage into resistance for data analysis. During the sampling of data, the measurement readings were simultaneously verified using a digital multimeter 50.

To find the resistance $R_s$ of the bend sensor the following equation was solved:

$$V_{out} = \frac{R_s}{(R_3 + R_s)} V_{in} - \frac{R_2}{(R_1 + R_2)} V_{in}$$

Sensors encounter resistance drift (see below) and in order to rectify this problem, the analysis of data was based on a mean value, obtained by averaging the sensor output over a short period of time (of the order of seconds) immediately after being bent around the mandrel 30.

During the experiment it became apparent that the values of the Images Scientific bend sensors (bi-directional) were not able to provide constant and sensible results when they were bent. Therefore, only the Abrams Gentile and Flexpoint bend sensors were used in the performance comparison.

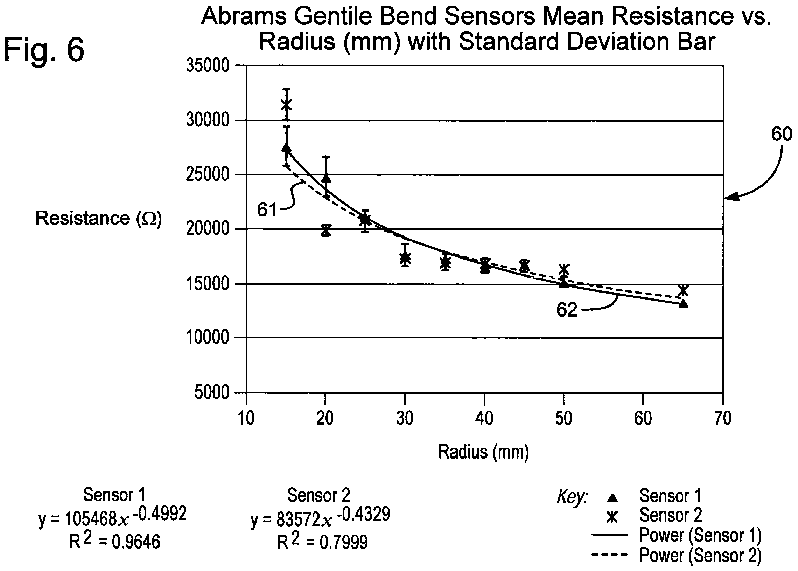

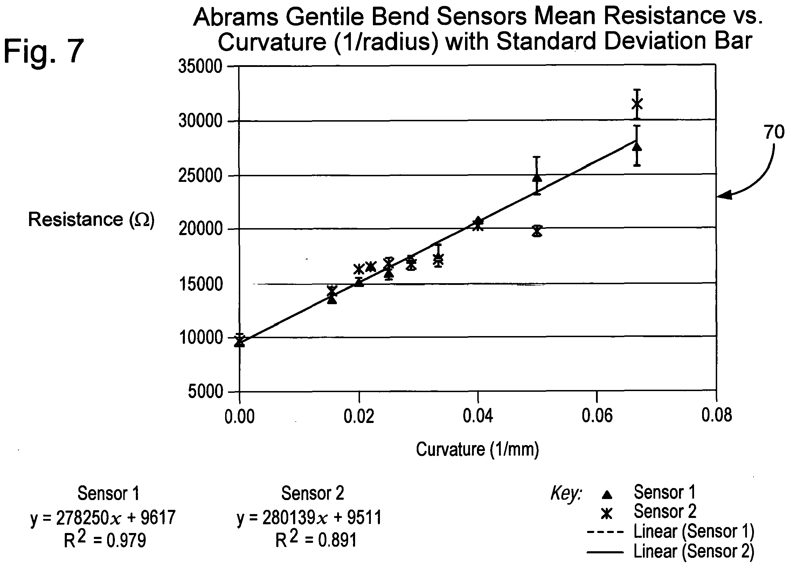

The recorded data were plotted into graphs and a linear regression model obtained for each bend sensor 44. Referring to Fig. 6 a graph 60 shows the measured resistance of the Abrams Gentile bend sensors 44 versus the radius of the bend sensor 44 on the mandrel 30. Power law curves 61 and 62 have been fitted to the data, with the formulae shown at the bottom left of the graph. In Fig. 7, the graph 70 shows the same data as in Fig. 6 except that radius, *r,* has been converted to curvature, $\kappa$, as $\kappa$= *1/r.* A linear relationship exists between curvature and resistance for the Abrams Gentile bend sensors; again the formulae are shown at the bottom left corner of the graph.

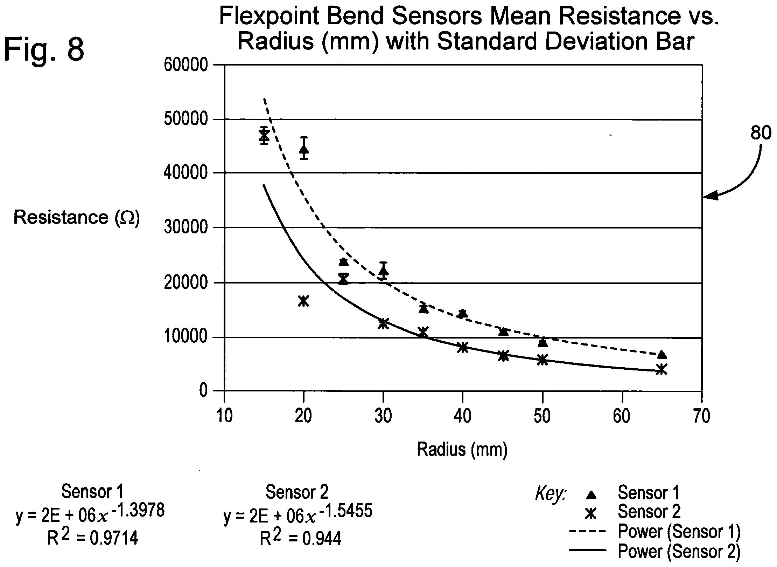

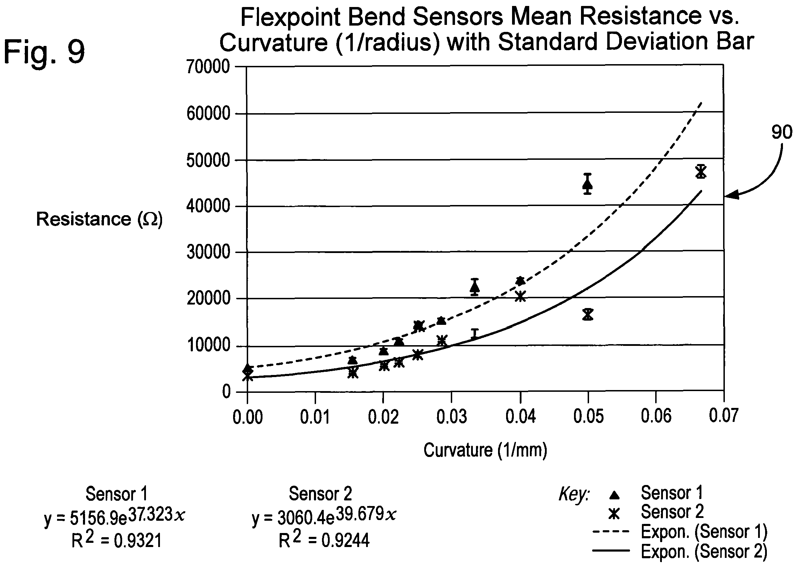

Figs. 8 and 9 show similar graphs to Figs. 6 and 7, but for the Flexpoint bend sensors. The Abrams Gentile sensors exhibit a linear relationship between curvature ($\kappa$) and sensor resistance output for a wider curvature ($\kappa$) range of from approximately 0 mm$^{-1}$ (flat) to 0.065 mm$^{-1}$, with a maximum-minimum resistance difference of approximately 5 kQ. In contrast, the Flexpoint sensors show an exponential regression relationship with a maximum-minimum resistance difference of 28.18 k$\Omega$ in their mean output values. Fig. 9 also shows a possibility of linear regression for both of the Flexpoint bend sensors, although this is limited to within a short curvature ($\kappa$) range of approximately from 0.015 mm$^{-1}$ to 0.035 mm$^{-1}$.

*-Sensor Drift*

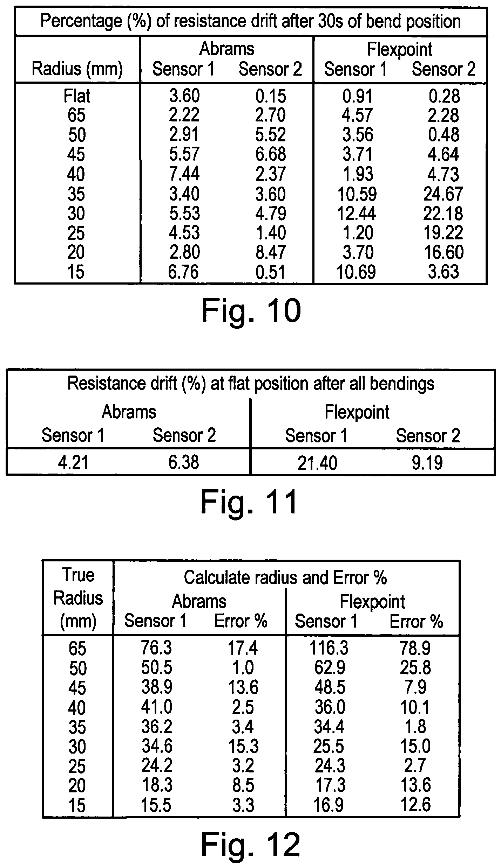

We noticed that the sensors appear to exhibit resistance drift. To investigate this, each bend sensor was placed on the mandrel 30 for a period of 30s at each radius interval. The resistance indicated at the beginning and end of the period was noted, and the percentage change calculated. Fig. 10 shows a table with the results for the two Abrams Gentile sensors and the two Flexpoint sensors. Although the results show that both bend sensors from Flexpoint have relatively less drift in resistance when bent at the higher radius range, they were not consistent throughout the whole range of radii. Overall, both bend sensors of the Abrams Gentile showed better performance when compared to the Flexpoint's in terms of consistency and lower resistance drift over time for a wider range of bending radii, where the maximum drift in resistance over time is less than 10 %. The largest drifts over time for each Flexpoint sensor are 12.4% and 24.7%. These results show that Abrams Gentile sensors have better stability performance over time compared to the Flexpoint's.

*-Repeatability*

Before performing the drift test above, the resistance of each sensor in the flat position was noted. After the drift test, each sensor was returned to a flat position and the resistance noted. From these two values, a percentage resistance drift was determined for each sensor and is shown in Fig. 11. The Abrams Gentile sensors exhibited lower resistance drift than the Flexpoint sensors.

*-Accuracy*

Having determined a regression fit for each sensor relating curvature to resistance (see Figs. 7 and 9) the accuracy of the bend sensors was tested. This was done for one Abrams Gentile sensor and one Flexpoint sensor. Each sensor was applied to the mandrel 30 at each radius along the stepped edge 32. At each radius the resistance of the bend sensor was measured. The respective regression model and measured resistance were used to calculate the curvature of the bend sensor, and finally to convert the curvature into a radius. Fig. 12 shows a table of the true radius of the mandrel 30, together with the calculated radius and percentage error. The maximum and minimum errors of the Abrams Gentile sensor are 1.0% and 17.4% compared to 1.8% and 78.9% of the Flexpoint sensor.

*-Summary*

Overall, it was found that the Abrams Gentile bend sensors demonstrate better performance compared to Flexpoint bend sensors in terms of stability, repeatability and linear regression fitting relationship between sensor outputs and curvatures. This will enable curvature estimation from measurement of resistance. Further adaptation (described below) will enable shape to be estimated. In addition, the Abrams Gentile bend sensors also seem offer the flexibility for end-user to modify the sensors because they have uniform conductive contact area on the surface. This enables alteration made to the sensor length and/or multiple outputs from one sensor at the same time via deposition of conductive paint to the surface.

### 3. Stretch sensors

One aspect of the invention is to use one or more stretch sensor together with a number of bend sensors.
A stretch sensor was obtained from Images SI Inc. (http://www.imagesco.com). The stretch sensor was made of elastic cylindrical conductive polymer with core diameter of 1.5 mm. The conductivity is typically achieved by impregnating a polymer, such as rubber, with carbon particles. A stretch sensor uses a concept similar to that of a variable resistor: the stretch sensor increases in resistance when stretched and returns to its nominal resistance when released. According to the datasheet of the stretch sensor, the average nominal resistance of the stretch sensors is 1 kΩ per linear inch (25.4 mm). However, when sensors are stretched to more than 50% of their original length, the resistance will be doubled to approximately 2 kΩ per linear inch. Any further stretching and the resistance begins to decline; therefore the recommended operating range of the stretch sensor is below 50% of original length. A particular advantage of using stretch sensors is that they offer the potential to be modified and woven into fabrics, to form a wearable device.

### 4. 2D Boundary Shape Estimation

As described above, the Abrams Gentile bend sensors were found to exhibit a linear regression relationship between the sensor output (resistance or voltage) and curvature. Furthermore, the uniform conductive contact patch area on the surface of the bend sensor allows flexibility for modifying sensor length, whilst retaining a linear output relationship. This section reports an investigation into the feasibility of using a series of the Abrams Gentile bend sensors combined with one or more stretch sensor in providing an estimation of different boundary shapes.
Three prototype belts were built and tested. The belts had the following configurations:

1. First belt: three full-length (114 mm) Abrams Gentile bend sensors and two stretch sensors (30mm), tested on a round object to which a 2D shape was generated without sensor error correction.
2. Second belt: five modified-length (three 50 mm and two 25 mm) Abrams Gentile bend sensors and a short stretch sensor (20 mm), tested on a round object, with error correction techniques introduced to the algorithm.
3. Third belt: eight equal-length (30 mm) bend sensors and a stretch sensor (15 mm), tested on an anatomically correct baby doll with a chest circumference (~260 mm) approximately the same as that of a premature neonate. A modified approach of transforming sensor outputs into coordinate points on the x-y plane is used. A compensation technique is introduced to minimise algorithm error.

*-Calibration*

As explained in section 3 above, for any given radius on the mandrel 30 the resistance generated by each bend sensor was different. Thus an individual linear regression model needs to be generated and stored (for example in computer memory, such as in the computer 14 of Fig. 2) for each bend sensor. This will be referred to as bend sensor calibration. The calibrations were performed using the mandrel 30: each bend sensor was placed flat on a flat surface before and after wrapping on each radii of the mandrel 30, thereby systemizing the sensor drift error. The electrical outputs of each sensor were acquired using the NI USB6212 DAQ 45 with a sampling frequency of 1Khz. For each sensor, over 1000 data samples were taken and converted into resistance values, of which a mean value was computed automatically. Following that, the mean resistance for each bending radius was plotted against its curvature of radius. The relationship between each sensor output and its bending profile was obtained within the specific range, through linear regression fit. The resulting equations were stored in memory of the computer 14. Alternatively it would be possible to store the equations on a portable computer-readable memory (e.g. CDROM, DVD, mass storage memory device, etc.) or to store the equations in a database on a remote server. If stored on a remote server, the equations could be accessed as needed by the computer 14 using the Internet for example. It is envisaged that each bend sensor could have an identity that uniquely identifies it to the server. In this way the computer 14 can send the identity to the remote server as a query to obtain the equation for that particular bend sensor. In other embodiments, it would be possible for the apparatus to comprise a small form factor processing device and memory (e.g. ASIC). The device may be preprogrammed with the equations of the bend sensors on the particular apparatus. In use, voltage/resistance measurements from each bend sensor are converted by the device output through a data cable to the computer 14.

As will be described in greater detail below, the equations are used to estimate the sensor bend radius or curvature based upon a measured output.

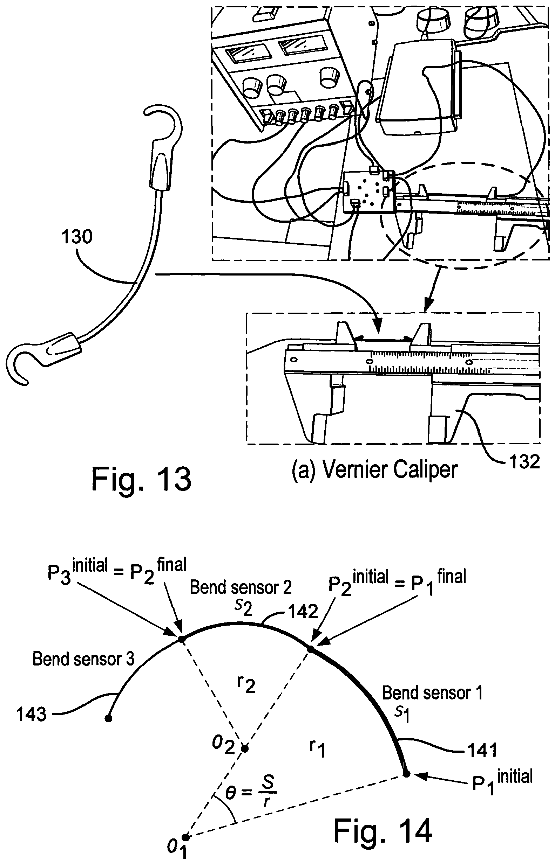

Referring to Fig. 13(a) and (b) the 30mm stretch sensor 130 was calibrated by stretching in 2 mm intervals using a Vernier calliper 132 until the stretched length reaches twice its original (unstretched) length. Data samples were obtained in the same way as for the bend sensors, i.e. with the DAQ 45, and a mean resistance determined for each 2mm interval. The mean resistance of the stretch sensor was plotted against stretched length.

*-Error correction based on included angle*

In use, each radius measured by a bend sensor corresponds to an included angle $\theta = s\kappa$. If working clockwise round the boundary the sum of the included angles should be -$2\pi$ radians (anti-clockwise would be a sum of +$2\pi$). A deviation from this represents the total angular error, which is corrected either by multiplying all estimated included angles by the ratio of their actual sum and -$2\pi$ radians or by subtracting the mean angular error that is, the difference of the sum of the angles and -$2\pi$, divided by the number of sensors, from each included angle.

As part of a method of estimating shape, this step of error correction may be performed before any co-ordinate generation. Below we describe various methods of estimating shape, as follows:

(i) a unidirectional algorithm;
(ii) a bidirectional algorithm; and
(iii) a derivative algorithm.

*-Sensor coordinates generation algorithm (uni-directional)*

A geometric rotational transformation algorithm was described by Starck *et al.* (1999) as modified below:

$$P_1^{initial} = O_1 + r_1$$

$$P_1^{final} = O_1 + \begin{bmatrix} cos(s\kappa_1) & -sin(s\kappa_1) \\ sin(s\kappa_1) & cos(s\kappa_1) \end{bmatrix} \left( P_1^{initial} - O_1 \right)$$

$$O_{i+1} = P_i^{final} - \kappa_i/r\kappa_{i+1} \left( P_i^{final} - O_i \right)$$

$$P_{i+1}^{final} = O_{i+1} + \begin{bmatrix} cos(s\kappa_{i+1}) & -sin(s\kappa_{i+1}) \\ sin(s\kappa_{i+1}) & cos(s\kappa_{i+1}) \end{bmatrix} \left( P_i^{final} - O_{i+1} \right)$$

where $O_i$ is the centre of the arc of each sensor, *i.* An initial starting point is chosen as the start of the first bend sensor,

$P_1^{initial}$. Subsequently, the final points $P_i^{final}$ of each sensor is obtained through *xy* plane rotation of each initial coordinate point about an angle $\theta = s\kappa_i$, where *s* is the length of the sensor and $\kappa_i$, the curvature of the $i^{th}$ sensor.

The effect of applying these equations is shown in Fig. 14. In particular, for a given arbitrary origin $O_1$, and a starting initial point, $P_1^{Initial}$ (which is the first starting point of the first bend sensor 141), the end point of the first bend sensor 141 can be determined based on the radius measured by the bend sensor, r and origin $O_1$ s shown in (1).

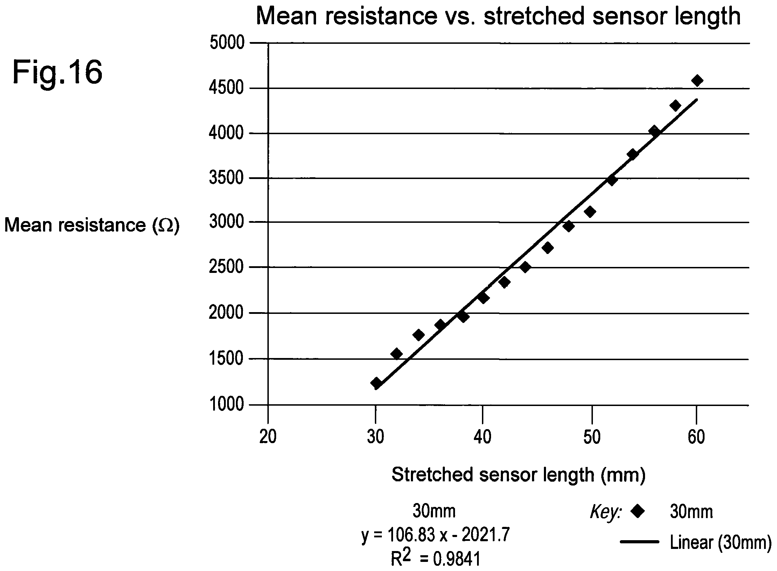

Subsequently, the final points $P_1^{final}$ of the first bend sensor 141 is obtained through x-y plane rotation of the initial coordinate point about an angle $\theta$= s/r, of the bend sensor 141 at its origin $O_1$, producing an arc length equivalent to the sensor length $s_1$. The new origin $O_2$ of the second sensor 142 arc is obtained from (3) (with i equal to 1) so that the points are continual (as depicted in Fig. 16) because all the sensors are joined continually. Thus, the final point of the first sensor is assumed to be the initial point of the second sensor and so on. By repeating this process for all bend sensors 141, 142, 143, a surface can be drawn in the chose co-ordinate system, preferably the Cartesian co-ordinate system.

For 2D boundary shape measurement and display of its geometric form, an algorithm was adopted and developed into Matlab programing code for transforming sensor outputs into two-dimensional Cartesian coordinate points (see Appendix A). The modified algorithm utilises the extracted bend sensor curvatures or radii and stretch sensor stretched length, and transforms these data into Cartesian coordinate points. These coordinate points provide a boundary shape of the measured shape when fitted with an appropriate curve or spline.

The following outlines the steps for generating a 2D shape:

1. Use linear regression models to convert sensor signal outputs into a radius value;
2. Generate initial and end coordinate points for first sensor through rotation of initial coordinate based on its radius value and known sensor length.
3. Set initial coordinate point of the next sensor to the end coordinate point of the first sensor. Determine end coordinate point for second sensor as per step 2. If a stretch sensor is joined by two bend sensors, its radius is assumed to be the averaged radii of its neighbouring bend sensors, and its length is determined from its resistance value using the respective linear regression model.
4. Repeat steps 2 and 3 until coordinates are determined for all bend sensors;
5. Fit arcs between each pair of sensor coordinates.
6. Interpolate a closed spline through all coordinates to form a shape based on coordinate positions and to provide an insight of the shape of real object shape.
7. Optionally, calculate errors in term of coordinate positional error (Euclidean distance) with respect to the total perimeter, and area error between the generated shape and true the shape.

• **First belt**

The calibration procedure described above was followed for the three bend sensors 141, 142, 143, and for two stretch sensors.

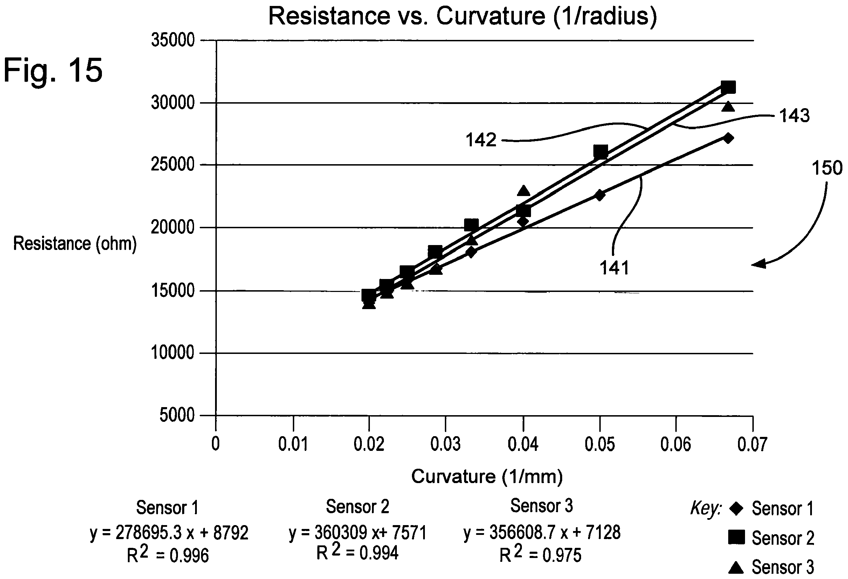

Fig. 15 is a graph 150 of curvature versus resistance for the three bend sensors 141, 142, 143. Linear regression fits have been made for each sensor, and the respective equation is shown just above the x-axis.

Fig 16 is a graph 160 showing length of the stretch sensor versus mean resistance (two stretch sensors were tested but only the linear regression fit for one is shown). A linear regression fit has been made to the data, and the equation is shown just below the x-axis.

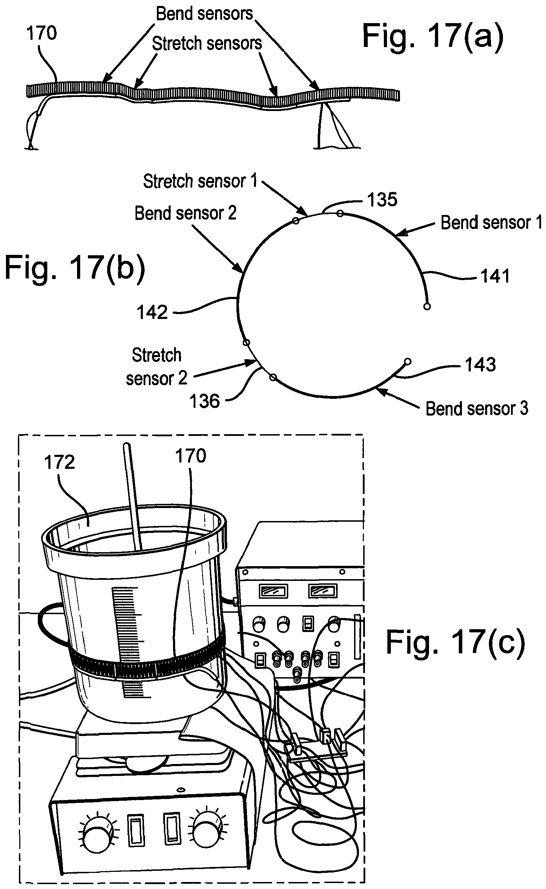

With reference to Fig. 17, after the calibration of each sensor, a belt 170 was constructed from the three full-length bend sensors 141, 142, 143, each 114 mm in length, and two stretch sensors 135, 136 each of 30mm in length, arranged in between the bend sensors. In use, the belt 170 was held in place with a short piece of elastic attached to each end of the belt. As shown in Fig. 17(a) the order of sensors was as follows: bend - stretch - bend - stretch - bend.

As shown in Fig. 17 (b), the belt 170 was then wrapped around a beaker 172 in order to test whether it is possible to estimate the shape of the beaker using the belt 170 and the aforementioned coordinate generation algorithm. The beaker 172 has a radius of 72.5 mm and was filled with water maintained at body temperature to simulate an *in vivo* measurement. The outputs of all sensors (bend and stretch) were sampled via the NI 6212 DAQ 45 simultaneously, and the mean resistance of each sensor was calculated and stored. The resistance value for each stretch sensor was converted to a length using the appropriate linear regression model, and the radius of each stretch sensor was calculated as the average of its two neighbouring bend sensors. These results were used in the sensor coordinate generation algorithm to generate a set of coordinates to which arcs were fitted. A visual representation of the surface was generated, although it is to be noted that this step would not be necessary for input into a forward model of and EIT reconstruction algorithm.

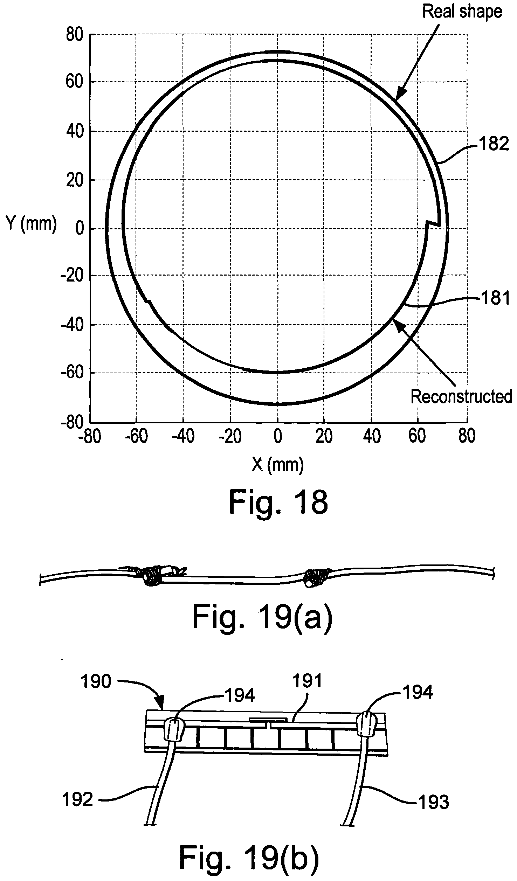

Recalling that the true radius of the beaker was 72.5mm, the radius calculated from the output of each bend sensor using its linear regression model was 68.6mm, 63.9mm and 64.9mm, with errors of -5.3%, -10.5% and -11.8% respectively. The linear regression model of the stretch sensor has an accuracy of ± 5%. The lengths of the stretched sensors calculated from their linear regression models based on their output while wrapped around the beaker were 34.6 and 33.2 mm respectively. The radius of each stretch sensor were assumed to be the average of the adjacent bend sensors' radii, joined at both ends of the stretch sensor. A two-dimensional boundary form of the beaker was generated using the above described algorithm program code and is shown in Fig. 18. The reconstructed shape 181 has a total perimeter error of - 10% compared to the true shape 182. This error is believed to be caused by different factors: individual sensor radius error, the accumulative errors of coordinate generation algorithm method, and measuring apparatus and insufficient in data points.

Although the error seems to be relatively high in these preliminary test results, it has established that it is possible to estimate the radius of the bend sensors through linear regression models based on the sensor outputs. A 2D boundary shape of a simple object can be generated using simple bend and stretch sensors. However, it has shown that with the bend sensor length of 114mm, the number of sensors and data points were limited, hence affecting the accuracy of generated shape.

**• Second belt**

The results from the first belt indicate that using the bend sensors as supplied (i.e. with a full-length of 114mm), the total number of bend sensors that can be used for measurement is limited. In turn this limits the accuracy of the shape generated. We suspected that an increase in bend sensor numbers with a shorter length would increase the number of measurement points, and that this might improve the uniformity of estimated shape.

We modified six original Abrams Gentile bend sensors into four 50mm long and two 25 mm long bend sensors. The modification to the sensor length was performed by cutting the sensor to the designated length and attaching carbon-wire to both ends of the conductive surface using conductive adhesive glue (obtained from http://www.smallbattery.company.org.uk/sbc_wire-glue.htm) without requiring soldering. Fig. 19 shows one of the modified bend sensors 190, comprising a portion of the original bend sensor 191, and two carbon wires 192, 193 attached to either end of the bend sensor 191 with dabs of conductive adhesive glue 194.

The calibration of these modified bend sensors and stretch sensors was performed as described above, but with each bend sensor supplied with a small constant current of 0.34 mA from a constant current source. The constant current is useful in order to maintain the stability and reliability of the sensor output and to condition the sensor output to within a range which is readable by the DAQ without additional signal amplification. The constant current was small and there was no significant heating effect in the bend sensors.

After obtaining and storing linear regression models for all the modified sensors, a second belt was fabricated with the bend sensors arranged in series as follows: 50 mm, 25 mm 50 mm, 50 mm, 25 mm and 50 mm followed by the stretch sensor (20mm) that can be stretched up to double its original length. This gives the second belt the flexibility wrap around any object with a circumference within the range from 270 mm to 290 mm (which approximates an average chest circumference range for newborn infants). For simplicity of the initial error analysis, the belt was tested on a cylindrical circular object where methods for improving sensor coordinate points generation algorithm could be developed and error correction factor for the individual sensor can be determined.

The belt was wrapped around a cylindrical object with a radius of 45mm and the data acquisition was carried out and processed as described in connection with the first belt. Simultaneously, outputs from the sensors were checked the Keithley 191 digital multimeter. The radius of each individual bend sensors o the second belt was determined from its linear regression model using the bend sensor's averaged (mean value) voltage or resistance. Subsequently, with the geometric profiles (radius, angle and sensor length) of all bend and stretch sensors, the sensor coordinates generation algorithm was used to generate a series of Cartesian coordinate points. These coordinates were connected through fitting of simple arcs, forming the measured shape in two-dimensions. The error of the sensor coordinates generation algorithm was examined in terms of coordinate positional error by comparing the generated shapes to the true object shape. The coordinate positional error ε is defined by the Euclidean distance between generated coordinate and true coordinate point on the boundary of object shape, calculated from the equation below, based on a least-square formula:

$$\varepsilon = \sqrt{(X_i - x_i)^2 + (Y_i - y_i)^2}$$

The results from coordinate positional error analysis are used to determine an optimal error correction factor for all bend sensors' radii, so that a reasonable 2D shape can be generated.

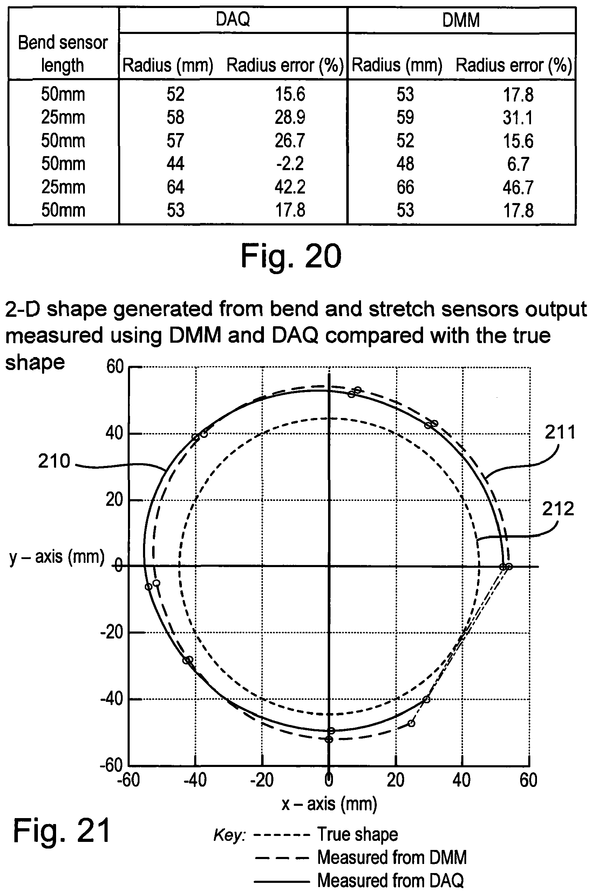

Fig. 20 is a table comparing the radii determined from the outputs of the modified bend sensors gathered using (a) the DAQ 45 and (b) the digital multimeter respectively. The percentage error compared to the true radius of the circular cylinder is also presented in the table. We observed that both bend sensors with a length of 25mm produce greater sensor radius error compared to the 50mm bend sensors; this suggests that a sensor with shorter length carries increased error. This error is believed to be caused by sensor output measurement noise owing to the use of the conductive glue which has an area of contact with the surface of the bend sensor. Fig. 21 shows the estimated 2D shape of the cylindrical object generated from the coordinate generation algorithm (a) from measurements made by the DAQ 45 (reference numeral 210), and (b) from measurements made with the digital multimeter (reference numeral 211), compared to the true shape 212. We found that the error difference between the generated shapes using both DMM and DAQ measuring methods is insignificant. However, both generated shapes have approximately 15% circumference error with respect to the true perimeter.

*-Total sensor error compensation factor*

This circumference error suggests that the shapes generated purely from sensor outputs using current coordinate generation algorithm do not approximate the real shape of the object. We suspect that this is due to the high sensor error as shown in Fig. 20. In contrast the errors associated between the two measurement methods (DAQ and multimeter), and the coordinates algorithm itself, are insignificant when compared to the high sensor error. To address this problem we propose a total sensor error compensation factor (of between 0.81 and 0.96), 'TCF', for modelling of the algorithm. A compensation factor can be generated for each sensor by using the mandrel to measure known radii. The result generated from the linear regression model for that sensor can be compared to the known radius of the mandrel. From that comparison a compensation factor can be determined, i.e. the compensation factor is that value which the calculated radius must be multiplied by in order to obtain the correct radius value. The TCF can be obtained for all of the sensors by determining the mean value of the individual compensation factors. It is to be noted that the TCF can be used in combination with the error correction based on included angle described above.

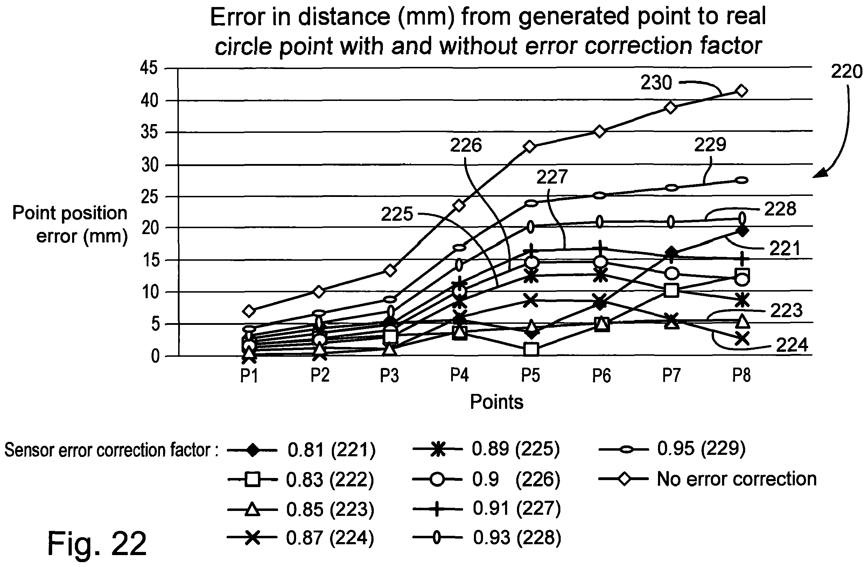

Fig. 22 is a graph 220 that illustrates the effect with and without TCF on the position error of the each generated coordinate point compared to the true position. The position error (y-axis) is shown cumulatively around the circumference for generated coordinates P1 - P8 (x-axis). The position error is the Euclidean distance between real coordinates and generated coordinates that represents the error of the generated shape with and without sensor radii correction. A TCF of 0.85 (reference number 223) was found to produce the minimal error for all coordinate points; this confirms the 15% radius error as previously demonstrated.

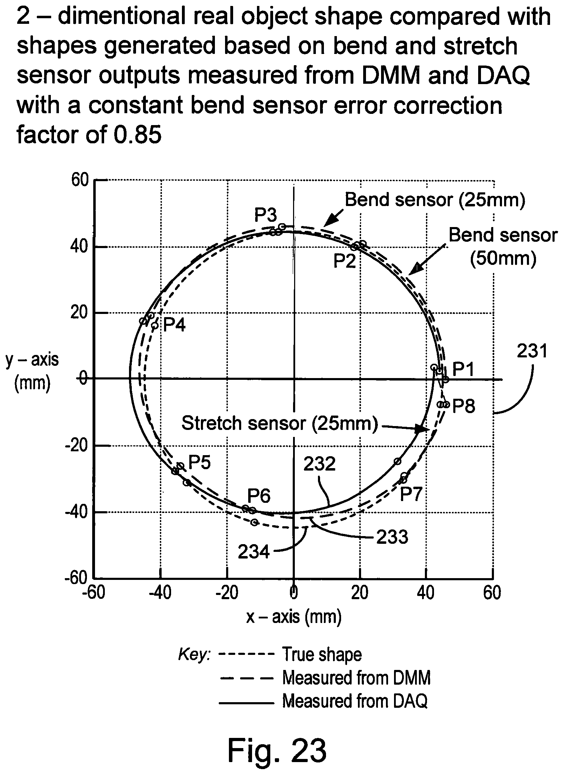

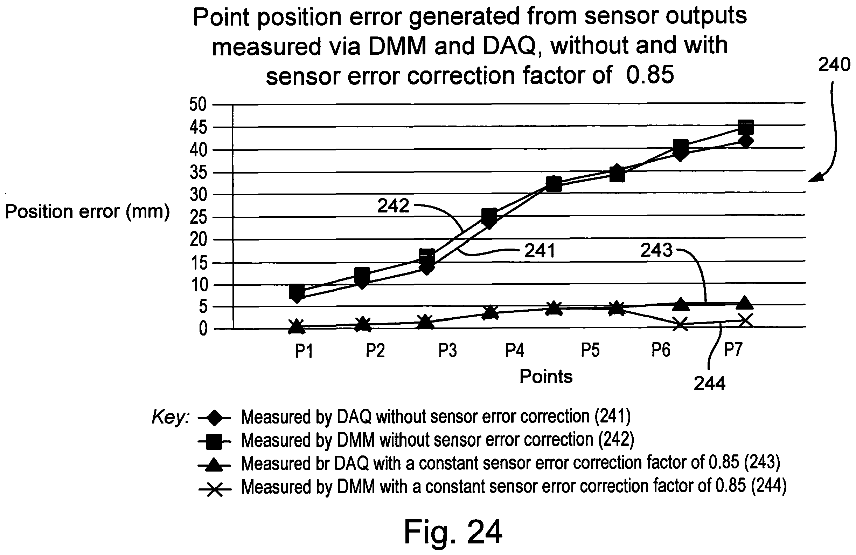

Referring to Fig. 23, a graph 231 shows estimated shapes generated with 0.85 TCF based on measurements made from both the NI USB6212 DAQ 45 (reference number 232) and Keithley 191 digital multimeter (reference number 233) compared with the true circular shape (reference number 234). As a result, the generated shapes have been improved to approximately 1% in perimeter error. A comparison graph 240 of coordinate positional error for each coordinate point, with and without sensor radius correction factor of 0.85, is shown in Fig. 24. With the introduction of 0.85 or -15% TCF to calculated sensor radii values, the maximum coordinate positional error improves to less than 6mm.

## • Third belt

A similar process of sensor length modification and calibration as described in relation to the second belt was carried out during this experiment. A third belt was fabricated from four 60 mm bend sensors and one 15mm stretch sensor. The 60mm bend sensors were divided into two separate 30mm sensor outputs via the attachment of a wire using the aforementioned conductive adhesive midway along the length of each bend sensor. As a result, eight bend sensors each with length of 30mm were produced. The stretch sensor connected between the first and the last bend sensor. This third belt was tested on an anatomically realistic baby doll with a chest circumference of approximately 260 mm (close to the chest size of a newborn premature infant). A baby doll was used instead of conducting an experiment involving a real baby because of difficulties in obtaining ethical approval.

The third belt was located at the nipple line. All bend sensor outputs were measured and sampled using the DAQ 45, except for the stretch sensor output. This is due to the limited number of input channels on the NI USB6212 DAQ (there are only eight input channels available for sensor voltage differential measurements). The output from the stretch sensor was taken using the Keithley 191 digital multimeter.

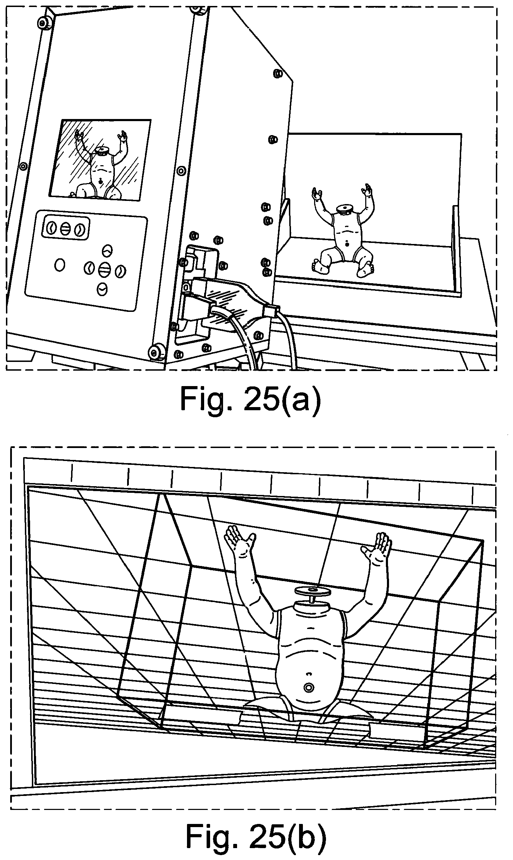

In order to check the boundary shape data obtained from data produced by the third belt, the baby doll was scanned with a 3D scanner to obtain a computer image of the shape of the thorax. Referring to Fig. 25 a Konica Minolta 3D digitizer 250 (from Konica Minolta Sensing Singapore Pte Ltd) was used on the baby doll 252 to generate a 3D computer model 254 (see Fig. 25 (b)) of the surface of the doll. The Konica Minolta 3D digitizer (Model: VI 9i) is a non-contact scanner that employs the triangulation light block method to acquire 3D surface data points of a 3D object. It has an (x,y,z) coordinate accuracy of $\pm 0.05$ mm (with field calibration system) and precision (z,$\sigma$) of 0.008mm using TELE lens at distance of 0.6 m at temperature 20°C. The 3D surface data generated by the 3D scanner 254 was transformed into 3D computer model 254 using Rapidform 2006 software.

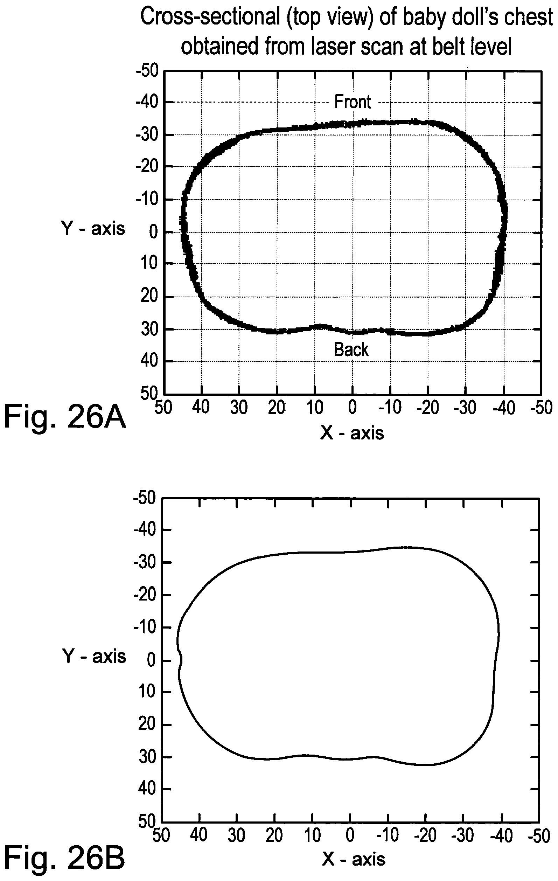

Using CAD the 3D surface data measured at the doll nipple level was extracted from this 3D computer model and transformed into a 2D boundary shape 260 (see Fig. 26 (a)). This boundary shape 260 was used as a 'gold standard' for determining error of boundary shape generated from the third belt. Matlab programming was used to obtain a cubic interpolation of the boundary shape 260 with the Matlab function 'csapi'. The closed cubic spline 262 is shown in Fig. 26 (b). The area enclosed by the spline is 4873.4mm$^2$

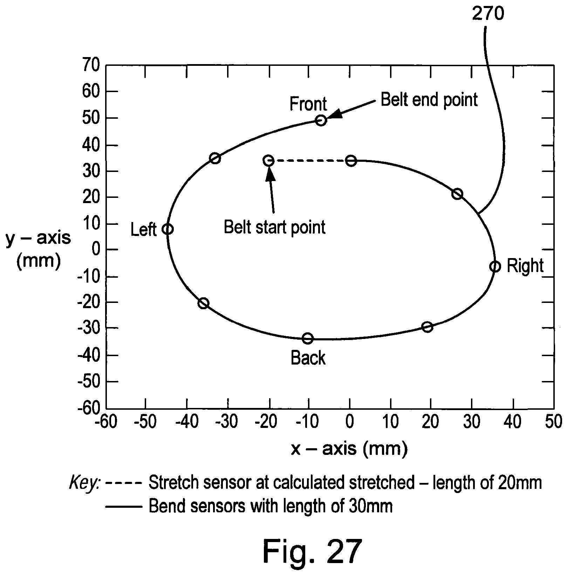

Fig. 27 shows a 2D shape 270 of the doll at nipple level generated from the third belt using the coordinate generation algorithm detailed above. This result shows the limitation of the coordinate generation algorithm in approximating a complex shape. In particular, the first coordinate of the first sensor and last coordinate of the last sensor do not coincide due to the accumulated coordinate positional error caused by the algorithm method and the initial sensor error. We realised that when coordinate points of bend sensors were generated consecutively (where series of coordinate points were obtained in sequence from the first sensor until the last sensor), there will be accumulated error. So we considered how the coordinate generation algorithm could be adjusted to generate better estimates of more 2D shapes more complicated than a circle.

-Sensor coordinates generation algorithm (bi-directional)

To improve the reconstruction of the boundary form from bend and stretch sensor data, we devised a different approach for the rotation of Cartesian coordinate points and identification of stretch sensor location. This approach was analysed and compared with the gold standard reference boundary shape in terms of coordinate positional distance and area size error.

In order to minimise the accumulated error and improve the resulting estimate, we devised a method that enables generation of sensor coordinates using information about the sequence of bend sensors and stretch sensor on the belt. In particular, the stretch sensor was placed on the anterior of the thorax where its curvature minimal and can be assumed to be flat, or a lying along a straight line. The stretched length is obtained from the measured resistance and the linear regression model. Once the stretched length is known the respective initial point of each neighbouring bend sensor can be determined in the coordinate system. Thus there are now two initial points. The algorithm then operates to rotate the coordinates as described above, but working clockwise from one of the initial point and anti-clockwise from the other initial point.

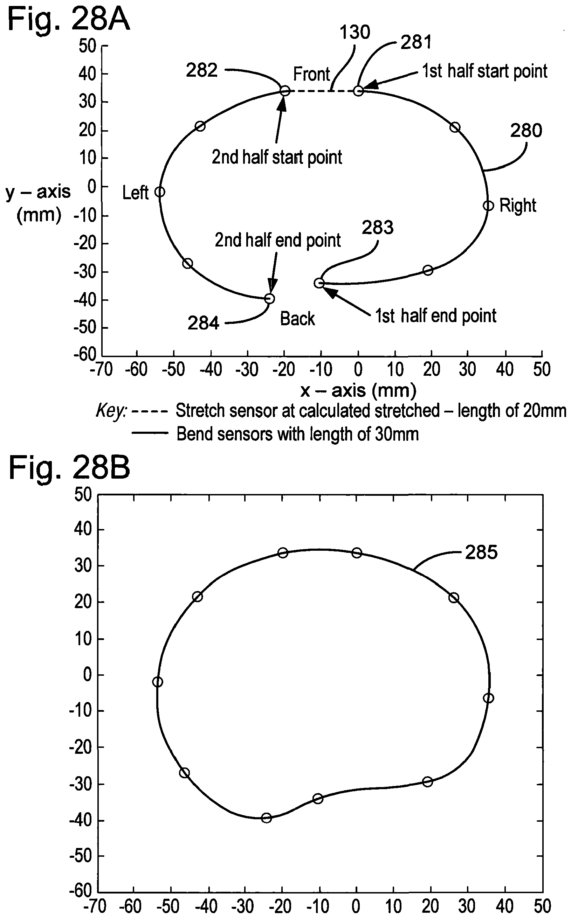

Fig. 28A shows the 2D estimated shape 280 using this bi-directional version of the coordinate generation algorithm. The stretch sensor 130 is at the top of the shape is generated as a straight line having a length equal to the measured length on the baby doll. A first half starting point coordinate 281 is the beginning of the clockwise operation of the coordinate generation algorithm which works around to a first half end point coordinate 283. A second half starting point coordinate 282 is the beginning of the anti-clockwise operation of the algorithm which works around to a second half end point coordinate 284. Even though the first and second half end point coordinates 283 and 284 do not meet, it is evident that the adapted algorithm provides an improvement in coordinate point generation. It is to be noted that no total sensor error compensation factor (TCF) was used in generating this 2D estimate. The generated shape 280 shown in Fig. 28A has an end-coordinate (between both end coordinate points) distance error of 14.9 mm, and a circumference error of 5.7% in the worst case scenario.

A closed spline 285 was interpolated around the coordinate points of the 2D shape to form a boundary representation of the thorax of the baby doll (see Fig. 28B). This representation of the 2D shape is useful for computer manipulation. In order to produce a smooth closed spline which interpolates through all coordinate points, the Matlab cubic spline interpolation function 'csapi' was used. The size area of the generated form enclosed by the cubic spline is 5187.5mm$^2$ which amounts to an error of approximately 6.5% with respect to the area of the 'gold standard' reference shape.

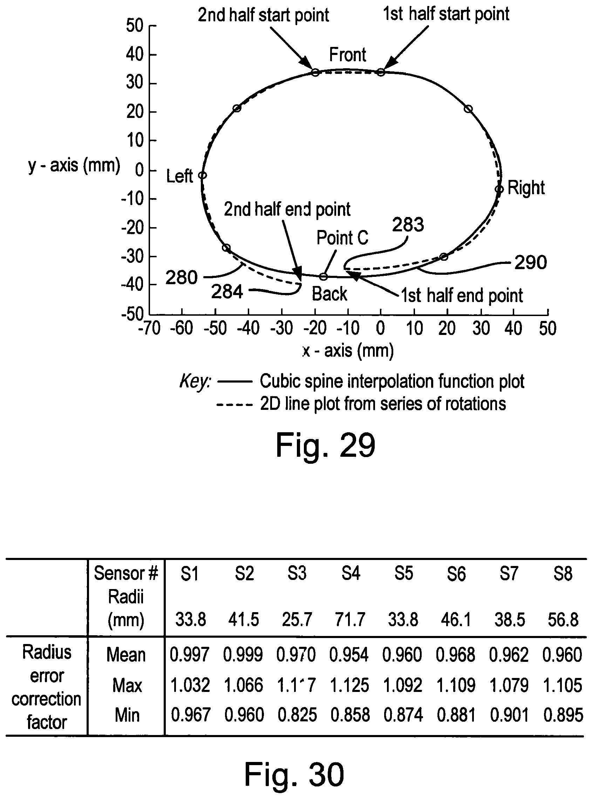

As mentioned above, since the bend sensor errors have not been corrected with TCF, the final end coordinate points do not match. However, we suspected that even if TCF is used, it is quite unlikely that the two ends would meet. This problem can corrected by introducing a new coordinate point, C, which is set at midway between both end points (see Fig. 29) so that a smooth spline 290 can be fitted. The spline 290 shows a significant improvement to the generated shape and a closer approximation of the true shape can be achieved provided that other error sources mentioned earlier are alleviated or taken into account.

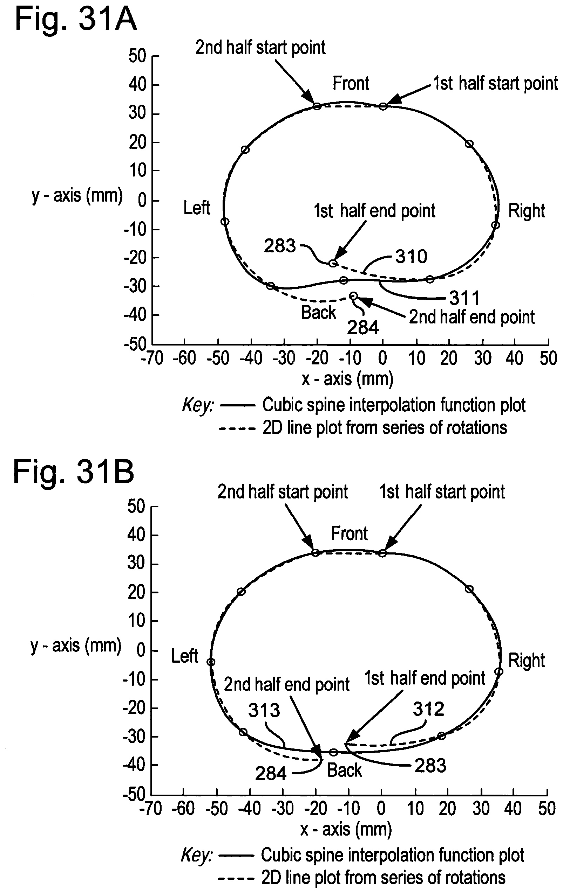

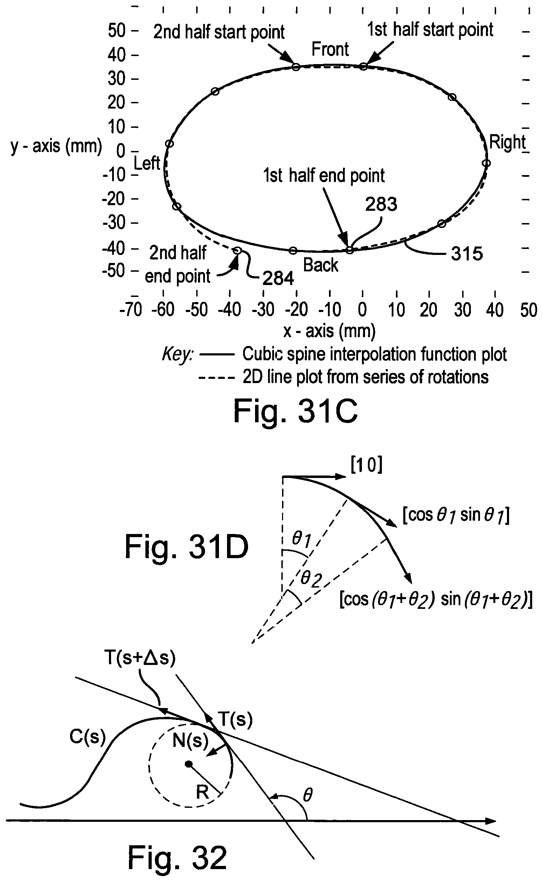

In particular, further analysis of 2D shape generation using the third belt was performed but taking into account individual sensor radius measurement error. The sensor radius error correction factor (minimum, mean and maximum) obtained from the previous section was applied to the radius measured by each sensor (see Fig. 30). Fig. 31A shows the 2D estimated shape 310 and cubic spline interpolation 311 using minimum error correction factor for each sensor; Fig. 31B shows the 2D estimated shape 312 and cubic spline interpolation 313 using mean error correction factor for each sensor; and Fig. 31C shows the 2D estimated shape 314 and cubic spline interpolation 315.

When the minimum, mean and maximum individual sensor radius error correction factor (based on Fig. 30) were introduced into the 2D shape generation algorithm, the distance error between the two end points 283, 284 was 12.9 mm, 9.2 mm and 33.7 mm respectively. The total area and error in the total area between the closed spline fitted to each generated shape and the true shape of the baby doll was: minimum error correction factor, 4302.6mm$^2$ (11% error), mean error correction factor, 4949.4mm$^2$ (1.6% error), and maximum error correction factor, 5908.2mm$^2$ (21% error).

This indicates that the mean sensor radius correction factor seems to provide a more accurate boundary shape when compared to the other two error correction factors. It also shows that by introducing a maximum radius error correction factor into each sensor, the distance error between end points 283, 284 worsens (33.7 mm) compared to no correction factor being used at all (14.9 mm distance error). This indicates that an appropriate and optimal error correction factor needs to be predetermined and justified before introducing sensor error correction into the shape generation algorithm. By doing so, error correction technique can be incorporated into the modified algorithm in the future when accurate sensors with known error are available.

In summary, the above results show that a useful two-dimensional shape of an anatomically correct baby doll's chest boundary (at the nipple line) can be achieved with the third sensor belt, with a total shape error of less than 6% with respect to the total perimeter. This was achieved by using the following factors:

a) a bi-directional sensor coordinate generating algorithm combined with fitting a smooth spline (e.g. using Matlab);
b) an appropriate mean error correction factor for each bend sensor; and
c) a suitable number of bend sensors (eight bend sensors in this case) plus one stretch sensor;
d) arranging the stretch sensor on a relatively flat part of the body (e.g. front or back of thorax).

It was also found that the initial sensor location was required, and that the arrangement of stretch sensors on the object boundary would greatly affect the shape errors. This is because the sensor coordinates generation algorithm is prone to sensor radius accumulated error. The stretch sensor can only provide displacement information, while its radius was determined from the average of two adjacent bend sensors' radii. We found that it is beneficial for the location of stretch sensor to be placed on a relatively flat area of the body part to be inspected, such as the front or back of the thorax. This factor was not as significant for a cylindrical object since its radius remains constant around its boundary. When using the belt on an object with an arbitrary shape, like the thorax shape of an average newborn infant, the belt should always be placed in the same location (e.g. with the stretch sensor on the front or back of the thorax). This ensures that initial starting point is always the same for the coordinate generation algorithm; the length of the stretch sensor can be determined first, and the two initial starting points can be set in the coordinate space.

*-Sensor coordinates generation algorithm (derivative method)*

As described above, it is possible to calculate each included angle from the radius measured by each bend sensor. Having evaluated all angles, $\theta_i = s\kappa_i$, and applied the appropriate error correction, the gradient of the first point in the curve is the initial condition [1 0], representing a horizontal vector. The gradient at the end of each sensor, *i,* is therefore

$$\left[ \cos\left(\sum_1^i \theta_i\right) \quad \sin\left(\sum_1^i \theta_i\right) \right]$$

Fig. 31D illustrates the process of generating *i* points. These values are used to interpolate a degree *n* B-Spline. This curve is integrated over its length to generate a degree (*n*+1) B-Spline which, when multiplied by the known perimeter, forms the reconstructed curve.

## 5. Bend sensor modelling

Despite the results in previous section showing a degree of uncertainty and error in shape estimate, we have found that the Abrams Gentile bend sensors might be useful in an apparatus for measuring body shape, such as a wearable device which could take the form of a belt.

The experimental data reported in the previous section shows that it is possible to estimate a reasonably accurate 2D shape of a non-uniform body using the modified algorithm, with some errors. These errors are believed to stem from the inability of current bend sensor technology to provide outputs with high accuracy and stability, the limitation of measuring apparatus, and the sensor coordinate generation algorithm. Accordingly, the uncertainty and error in the current design may therefore be overcome if higher accuracy sensors become available, and with the use of more sophisticated measuring apparatus and robust generation algorithms.

This section focuses on:

(a) testing the robustness of the proposed sensor coordinate generating algorithms; and
(b) the development of a modelling algorithm for determining the optimal number of sensors with minimal error.

To address (a), the robustness of coordinate generation algorithm was assessed by examining what error is induced, if any, when it is used to model ideal shapes such as a circle and ellipse. The modelling was performed in three scenarios:

1) Model a circle of known radius using the algorithm based on minimum number of segments and radius, which segments are equivalent to a number of bend sensors and their radius.
2) Model an ellipse, whose shape and total arc length approximate the shape and circumference of a premature infant thorax respectively.
3) Model of a real thorax shape of an infant aged one-year-old based on shape information obtained from a CT scan.

All modelling and tests were carried out using the Matlab programming language. Before performing this modelling, the geometric properties used in the modelling are defined in following sections.

*-Curvature*

The two geometric properties used in this modelling were the curvature and the length of a particular arc segment, analogous to the individual bend sensor curvature and length respectively. In general, curvature is a measurement of the properties of a curve, spline or surface plane at a particular point, for instance in a plane view, a straight line has zero curvature, a curve that bends slowly has a low curvature while a small circle or sharp bending curve possesses high curvature value. A cross-sectional view of the bend sensor plane when bent resembles an arc that possesses a curvature property; in contrast it has zero curvature when it is flat. In the following, the curvature, $\boldsymbol{k}$, of the bend sensor is presumed as the inverse of radius, $\boldsymbol{k} \equiv \dfrac{\boldsymbol{1}}{r}$, where $\boldsymbol{r}$ is the radius of the bend sensor when bent. In the case where a bend sensor is of a uniform curve or arc, the arc length is equivalent to the sensor's length and there is a uniform curvature value along the arc.

The mathematical definition of curvature, $\boldsymbol{k}$ at a point of a smooth parameterized curve, $\boldsymbol{C(t)}$ in two-dimensions is the rate of change of direction of the tangent line on the curve with respect to a variable, $\boldsymbol{t}$ which can be arc length, time or angle. From this definition, let the centre line along a bend sensor plane in a bent position be analogous of a smooth curve, $\boldsymbol{C(s)}$, parameterized by its length s (see Fig. 32). The curvature $\boldsymbol{k}$ of a point $\boldsymbol{C(s)}$ with respect to the sensor arc length can be determined in several different ways, as follows:

a) Change of the slope angle of the tangent line measured in counter clockwise relative to the positive x-axis, given by the following equation:

$$k(s) = \frac{d\theta(s)}{ds} = \frac{dT}{ds} \qquad \text{(i)}$$

where $\theta\boldsymbol{(s)}$ is slope angle measured counter clockwise from the x-axis to the unit tangent.

b) Second derivatives along the curve/spline using the following equation:

$$k = \frac{x'y'' - y'x''}{(x'^2 + y'^2)^{3/2}} \qquad \text{(ii)}$$

where $\boldsymbol{x(s)}$ and $\boldsymbol{y(s)}$ are the parametric functions with respect to arc length $s$.

25

c) Curvature at position **C(s)** can also be determined from the unit tangent vector **T(s),** unit normal vector **N(s)** or radius **r(s)** of the osculating circle based on the following equations:

$$T = \frac{C'}{\|C'\|} = (x', y') / \sqrt{x'^2 + y'^2} \qquad \text{(iii)}$$

$$N = (-y', x') / \sqrt{x'^2 + y'^2} \qquad \text{(iv)}$$

The sign of the curvature $k$ indicates the direction in which the unit tangent vector rotates as a function of the parameter along the curve.

Curvature information used for modelling in this thesis was determined using method (b) above because parametric functions (x(t) and y(t)) were used in plotting the circle and ellipse.

*-Assumptions*

In modelling bend sensor coordinate generation in an ideal scenario, the following assumptions were made:

a) A uniformly bent sensor is equivalent to a circular arc.
b) All bend sensors produce accurate curvature /radii information which corresponds to their physical bending profiles.
c) Each bend sensor is short enough that the radii of all the points on the plane are constant along its length when bent.
d) All bend sensors are joined and arranged in series.
e) There is no error in sensor and measuring apparatus.

*-Circular form*

The uni-directional version of the sensor coordinates generation algorithm as described above was tested on a cylinder with a radius of 45 mm in order to examine its robustness. A cylinder is used as a mean and standard measure for modelling the robustness of the above-mentioned algorithm because the radius of cylinder is constant, resembling an ideal sensor scenario with no error. Hence, any error shown in the generated shape may be assumed as error due to the algorithm itself.

All of the following procedures were developed in Matlab programming codes in order to test the robustness of the sensor coordinate generation algorithm (see appendix B). The systematic procedure is described in details as follow:

1. A circle with a known radius of 45 mm was constructed using the parametric functions:

$$x\,(t) = a * \cos(t) \qquad \text{(A)}$$

$$y(t) = b * \sin(t) \qquad \text{(B)}$$

where $0 \le t \le 2 * \pi$; and a=b=45 mm

2. The total circumference of this circle defined as:

$$S(t) = \int_{t=0}^{t=2\pi} \sqrt{x'(t)^2 + y'(t)^2} \; dt \qquad \text{(C)}$$

was calculated using the Matlab 'Quad' function, which is an adaptive Simpson quadrature for evaluation of an integral numerically within an error of $10^{-6}$.

$$\mathrm{S = integral(@(t)sqrt((a.*cos(t)).^2 + (b.*sin(t)).^2), 0, 2*\pi)} \qquad \text{(D)}$$

3. Once the circumference for the given radius was obtained, it was divided equally into a minimum number of segments, n. Four segments were used in this case in order to manifest the worst-case scenario, where each segment length is the length for each ideal bend sensor, L.

$$L = S/n \qquad\qquad (E)$$

4. The segment length, L defined from step 3 was used to calculate the parametric, t for a given L using the Matlab's 'FMINSEARCH' function, which is multidimensional unconstrained nonlinear minimization (Nelder-Mead) function that is able to find a local minimizer of the scalar arc length function S(t) and L defined by the number of segments. See Appendix B for the code using this FMINSEARCH function.

5. Once the parametric values (t) were obtained, the new initial and final coordinate points of each segment can be determined using (A) and (B), based on information of each segment length and value t.

6. Steps (3 to 5) were repeated but with new number of segments, N = 2n in order to find the midpoint coordinate of each original segment on the constructed circle, and its curvature, K, as the mean curvature of the ideal bend sensor using modelling assumptions (b) and (c) above. The curvature is calculated using equation (ii) but based on x(t) and y(t) parametric functions of the midpoints of all the segments, thus, giving the radius of each ideal sensor as $r_i = 1/K_i$, where $i$ is the index number starting from one to the total number of segments. In this case, $i$ is from one to eight.

7. The calculated radii $r_i$ for all sensors were used in the sensor coordinate generation algorithm in order to construct a series of coordinate points. Following that, circular arc segments were fitted through the all generated coordinates.

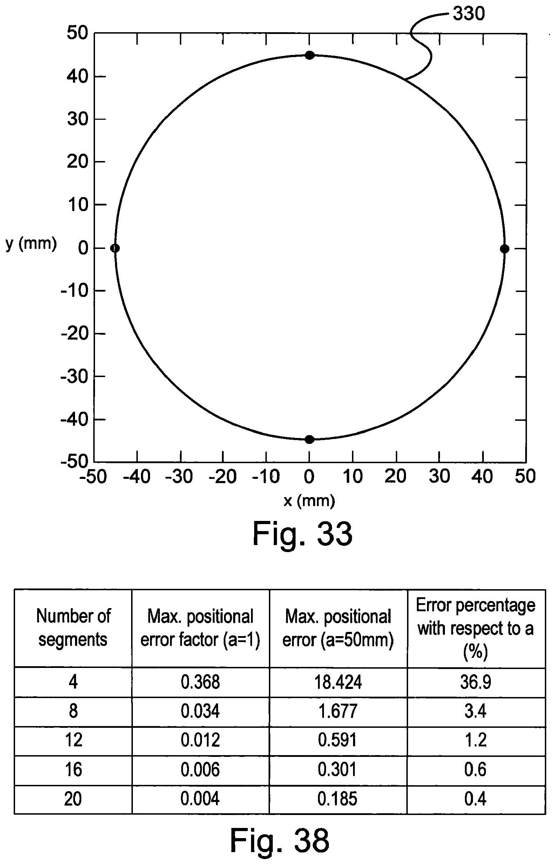

Fig. 33 shows the shape 330 generated from the above procedures (1-7) based on the calculated radii for the sensor segment and their length. As can be seen the reconstructed circle coincides with the true circle (radius of 45 mm), even though only four equal segments were used. We conclude that the sensor coordinate generation algorithm works well for generating a circular shape and by implication is robust in reconstructing circles of different radius and number of segments.

*-Elliptical form*

The modelling procedures (1-7) as detailed above were repeated but for testing on an ellipse of various semi-minor to semi-major b/a ratio values for a range of segments, numbering between four and twenty.

The aims of this ideal sensor scenario modelling (assuming no errors in sensor radius) using various ellipse are:

1) to establish an optimum working range of the sensor coordinates generation algorithm;
2) to compare maximum positional error on generated shapes modelled using a range ellipse b/a ratios for different number of segments used and,
3) as an initial reference to decide on optimum number of sensors to use for measuring the thorax boundary of any neonate with chest depth-to-width ratio within the b/a ratio.

The range of b/a ratio was set in a way that the value of semi-minor, a in x-axis fixed at unit factor 1 and semi-major, b in y-axis varies from 0.2 to 2.4. The purpose of using b/a ratio (factor) was to enable determination of the difference in distance between the generated and true coordinates, in the forms of both factor unit and position (mm) errors for any given x- and y-axis metric values.

A positional error factor $d_i$ between each generated point and the corresponding actual point on the true ellipse can be defined as follows:

$$d_i = \sqrt{(X_i - x_i)^2 + (Y_i - y_i)^2} \qquad\qquad (1)$$

where $[X_i, Y_i]$ and $[x_i, y_i]$ are the coordinates of the true ellipse and generated ellipse respectively, at the $i^{th}$ point. Thus the mean error $d_{mean}$ is calculated using

$$d_{mean} = \frac{\sum_{i=1}^{n} d_i}{n} \qquad\qquad (2)$$

where *n* is the number of points generated.

Errors can be further analysed by expressing each $d_i$ as a factor of the major semi-axis, *a*, such that

$$d_{f,i} = \frac{d_i}{a} \qquad (3)$$

The relationship of the errors can therefore be examined in terms of error factor $d_f$ with respect to the aspect ratio of the ellipse (*b/a*). As will be explained below, the point with the largest positional error is also of interest and this will be referred to as the maximum positional error factor $d_{f,max}$.

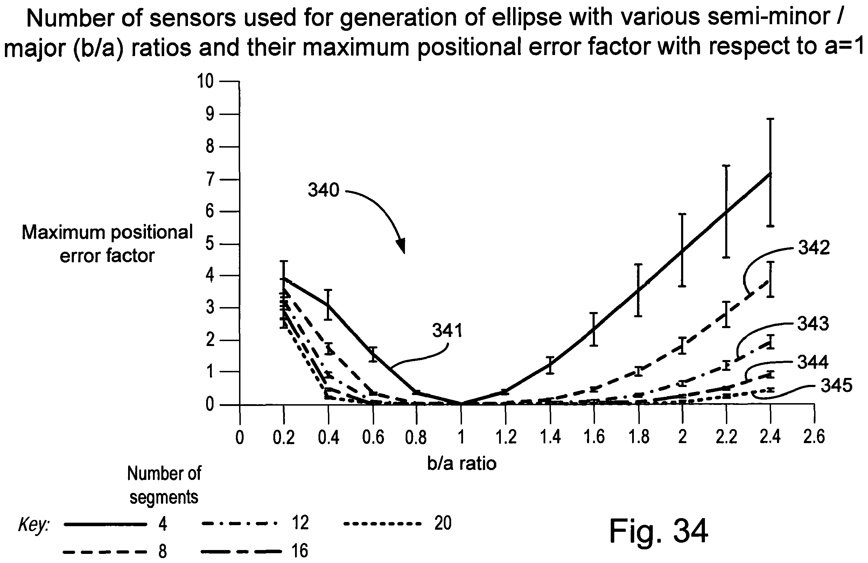

Fig. 34 is a graph 340 showing how the maximum positional error factor $d_{f,max}$ varies with semi-major/minor, or b/a-axis ratio (where the a-axis is 1 and the b-axis varied from 0.2 to 2.4). The graph has been produced for a number of different segments (i.e. number of bend sensors) around the ellipse: four segments 341, eight segments 342, twelve segments 343, sixteen segments 344 and twenty segments 345.

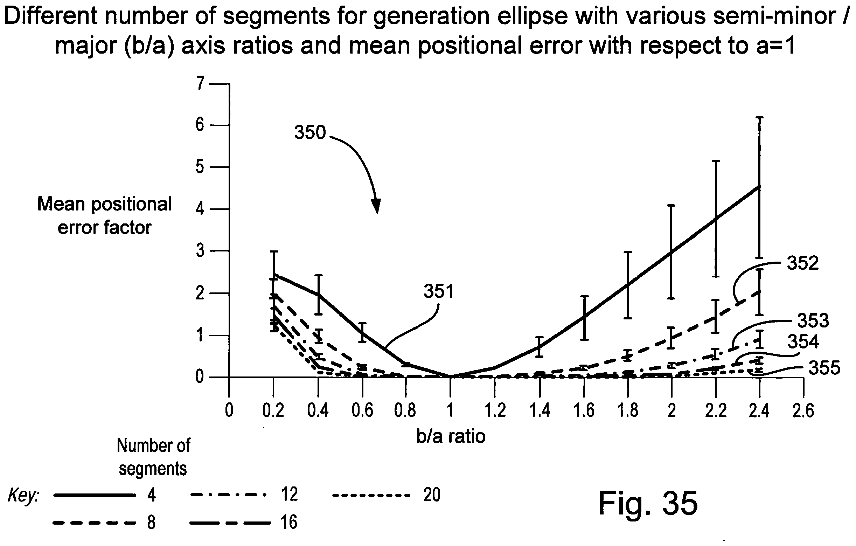

Fig. 35 is a graph 350 showing how the mean positional error factor $d_{mean}$ varies with semi-major/minor, or b/a-axis ratio (where the a-axis is 1 and the b-axis varied from 0.2 to 2.4). The graph has been produced for a number of different segments (i.e. number of bend sensors) around the ellipse: four segments 351, eight segments 352, twelve segments 353, sixteen segments 354 and twenty segments 355.

The number of segments used in modelling is an analogous to the number of sensors used in an apparatus for estimating shape, such as a belt. Accordingly, this modelling process can be used to determine the minimum number of bend sensors that is required to estimate a shape with a positional error within a predetermined threshold. Figs. 34 and 35 confirm that maximum and mean positional error factors decrease with an increase in the number of segments used across all b/a ratios. This means that error can be reduced significantly by increasing the number of sensors used. However, the number of bend sensors used has to be practical having regarding to implementation in the clinical environment, taking into consideration the physical thorax size, the increased cabling and data processing requirements with increasing number of sensors, and the limitation of bend sensor length. There is a further balance to bear in mind: the number of sensors used is a trade-off with sensor length; for a thorax of certain length perimeter, a greater number of bend sensors means that each bend sensor must have a shorter length. A bend sensor of shorter length has increased stiffness, and reduces the ability of the bend sensor to conform to the shape of the subject.

Therefore it is suggested that the fewest number of bend sensors are used that keeps the positional error under a predetermined threshold.

The predetermined threshold can be set accordingly to implementation requirements. The mean positional error factor $d_{mean}$ or the maximum positional error factor $d_{f,max}$ could be used to set the predetermined threshold. In the example below we have chosen to use the maximum positional error factor $d_{f,max}$ as this represents a worst-case scenario. Whilst it is apparent from Fig. 34 that by increasing the number of segments $d_{f,max}$ can be reduced in magnitude over an increasing b/a ratio range, regard must be had to the practical implications of greater numbers of segments (and therefore bend sensors). With that in mind, 12 segments seemed to offer the necessary balance between accuracy and practical complexity.

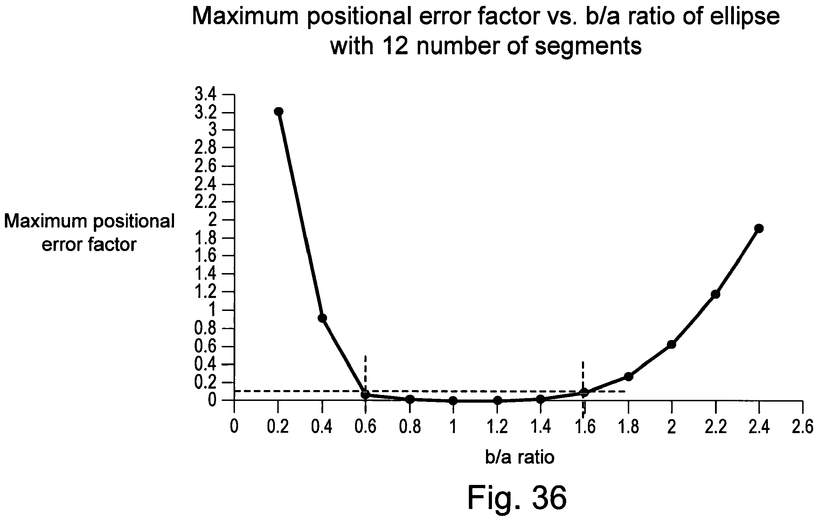

The curve of maximum positional error factor $d_{f,max}$ for 12 segments is shown in greater detail in Fig. 36. Fig. 36 shows that there is a range of b/a ratio where $d_{f,max}$ is very low, in this case below 0.1. The corresponding b/a ratio is between about 0.6 and 1.6.

Next we looked at data to identify an ellipse that closely resembles the chest dimensions of new-born babies. From data collected from Great Ormond Street Children Hospital (GOSH), the average chest circumference, width and depth of 15 new-born neonates aged between 1.71- 11.71 weeks, was found to be 301±36mm, 102±15mm, and 80±17mm respectively. Based on this data, the approximation of neonate size using an ellipse gives semi-major, a, and semi-minor, b, axes of approximately 50 mm and 40 mm respectively; an equivalent b/a ratio is 0.8. Further error analysis was performed based on this b/a ratio, and the results showed that the maximum positional error factor for 12 segments is 0.011, meaning that for an ellipse with semi-major a-axes of 50mm, the maximum coordinate positional error is approximately 0.6 mm with respect to the a-axis. This error is relatively small: 0.12% of the length of the semi-major axis.

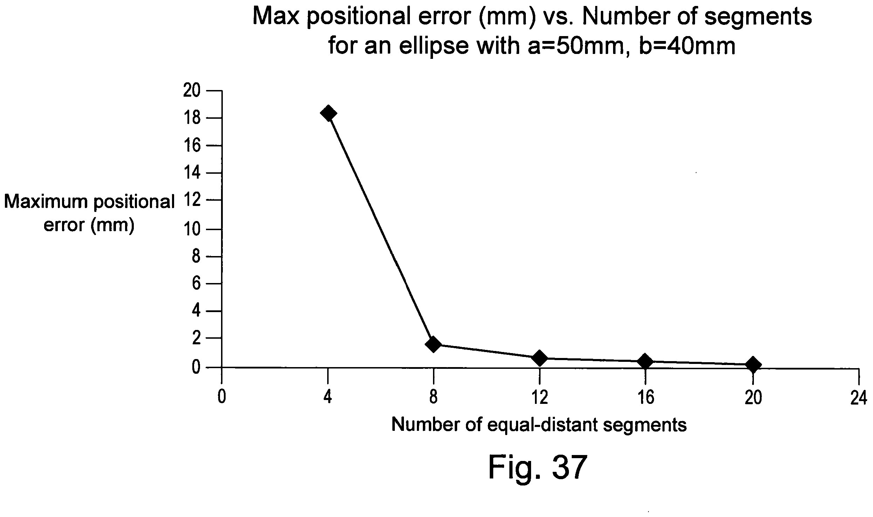

We then examined what effect different numbers of segments (between 4 and 20) would have on the maximum positional error factor $d_{f,max}$ when fitting arcs around an ellipse with a b/a ratio of about 0.8 (i.e. approximately the cross-sectional shape of a new-born infant between 1.71 and 11.71 weeks old). Fig. 37 shows the maximum positional error factor of such an ellipse versus number of segments. Fig. 38 is a table of the values of maximum positional error factor and percentage error.

As can be seen, there is a dramatic improvement (lowering) in maximum positional error between 4 segments and 8 segments (down from about 18mm to 1.6mm). Further reduction of the maximum positional error factor is obtained when increasing beyond 8 segments, but the improvement is very much smaller. It is believed that the increased wiring and data processing complexity beyond about 8 segments means that it would not be desirable to design an apparatus with more than about 8 bend sensors for a new-born infant (although it would be possible to make an apparatus with more bend sensors of course).

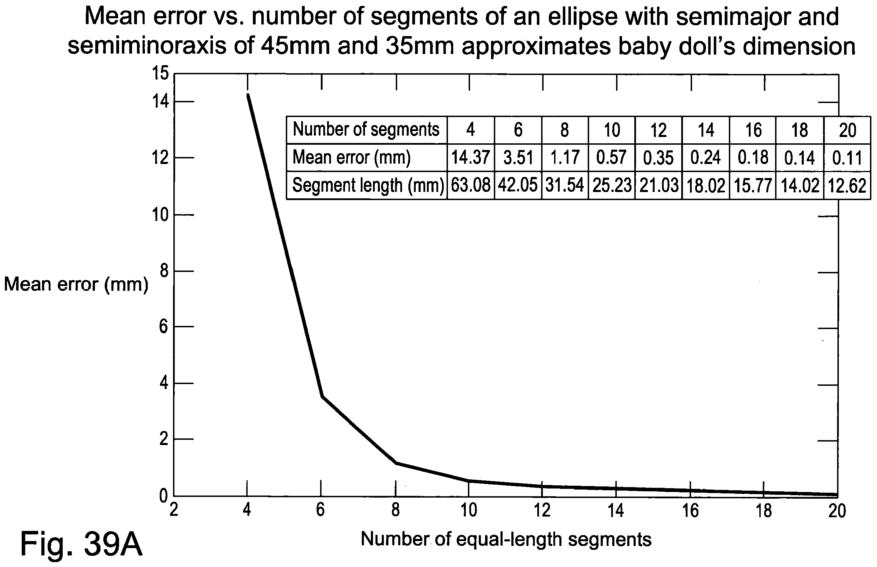

The GOSH data above was obtained for new-born infants up to about 11 weeks old. Accordingly the average figures given by the study are likely to be somewhat higher than the chest dimensions of a pre-term neonate. Accordingly, we re-visited the 'gold standard' reference model obtained from the anatomically correct baby doll described above. The chest circumference of the gold standard was approximately 260mm. We found that an ellipse with semi-major and -minor axes of 45 mm and 35 mm best approximated the shape of the reference model. We then repeated the process of fitting arcs to the ellipse to examine how the mean positional error factor changed with different numbers of segments. Fig. 39A is a graph 390 of mean positional error factor versus number of segments, or bend sensors. The table 391 inset on the graph shows the specific data, including the bend sensor length ('segment length') that would be required for a given number of segments around a premature infant. The graph 390 shows that a mean positional error factor of less than 1.17mm with respect to the semi-major axis can be achieved by using 8 segments, with each segment approximately 31.54mm in length.

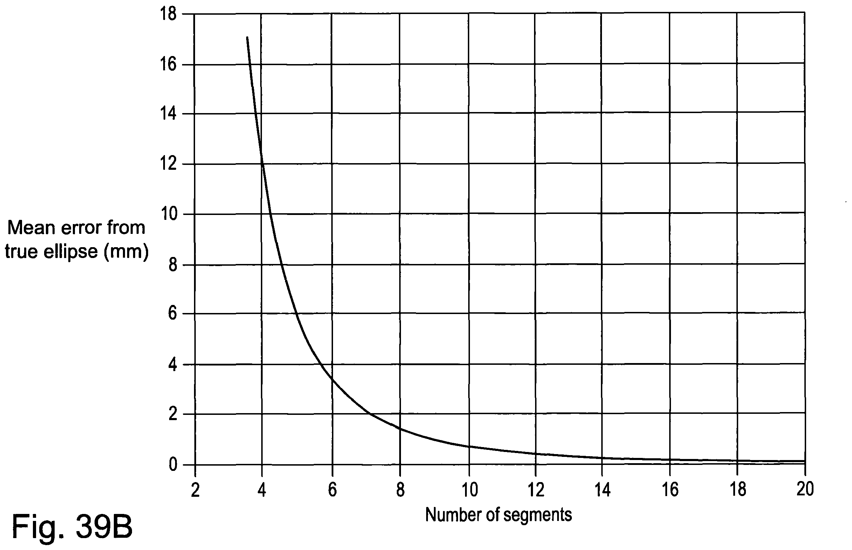

Fig. 39B shows that the data in Fig. 39A approximates an inverse cube relationship of the form $d_{mean} \approx \frac{750}{n^3}$, where $n$ is the number of segments or bend sensors. According to this approximation, $d_{mean}$ for eight bend sensors is 1.46mm. For the purposes of EIT image reconstruction, this is of an acceptable order, whereas reduction to 6 sensors generates more than twice this error (3.47mm).

The average radius $r_{average}$ of an ellipse can be approximated as $r_{average} \approx \sqrt{\frac{a^2+b^2}{2}}$. Accordingly, the mean error $d_{mean}$ can be expressed as a percentage of the average radius of the true ellipse as follows:

$$\frac{100 \times d_{mean}}{r_{average}}$$

Referring to the table inset on the graph in Fig. 39A, the percentage error in $d_{mean}$ changes according to number of segments is as follows:

| No. of segments | 4 | 6 | 8 | 10 | 12 | 14 | 16 | 18 | 20 |
|---|---|---|---|---|---|---|---|---|---|
| % error | 35.6 | 8.70 | 2.90 | 1.41 | 0.87 | 0.60 | 0.45 | 0.35 | 0.27 |

Using the relationship $d_{mean} \approx \frac{750}{n^3}$ above, 7 segments is expected to generate a $d_{mean}$ for this ellipse of about 2.19mm, which we believe to be a tolerable error for EIT image reconstruction. As a percentage of the average radius of the ellipse this is 5.42%, or less than about 6%.

Based on these results, we can set a predetermined threshold for the difference between the true ellipse and the generated ellipse of less than about 6% between $d_{mean}$ and the average radius of the true ellipse. However, it is preferred if the predetermined threshold is less than about 3%, and more preferably less than about 2%.

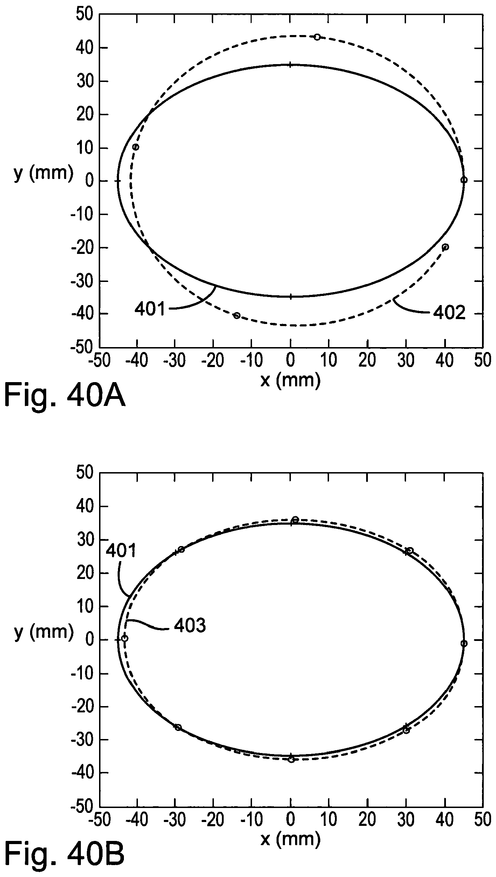

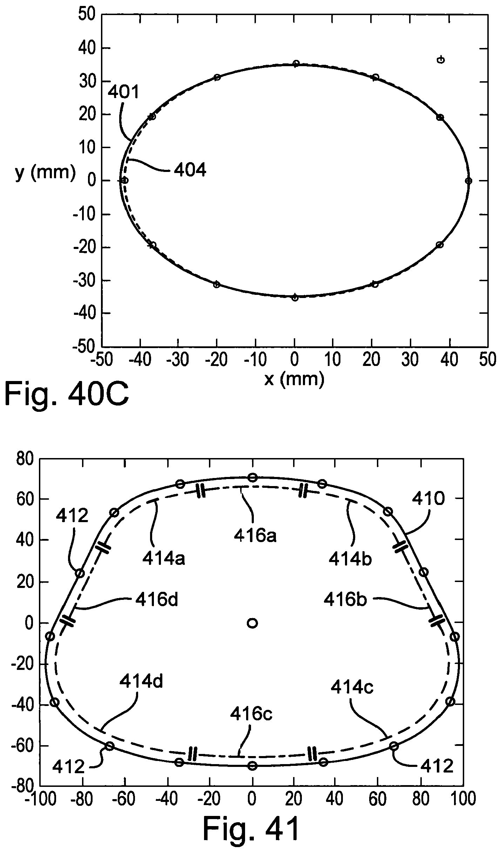

Figs. 40A, 40B and 40C show a true ellipse 401 (a= 45 and b=35 mm, b/a approximately 0.8) and shapes generated by fitting arcs with the unidirectional coordinate generation algorithm. The shape 402 was generated using 4 segments; the shape 403 was generated using 8 segments; and the shape 404 was generated using 12 segments. These Figures confirm that the greater the number of sensors used, the more accurate the estimated shape and coordinates are. Use of 12 segments 404 results in segment length of approximately 21 mm. However, we believe that this would be impractical for incorporation into a belt owing to: the increased physical stiffness of a bend sensor of this length, and other related application issues such as complexity of electronics, increased cabling and high probability of error sources. It is noted that the ellipse reconstructed using 8 equal-length segments has positional error relatively similar to 12 segments.

Results from this modelling therefore support the conclusion that somewhere in the range 7 to 12 bend sensors, each of 30mm length, would be suitable for incorporation into an apparatus for estimation of the thorax shape of the average new-born infant. In a preferred embodiment, the number of bend sensors is 8 for measuring thorax shape of the average new-born infant.

*-Neonatal form*

The modelling of an ideal bend sensor scenario based on ellipses is workable, but doesn't completely accurately reflect a complex shape such as the human thorax. Therefore testing of both the unidirectional and bidirectional sensor coordinate generation algorithms was performed in a realistic scenario to acquire a more accurate error analysis of the boundary measurement methodology. This test was carried out as follows:

1) A true 2D thorax boundary shape was generated as a reference for comparison, based on 3D data from a CT scan obtained from a neonate. This data was adopted from Dr Andrew Tizzard's (Middlesex University) work in development of neonatal FEM thorax model using NURB spline fitting which was published in the paper byBayford *et al.* (2008).
2) A series of curvatures for the NURB spline that represents the true thorax boundary shape were calculated and averaged across each segment for each sensor length, based on number of sensors. The mean curvature of each defined segment was assumed as the curvature of each sensor.
3) Once all mean curvatures were established for all bend sensors, these curvatures then were modelled, as they were the true curvature of each bend sensor.
4) These modelled sensor curvatures were used in the sensor coordinates generation algorithm where the first sensor initial points were known, in order to generate a series of sensor coordinate points.
5) Circular arcs and the proposed Matlab cubic smooth interpolation spline were fitted through the obtained coordinate points.
6) The positional error of the end-coordinates was calculated.

Fig. 41 shows the true thorax boundary shape 410 of the thorax boundary (at nipple level) of an infant of a few months age using a NURB spline. This was plotted based on a CT scan performed on the infant. The boundary shape comprises first regions 414a, 414b, 414c, 414d (shown by dashed lines) and second regions 416a, 416b, 416c, 416d (shown by dot-dash lines). Each of the first regions 414a, 414b, 414c, 414d has a curvature greater than any of the second regions 416a, 416b, 416c, 416d. An apparatus for estimating shape may comprise a string of sensors for estimating the boundary shape 410. By assessing the shape to be estimated it is possible to determine the location of bend and stretch sensors. It is preferred if bend sensors are used wherever possible since these give better information about curvature than stretch sensors. Accordingly, a useful arrangement for the string of sensors would be a string of bend sensors arranged end-to-end to cover the area 414c, 416d, 414a, 416a, 414b, 416b and 414d. A single stretch sensor would cover the region 416; the stretch sensor would provide some degree of flexibility for variation in the length of the boundary shape 410, for example caused by breathing, and/or patients of different size. Another similar possibility would be for the single stretch sensor to be arranged in region 416a of the string of sensors and for the remaining sensors in the string to be bend sensors.

In other embodiments, it may be desirable to provide a greater degree of flexibility in the apparatus (for example so as not to restrict breathing). If one or more additional stretch sensor is to be used in the string of sensors, it is preferred that it is located in one or more of the regions of relatively lower curvature, such as any of regions 416a, 416b, 416c and 416d. It is not desirable to place a stretch sensor in any of the regions of relatively higher curvature such as 414a, 414b, 414c and 414d.

It will be understood that the boundary shape 410 was generated from an infant of a few months in age and that older and younger patients will have different boundary shapes. It is also envisaged that the stretch and bend sensors may be arranged so as to accommodate any abnormalities in patient shape. Accordingly it will be possible to use the guiding principles described above to arrange bend and stretch sensors in a string for the purposed of estimating boundary shape.

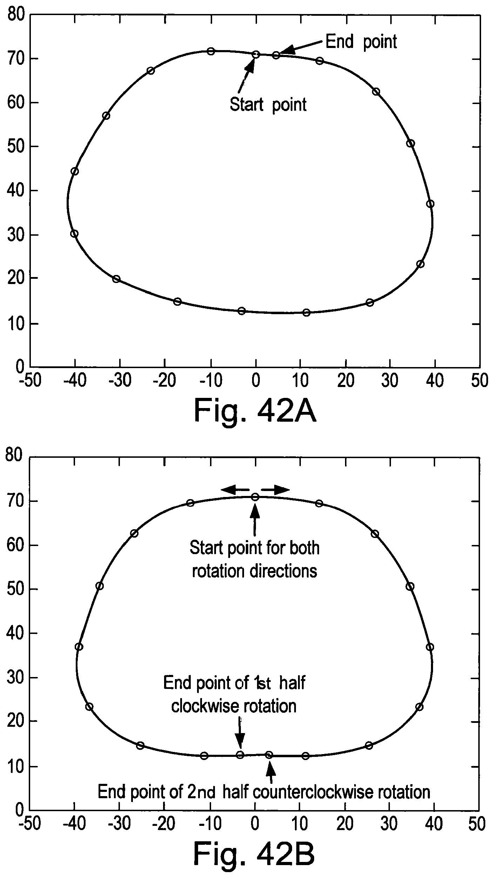

Fig. 42A shows the thorax boundary shape 420 reconstructed based on the average curvature information derived from the NURB spline of Fig. 41 (see step (2) above). The mean curvature was assumed to be the curvature for the bend sensor at each segment (between electrode points 412) on the NURB spline, so as to simulate the ideal sensor scenario. Hence, this enables circular arcs of each segment to be generated from these curvatures, using the unidirectional sensor coordinate generation algorithm, and using the bi-directional sensor coordinate generation algorithm, both described above. Fig. 42A shows the result of applying the unidirectional algorithm and Fig. 42B shows the result of applying the bi-directional algorithm.

The distant position error of the end points for both methods is -4.5mm in Fig. 42A and -6.2mm in Fig. 42B, which approximates to 0.8% and 1.1% error with respect to the total circumference (544.5mm) of the boundary shape 420 in Fig. 41. The negative sign (-) indicates that the points are overlapping in opposite directions.

The outcome of this testing validated the sensor coordinates generation algorithm on a realistic neonate, assuming bend sensors are perfect circular arcs. The algorithm is also shown to work for real world bend sensors, provided that error compensation techniques described above are employed (thereby making each bend sensor closer to the ideal case), and that the starting points for the algorithm are known or set prior to use (as described above, this can be achieved using a stretch sensor positioned on a flatter part of the thorax). This also suggests that the electrode positions incorporated with a bend sensor network may increase boundary measurement points.

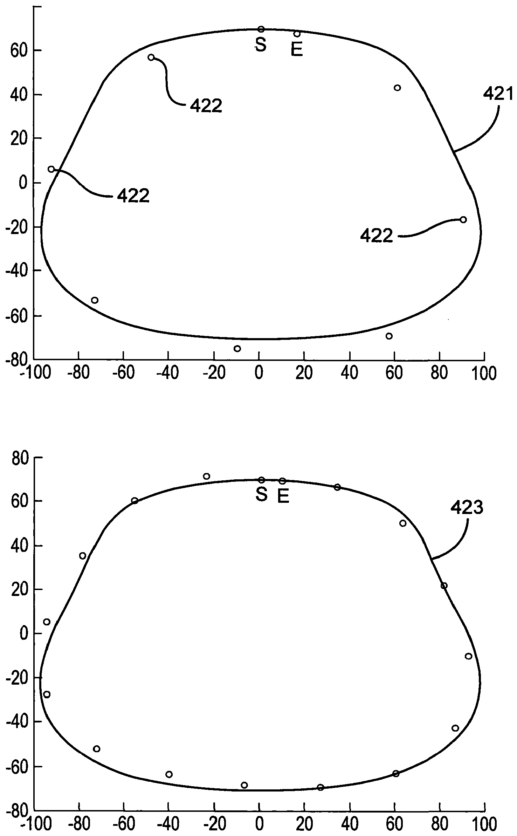

Fig. 42C shows a cross sectional boundary shape 421 through the neonate generated using the unidirectional sensor co-ordinate generation algorithm beginning at point 'S' and ending at point 'E'. The step of correcting radius values based on included angle was performed prior to performance of co-ordinate generation. Endpoints 422 of the 8 bend sensors are superimposed on the Figure. Fig. 42D shows a boundary shape 423 generated in a similar way, but with 16 bend sensors. Performance is measured in terms of distance from the parametric point on the curve relating to the sensor length divided by the parametric distance from the start of the curve and expressed as a percentage.

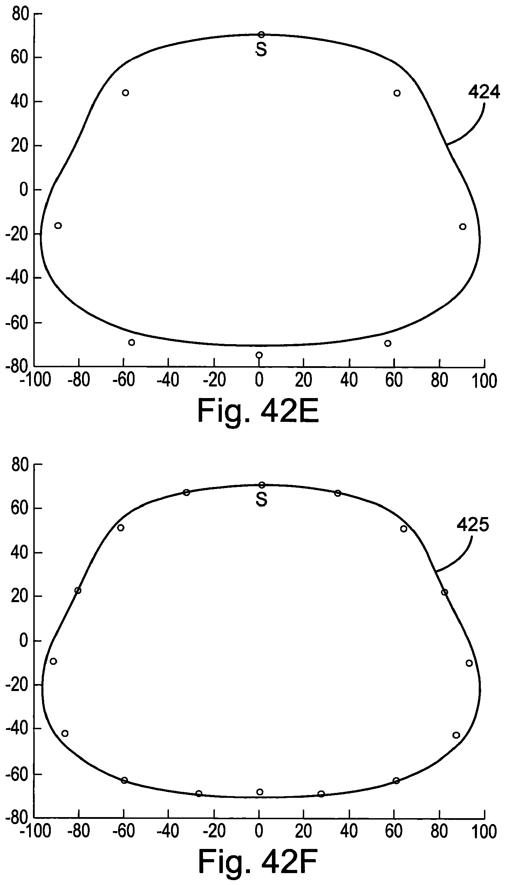

Fig. 42E shows a cross-section boundary shape 424 similar to the boundary shape 421 in Fig. 42C, but generated using the bi-directional sensor co-ordinate generation algorithm beginning at point 'S', and estimating each half of the shape in turn. The final point from each side is then averaged to define the mid-point (u = 0.5) of the boundary curve. Fig. 42F shows a boundary shape 425 generated in a similar way, but with 16 bend sensors.

The table below summarises positional results for both the undirectional reconstruction and the bidirectional reconstruction. The mean and standard deviation of the distances of the reconstructed position from the actual parametric curve position is given along with the significance of a normality test.

| Reconstruction | Full boundary | | Half boundary with mid-point average | |
|---|---|---|---|---|
| Number of Sensors | 8 | 16 | 8 | 16 |
| Mean distance (mm) | 11.34 | 7.16 | 8.08 | 3.45 |
| Standard deviation | 4.79 | 4.26 | 5.24 | 2.73 |
| Maximum (mm) | 15.99 | 12.92 | 13.16 | 6.88 |
| A-D test ($p$) | 0.033 | 0.304 | 0.033 | 0.017 |
| Median (mm) | 11.21 | 6.98 | 10.49 | 3.03 |
| Average Rank | 40.1 | 27.9 | 29.9 | 16.1 |

The distributions of the distances were all non-Gaussian except for the 16-sensor unidirectrional reconstruction. The 16-sensor bidirectional reconstruction generated the smallest average distance error of around 3 mm. A Kruskall-Wallis analysis confirmed that there was a significant difference across the distance medians ($p$ = 0.001). On first sight, it would appear that there is little difference in the median distances between the two eight-sensor reconstructions, though a Mann-Whitney analysis is significant to 0.09, which does not support this. However, a similar test on the 16-sensor reconstruction is significant to 0.008, rejecting the null hypothesis of equality.

The next table summarises similar results expressed as errors for both the full boundary reconstruction and that constructed from two halves. The mean and standard deviation of the errors expressed as distance divided by curve length from the start point to the actual curve parametric point is given along with the significance of a normality test; in all cases, except the eight-sensor full boundary, the distribution of the errors is Gaussian.

| Reconstruction | Full boundary | | Half boundary with mid-point average | |
|---|---|---|---|---|
| Number of Sensors | 8 | 16 | 8 | 16 |
| Mean error % | 5.16 | 2.49 | 4.41 | 1.62 |
| Standard deviation | 1.46 | 4.26 | 1.62 | 3.43 |
| A-D test ($p$) | 0.034 | 0.099 | 0.248 | 0.191 |

For the eight-sensor reconstruction, there was no significant difference between the mean errors when subjected to a paired t-test with $p$ = 0.109 and a 95% confidence interval of -0.2% to 1.7%, though this cannot be firmly asserted as the full boundary error distribution was not Gaussian. However, a Mann-Whitney analysis yielded medians of 3.5% and 2.6% for the unidirectional and bidirectional boundary errors respectively with insignificant difference ($p$ = 0.45). For the 16-sensor test, the bidirectional boundary reconstruction produced significantly reduced mean error of 0.87% with a 95% confidence interval of 0.38% to 1.36% ($p$ = 0.002).

*-Section summary*

Sections 4 and 5 explored two issues: experimental data, and the modelling of ideal bend sensors, where sensors provide accurate curvature information with minimal error. The two objectives of this chapter were:

1) Validating the feasibility of using a series of bend and stretch sensors in providing boundary shape measurement via experimental method, and
2) Test the robustness of the boundary shape algorithm via modelling. The robustness in this sense is defined by the errors of circles and ellipses generated using the algorithm, as compared to true circles and ellipses.

Results from the experimental data demonstrate the potential of bend and stretch sensors in providing boundary shape information of different object shapes, including a thorax shape of an anatomically correct baby doll. Error analysis was performed, and an improved method of constructing coordinate points is described above to reduce error. Improvements were made to the generated shape using modified length, modified algorithm and optimal error correction factor.

A useful working range of bend sensors for a neonatal-size shape measurement was identified through modelling an ideal sensor scenario with different depth/width shape ratio. The useful working range is 7 to 12 bend sensors, each of about 30mm length. A preferred number of bend sensors is 8. The number of bend sensors for patients with thoraxes of different size can be determined by generating a 2D shape which approximates the shape of the thorax to be measured, fitting circular arcs to the 2D shape and determining an error between the shape formed by the circular arcs and the 2D shape. This process is repeated with circular arcs of varying length (equating to bend sensors of different length). The errors are compared and the fewest number of bend sensors can be identified as that number of segments which reduces the error below a predetermined threshold, which can be expressed as a percentage difference between mean positional error $d_{mean}$ and the average radius of the generated 2D shape.

The coordinate generation method was validated via modelling based on the averaged curvatures at a series of points along each segment resembling a bend senor curvature using a realistic model through data obtained from a CT scan. This was to investigate the usefulness and robustness of the proposed sensor coordinate generation algorithm based on ideal sensor curvature and length information.

## 6. 3D Boundary Shape Estimation

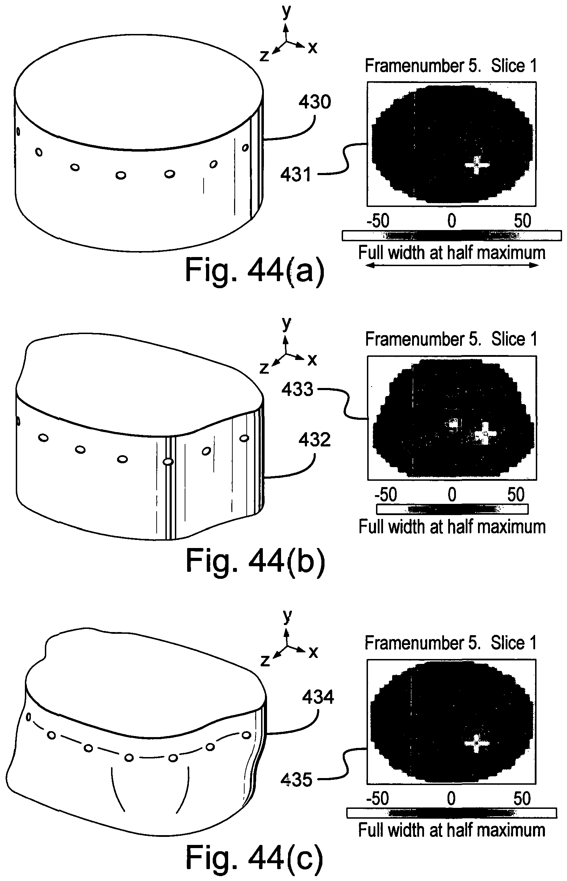

Studies undertaken by Bayford *et al* (2007; 2008) and other research groups have established that a 2D boundary shape model is insufficient for the 3D reconstruction problem of the EIT. A full three-dimensional FEM model based on boundary shape of the subject is required for accurate image reconstruction. Fig. 43A - C shows the evidence of the importance of 3D realistic model on reconstructed images. Fig. 43 shows reconstructed images (on the right) 431, 433, 435 based on 3D forward models (on the left) shaped a) a cylinder 430, b) an 2D thorax shape 432 extended in the z-direction, c) a realistic neonatal model 434. Reference is made particularly to Bayford 2008 to see a colour version of the reconstructed images 431, 433, 435.

A subject-specific 3D FEM model is a true representation of the real subject in terms of its geometric profile. In order to generate a patient-specific 3D FEM model, 3D data of the subject's boundary shape is essential.

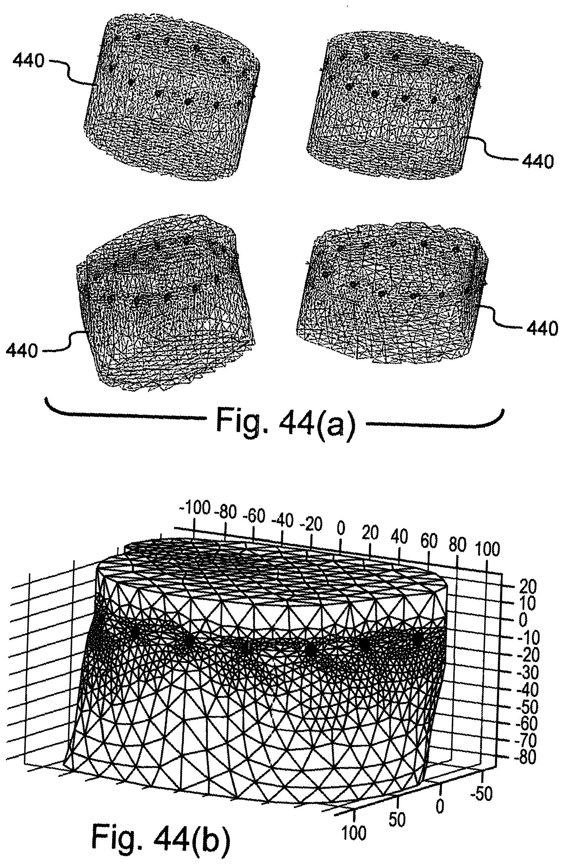

One aspect of this invention is the methodology by which the 3D data of the patient can be generated. Once that is generated, known methods for producing 3D FEM meshes and solid models can be used (see for example Bayford et al. 2007; Bayford et al. 2008; Adler et al. 2009). For example, the measured 3D boundary shape data can subsequently be used to generate a 3D surface model (either in the forms of graphical or mathematical expression) of the real subject, where an existing FEM mesh can be warped to fit the desired boundary form. The warping method (Tizzard & Bayford 2007) is achieved through warping of a pre-developed FEM mesh 440 to fit the 3D surface model of the subject's thorax boundary. Fig. 44A shows FEM meshes 440 developed in Bayford *et al.* 2007 and Fig. 44B shows a neonatal FEM mesh 445 developed by Bayford *et al.* and described in Adler 2009. The benefit of using the combination of the measured boundary surface model in conjunction with the warping method is that it can reduce the time required to regenerate a subject-specific 3D FEM model for different subjects.

*-Premature infant torso size constraints*

We expect that 3D boundary data measurement performed on small premature infants direct using mechanical sensors (i.e. the various belts comprising bend and stretch sensors), will present a number of challenges. In particular, use of the belt there are a number of constraints such as small chest circumference, and thoracic cavity boundary area, sensitive skin etc.

The anthropometric data of newborn (full-term or premature) infants varies widely between individual subjects, thus it is necessary to consider what range of sizes an apparatus, such as a belt, must be able to accommodate. Some anthropometric data for small infants has been reported in the literature:-

- The average chest circumference measured from 10 newborn infants in a EIT research study carried out by Taktak *et al.* (1995) was 258 $\pm$ 43 mm.
- Crelin (1973) reported an average length from crown-to-heel of newborn preterm infants as about 380 mm.
- Hartwig *et al.* (2009) produced a 3D thorax model of a newborn weighted 2.10 kg and 102.4 x180.2x181.7 mm in dimensions, showing that the length of z-axis approximated the width of the chest of the infant.

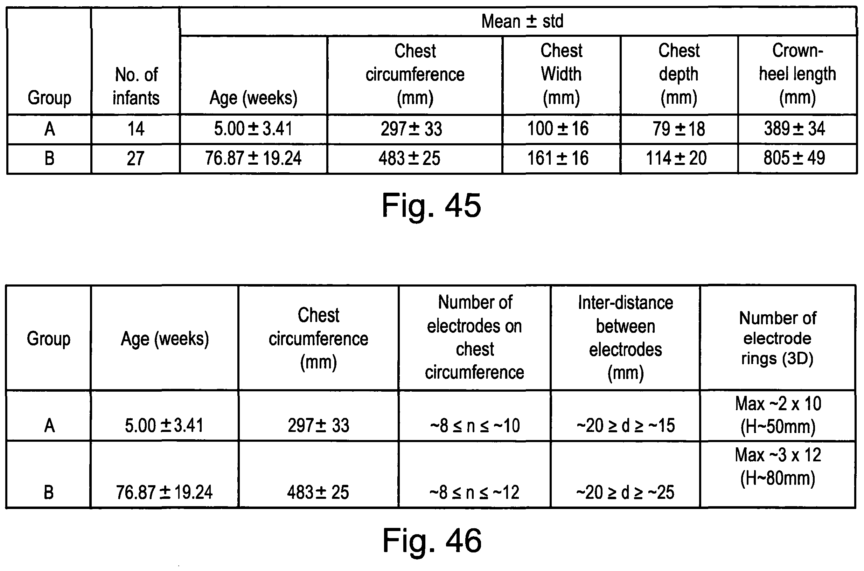

Since one aspect of this invention is to estimate the thorax shape of premature infants based on a network of bend and stretch sensors, the average chest size of premature infants for a defined age group must be considered so that the apparatus is suitable for a range of premature infants. The reference average thorax size range for a group of premature infants is based on the anthropometric data of 41 premature infants (less than 37 gestation weeks), collected in 2008 from the GOSH in United Kingdom. The preterm infant ages ranged from 1.71 to 100.43 weeks and were divided into two age groups: of 1.71 to 11.71 weeks and 36.86 to 100.43 weeks. Fig. 45 shows that the average chest circumferences, width, depth and crown-heel length of these two groups.

In Group A, the average chest circumference of the premature infants with age of 5 $\pm$ 3.41 weeks is 297 $\pm$ 33 mm, which provides a closer reflection of the shape of a newborn premature infant in terms of chest size and dimension. It is consistent with the data reported in the literature. This is a useful guideline number to use in designing an apparatus, such as a belt, for premature infants.

However, in terms of three-dimensional boundary shape modelling, both horizontal and vertical thorax sizes need to be known. We carried out investigation on this point for both physical placement of electrode arrays (for EIT measurements) and bend-stretch sensors network (for boundary shape measurements).

*-Anatomical landmark for electrode serving as registration points*

In an attempt to solve the forward problem, the complete electrode model must be employed for accurate prediction of the electrode voltages (Somersalo *et al.* 1992). Knowledge of electrode locations and size are therefore required (see introduction for more details).

From the literature (Crelin 1973; Seidel *et al.* 2009), the geometry length (vertical) of newborn's lungs at rest is about 25 mm. The lung apices can expand to up to 8 mm above the level of the sternum (superiorly), and their bases lie at the eleventh rib, T11 (posteriorly) during inspiration (Crelin 1973). According to Seidel *et al.* (2009), the chest of the newborn is often considered round with its anterior-posterior diameter approximately equal to the lateral diameter. The locations of the lungs, from the lateral-view, underlie from the peak of the axial to the 7th or 8th rib. During deep inspiration, the lungs rise to about the level T1 and their bases descend to about T12 (posteriorly).

In the EIT, when electrodes are placed individually in sequence, the four basic anatomical points such as the anterior imaginary line followed the sternum; the posterior line of the spinal column, and the two side points located at left and right midaxillary lines are commonly used as registration points for placement of electrodes. These four reference points enable the simplest and most practical method of positioning electrodes. Once these reference points are fixed, the number of electrodes and spacing can then be determined according to the circumference and height of thorax regions of the newborn infant age range. Another common practice of EIT electrode placement is placing the first ring of electrodes according to a defined level (a few millimetres) below the nipple line, which is approximately at the level of 6th-7th intercostal space at the parasternal line (Adler *et al.* 2009); the subsequence electrode rings are placed vertically down to the 10th intercostals space (above navel). Thus, anatomical landmark information is used to approximate the locations, vertical length and, thorax dimension for EIT electrode measurements and sensor-network placement.

According to the anthropometric study of the proportion of human body length, the torso length between nipple line and the navel (bellybutton) of a newborn is approximately one-eighths of the total body length. This means that the torso length (between nipple line and the navel) of Group A in Fig. 45 is approximately 50 mm. However, electrodes placement close to the navel line may lead to error in readings as a result of the mediastinum or abdominal impedance changes which should be avoided (Adler *et al.* 2009).

According to certain aspects, the vertical body length for electrode placement is less than about 50mm below the nipple line (since this is within the expansion region of the lungs, but avoids imaging of regions such as the stomach region). For boundary shape modelling, the vertical area of the thorax region can be extended to 70 mm below the nipple line to allow placement for at least three arrays of bend sensors around the body, so that sufficient number of vertical geometric data can be obtained for fitting of spline or curve. The at least three arrays may be part of one apparatus, such as a belt.

*-Electrode size and number*

When designing the wearable bend-stretch sensor network that incorporate the EIT electrodes, a minimum distance of 10 mm between electrodes is necessary to prevent electrolytic bridging (Taktak *et al.* 1995). If the Ambu BRS electrodes (discussed in Rahal 2009), with the electrode dimension (Width x Height) of approximately 16 mm x 19 mm are used, the number of electrodes required for a belt can be calculated based on electrode dimensions, minimum separation distance, chest circumference and thorax height region according to the group's average age (seethe section above on anatomical data). The minimum and maximum number of Ambu BRS electrodes (D16 x H19 mm) needed on the specified thorax boundary range for newborn premature infant age in groups A and B is summarised in Fig. 46. The electrode geometry arrangement for both 2D and 3D settings meet the criteria of at least 10mm gap distance between electrodes. Total number of electrodes allowed for 3-D thorax settings is calculated based upon the number of rings of electrodes according to maximum thorax region defined previously (height starts from the nipple level inferiorly toward but above the navel) that can provide sufficient coverage of lung regions.

The small thorax volume (for the height of the torso segment) limits the total of number of electrodes (electrode arrays in 3D) used, presenting another constraint to the signal-to-noise ratio.

*-Sensor physical properties constraints*

As described above, commercially available off-the-shelf bend and stretch sensors are not specifically made for high accuracy measurement. The limitations and challenges of using bend and stretch sensors for 3D boundary data measurement includes:

1. One-directional/axial bending with output resistance responses linearly to its curvature of bending within a pre-calibrated curvature range.

2. The dimensions of bend sensor (L114 x W6 x D0.5mm) can present a challenge in terms of size of sensor versus area of subject, particularly when the subject is small (e.g. a neonate). As the original sensor provides only one output per bend, a sensor length 114mm could not correctly represent the geometric profile the sensor if single curvature information is available from the sensor output. Hence, the bend sensor can be modified as described herein to an appropriate length, allowing smooth bending with its resistance output reflecting its bent profile.

3. Bend sensor is short enough that the radii of all the points on the plane are constant along its length when bent. Bending profile of a modified bend sensor is assumed to be of circular arc, which possesses a constant radius as to of an osculating circle.

4. The length of the Abrams Gentile bend sensors should not be shorter than approximately 30 mm. It has been observed that if it is shorter than this, it will experience stiffness and will be difficult to bend. It will be appreciated that other bend sensors may be modifiable to shorter or longer lengths depending on their stiffness.

5. When the sensor length is longer than 40 mm, multi-tapping (via connecting soft carbon wires to the conductive contact area on the bend sensor using conductive glue) on the sensor can be performed in order to obtain more outputs from one bend sensor. In this way it is possible to divide the bend sensor into shorter sections, without having to physically cut it. Measuring the voltage or resistance of each shorter section provides local curvature between two tapping locations. However, this also increases the complexity of wire cabling and its practicality.

6. Calibration is required for each sensor due to the fact that each sensor has a variable linear regression relationship. The need to calibrate each sensor increases the complexity and practicality of the measurement method.

7. As discussed above the coordinate point generation algorithm is based on the geometric rotational transformation of a starting coordinate, the rotation being determined according to the radius calculated from the output of the respective bend sensor. A circular arc is fitted between the starting and finishing coordinates. This is repeated sequentially until a shape has been generated; a spline can then be fitted to the shape to provide a smooth surface for use in the forward model. Hence, for 2D boundary shape estimation, sensors are arranged in a way that the starting location of the first bend sensor is known and fixed, followed by another sensor successively in a closed loop where the subsequent coordinate points are determined based on each sensor's curvature information. The challenge for 3D boundary data measurement, is that required that bend sensor network should be arranged in a way that enables the acquisition of 3D coordinate (x,y,z) points, from which a surface model can be generated.

8. Modelling is performed in an ideal bend sensor scenario, assuming that all bend sensors produce accurate curvature /radii information correspond to its physical profile.

The physical constraints of the stretch sensor are that it does not provide useful curvature information and only provides displacement information or its stretched length. However, a stretch sensor is useful as it allows a belt to accommodate a range of size subjects. To address this, we found that the stretch sensor should be placed on the front or back of the thorax where the topology of the surface is almost flat. Stretch sensors also present a challenge in terms of electrical output measurement, which requires the introduction of an electric wire connection into the belt. It also requires calibration prior to use. Nevertheless, we found that good results were achievable using a stretch sensor.

*-3D modelling*

3D modelling is a process of developing a computer model representation of a 3D scene or object with the aid of specialized 3D modelling and computer graphics software (Badler & Glassner 1997). A realistic 3D model can be in the form of mathematical representation of a subject's shape (for modelling, simulation or manipulation), or displayed as graphical 3D model for visualisation.

3D models are broadly categorized into two main groups: boundary/surface and volumetric. The volumetric model is represented by sets of volume primitives-voxels, whereas the boundary (surface) model representation is either in the forms of mesh (set of polygons), parametric curves (Bezier or B-splines) or implicit algebraic surface.

The simplest type of a surface model is a 3D coordinate point cloud (scattered or organized), where different methods of surface reconstruction can be employed to recover the shape of the real subject in a 3D domain. Typically, a highly dense point cloud is acquired often with 'noisy' data, which may therefore require pre-processing of data. After the data processing, a simple surface model can be produced through connecting the 3D point cloud with line segments forming polygonal mesh or approximated by curved spline or surface patches to form a smoother and realistic model. There are numerous 3D coordinate input methods used for developing a realistic model:

- 3D coordinate data measured directly from a real object using either mechanical or optical bend sensors and mechanical coordinate measuring machines (CMMs)
- Image photogrammetric methods
- 3D digitizer laser scanning

However, as described above any measurement method that requires line-of-sight scanning is not practical.

## 7. 3D Data Measurement

*-Error tolerance of the boundary shape model*

Preferably, the accuracy and error of the proposed boundary shape modelling should meet the required tolerance suggested in the literature. Barber & Brown (1988) showed that for a body circumference of 100 mm, an electrode positioning error of less than 3.3 mm was required for a useful image. This suggests that the desired/required accuracy for the boundary model by the neonatal EIT image reconstruction for a neonate (with a chest circumference of approximately 300 mm) in 2D should be no more than approximately 9.9mm, or 3% of circumference.

Another study (Gersing et al. 1996) has also shown that the error caused by boundary geometry varying and deformation on the resistivity changes was of 1% in the axis length of cross-sectional of the shape.

In a recent study, Grychtol *et al.* (2012) quantified the errors resulting from boundary shape mismatch between the actual body shapes (human and healthy pig) in 3D. They found that shape mismatch (area of symmetric difference or nonoverlapping area between actual shape and shape model) of greater than 4% show significant errors on images generated from all tested reconstruction algorithms (GREIT, NOSER, TSVD and Laplace). They claimed that no conclusions on which particular features of a subject's shape are the most important to preserve, and that this 4% shape mismatch, or 4% threshold (found from their study), should not be interpreted as necessary condition for obtaining quality reconstruction shape. However, they recommend that: "The EIT data be reconstructed on models closely resembling the actual shape of the body and reflecting the true positions of the electrodes."

This information is used as a guideline for the estimation of the tolerance of 3D boundary data measurement methodology as complexity increased with number of sensors and measurement data.

*-multiple 2D shape sensors*

In the preceding sections we have demonstrated how it is possible to use a belt comprising a number of bend sensors and at least one stretch sensor to measure a 2D shape (for example in the x-y plane). Consideration is now given to how the measurement technique can be expanded in the z-direction, in order to produce 3D boundary data for surface model generation.

Various algorithms for transforming sets of transverse 2D planes into a smooth 3D model have been reported (Braude et al. 2007; Marker et al. 2006). The two simplified approaches commonly used are contour stitching and volumetric methods. Contour stitching is a method of creating a continuous mesh between contours by connecting the vertices of the adjacent contours. Surface reconstruction based on contour-stitching involve solving of the problems related to methods: for connecting vertices between contour, for creating mesh, and for handling multiple number of contours on different contour planes. Volumetric approaches for the contour-based surface reconstruction introduce algorithms for the spline interpolation technique to the coordinates on the contours in z-direction. For example, Braude *et al.* (2007) demonstrated a technique of how 3D model can be achieved through fitting of closed surface to a set of 2D contours extracted from a 3D scan.

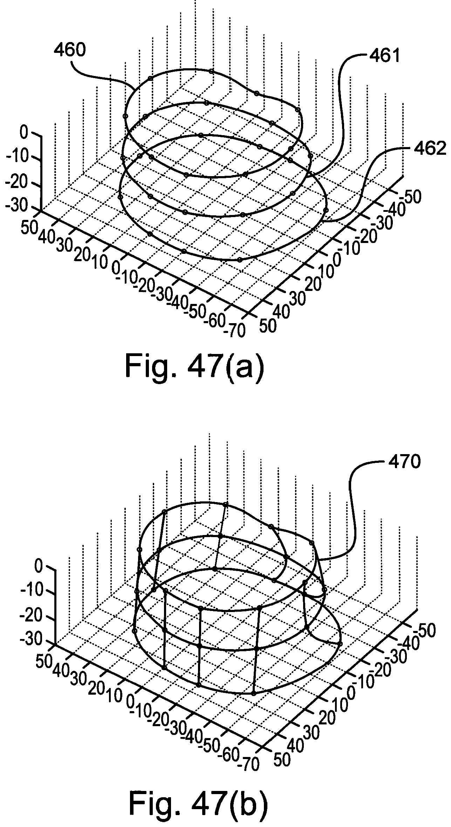

For simplicity, it is proposed that multiple sensor belts can be wrapped around a subject's thorax with known inter-vertical distance (in z-axis direction) between sensor arrays. Each sensor belt is similar in construction to the third belt described above, i.e. where a plurality of bend sensors are arranged end to end in sequence joined by one stretch sensor. The bi-directional sensor coordinate generation algorithm is used. By setting the medial centre reference (x,y) for all 2D boundary data via geometry transformation, and assigning z-location of the first 2D boundary data 460 or closed curve plane to zero, successive boundary data planes 461, 462 can be stacked downward or upward (geometrically) in z-axis direction accordingly to inter-distance between arrays. Once all of the 2D closed curve planes are arranged in the right order as illustrated in Fig. 47A, an appropriate technique for blending the 2D closed curves into a 3D model can be applied to recover the 3D model of the boundary shape. For example, Fig. 47B shows a 3D model 470 where cubic splines were interpolated in z-direction through the boundary coordinates using a spline construction function in Matlab 2010

Our idea of using multiple bend sensor arrays for 3D boundary model generation appears to enable a reasonable 3D model to be produced quickly comparatively simply. However, due to the physical size constraints of an infant, only limited number of horizontal sensor arrays could be used thereby providing limited horizontal boundary shape information for creating the 3D model. Therefore the generated 3D model may not fully reflect the real shape of arbitrary boundary shape, e.g. infant thorax shape, due to lack of sufficient vertical data.

*-Axial bend and/or stretch sensors*

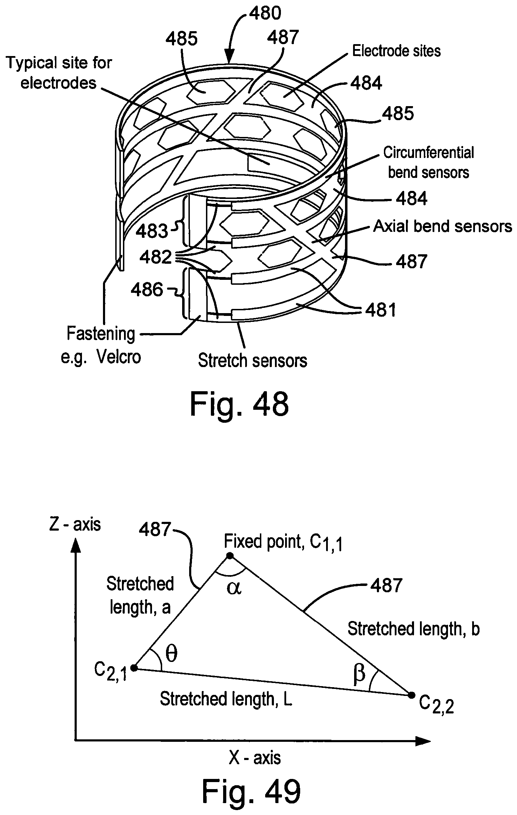

In order to enhance the method for 3D boundary data acquisition and its resulting 3D model, we have designed a fourth belt. Referring to Fig. 48, the fourth belt 480 comprises a plurality of sensor strings 481. Each sensor string 481 comprises a plurality of bend sensors and one stretch sensor 482, like the third belt described above. A releasable attachment such as Velcro (RTM) is provided at either end of the belt so that it may be releasable fastened around a patient. The overall length of the belt 480 is 260mm long and about 60mm in height.

A first pair of bend sensors 483 is held by a material (such as a flexible textile) 484 that comprises a plurality of electrode openings 485. A second pair of bend sensors 486 is fabricated in a similar way. The first and second pairs of bend sensors 483, 486 are joined by axial bend and/or stretch sensors 487. One option (not shown) is for there to be one or more pairs of stretch sensors between each string 481, each member of the pair oriented in opposite senses. Thus, each pair of stretch sensors may form a 'V' shape. The base of the V is arranged to coincide with the centre of an electrode hole 485. The other end of the V is sized so that each stretch sensor points toward the centre of another electrode hole in the second pair of bend sensors 486. The axial bend sensors 487 serve to hold the first and second pairs of bend sensors 483, 486 a fixed distance apart, but allow curvature estimation in the z-direction.

The aforementioned diagonal arrangement of stretch sensors is to enable displacement measurement between coordinates in the z-direction, as well as the diagonal displacement information between two coordinates in 3D space. It is suggested that by fixing the first bend sensor array with first electrode ring to the chest anatomical landmark, a series of coordinates on the thorax where the bend sensor array is placed, can be obtained. Given the 2D coordinates measured from the second bend sensor array and initial parameters of stretch sensor i.e. original length and point of connection for diagonal stretch sensor, a series of coordinates for the second bend sensor array in 3D space can be determined based on stretch sensor displacement information between coordinates of two sensor arrays using the geometric triangulation method. The triangle arrangement of two stretch and a bend sensor is shown in Fig. 49, with the assumptions that it forms a triangular facet where coordinates $C_{1,1}$ represent the first known coordinate on the first bend sensor array. $C_{2,1}$ and $C_{2,2}$ are the two unknown coordinates on the second bend sensor array which can be determined based on stretched length a and b given the length of bend sensor, L. The angle $\alpha$ can be determined using the formula derived by Dunnet (1969) below:

$$cosh(L) = \cosh(a)\cosh(b) - \sinh(a)\sinh(b)\cos(\alpha)$$

Assuming that the triangle is a hyperbolic triangle where the sides of the triangle are geodesics, the coordinates of $C_{2,1}$ and $C_{2,2}$ can be determined.

Since simplicity in clinical settings is crucial, especially the neonatal setting, the diagonal stretch sensor network is arranged in that permits other clinical measurements to take place. The simplicity of sensor-network is defined as with minimal number of sensors and simple arrangement to provide sufficient boundary data for estimation of the 3D boundary form as opposed to irrelevant data.

Global deformation such as twisting and bending of thorax is not much of a concern in the context of neonate boundary measurement, since the twisting is minimal due to the constraint of body size and careful handling of the premature baby. Significant distortion or twisting of the body segment is therefore unlikely. It is suggested that the used of minimal stretch sensor arranged diagonally in this 3D boundary measurement sensor network enables estimation of volumetric deformation of the thorax caused by breathing. It is also believed that this sensor arrangement will allow flexibility of body movement and accessibility for electrodes placement.

**APPENDIX A**

*-Simple bend sensor co-ordinate generation algorithm*

```
function [P, u, theta] = simple_bend(crv, res, ns, etyp, half)
% P are the sensor end points
% crv is the b-spline curve
% res is the length of sensor portion in mm (normally around 4 mm for Abrams
% Gentile
% ns is the number of sensors in total
% etyp the error treatment type:
% 0 none, 1: equally distributed, 2: proportional
% half = 0: full curve, 1: rhs / cw, 2: lhs / ccw
% table contains columns defining [u, dist, crvlen to u, dist/crvlen]
if (nargin < 5)
 half = 0;
 end
 n = ns/2;
 stheta = 1;
 switch half
 case 0
    st = 0;
    en = 1;
    n = ns;
 case 1
    st = 0;
    en = 0.5;
 case 2
    st = 1;
    en = 0.5;
    stheta = -1;
    end
[mK, Lb] = curvaturesfromcrv(crv, res, n, st, en);
theta = stheta*mK.*Lb;
econst = (stheta*n/ns*2*pi + sum(theta))/n;
efac = abs(n/ns*2*pi/sum(theta));
if (etyp == 1)
 theta = theta - repmat(econst, size(theta));
 end
 if (etyp == 2)
 theta = theta*efac;
 end
O = zeros(2,n);
c = cos(theta(1));
s = sin(theta(1));
T = [c -s; s c];
P1i = crv.coefs(1:2,1);
r1 = [0; -1/mK(1)];
O(:,1) = P1i - r1;
Pf = zeros(2,n);
Pf(:,1) = O(:,1) + T*r1;
for i = 1:n-1
```

```
r = Pf(:,i) - O(:,i);
O(:,i+1) = Pf(:,i) - mK(i)/mK(i+1)*r;
c = cos(theta(i+1));
s = sin(theta(i+1));
T = [c -s; s c];
Pf(:,i+1) = O(:,i+1) + T*(Pf(:,i) - O(:,i+1));
end
P = [P1i Pf];
u = st:stheta/ns:en;
```

*-Unidirectional sensor co-ordinate generation algorithm*

```
function [P, table] = simple_bend_uni(crv, res, ns, etyp)
 % P are the sensor end points
 % crv is the b-spline curve
 % res is the length of sensor portion in mm (normally around 4 mm for Abrams
 % Gentile
 % ns is the number of sensors in total
 % etyp the error treatment type:
 % 0 none, 1: equally distributed, 2: proportional
 % table contains columns defining [u, dist, crvlen to u, dist/crvlen]
 [P, u, theta] = simple_bend(crv, res, ns, etyp, 0);
 Pn = nurbpoints(crv, u); dists = sqrt(sum((P - Pn(1:2,:)).∧2))';
 L = [1; crvlen(crv, u(2:length(u)))];
 err = dists ./ L;
 L(1) = 0;
 nurbplot(crv, 100, [0 0]);
 hold on;
 plot(P(1,:), P(2,:), 'ro');
 view(2);
 table = [u' cumsum([0 theta])' dists L err];
```

*-Bidirectional sensor co-ordinate generation algorithm*

```
function [P, table] = simple_bend_bi(crv, res, ns, etyp)
 % P are the sensor end points
 % crv is the b-spline curve
 % res is the length of sensor portion in mm (normally around 4 mm for Abrams
 % Gentile
 % ns is the number of sensors in total
 % etyp the error treatment type:
 % 0 none, 1: equally distributed, 2: proportional
 % table contains columns defining [u, dist, crvlen to u, dist/crvlen]
 [PI, u1, theta1] = simple_bend(crv, res, ns, etyp, 1);
 [P2, u2, theta2] = simple_bend(crv, res, ns, etyp, 2);
 Pm = (P1(:,ns/2+1) + P2(:,ns/2+1))/2;
 P = [P1(:,1:ns/2) Pm P2(:,ns/2:-1:1)];
 u = [u1(1:ns/2+1) u2(ns/2:-1:1)];
 theta = [theta1(1:ns/2) -theta2(ns/2:-1:1)];
 Pn = nurbpoints(crv, u);
 dists = sqrt(sum((P - Pn(1:2,:)).^2))';
 L = [1; crvlen(crv, u(2:length(u)))];
 err = dists ./ L;
 L(1) = 0;
 nurbplot(crv, 100, [0 0]);
 hold on;
 plot(P(1,:), P(2,:), 'ro');
 view(2);
```

```
table = [u' cumsum([0 theta])' dists L err];
```

**APPENDIX B**

**Matlab Programming codes 2D coordinates reconstruction algorithm modelling**

```
%% This function file is to plot circle/ellipse, calculate its
% circumference, % and number of segment defined by user
% RuMi 03.09.2010
function [theta, arcLength,x_t,y_t,final_C] = Ellipse_Sensor_Error(a, b,
num_segments)
    %a is ellipse semi-major axis
    %b is ellipse semi-minor; for plotting a circle a=b
    % num_segments is number of segments of the arc
    %lower_angle and upper angle in radian
 %*******************************************************************
**
%% To plot a complete circle or ellipse
lower_angle=0;
    upper_angle=pi*2;
    theta_vec=linspace(0,upper_angle, 1000);
    for idx=1:length(theta_vec),
            xellipse(idx)=a*cos(theta_vec(idx));
      yellipse(idx)=b*sin(theta_vec(idx));
    end
    plot(xellipse,yellipse,'b-')
    axis square
        hold on
 %*******************************************************************
**
%% To find the circumference (Arc_Length) of ellipse/circle from 0 to pi/2
%using Matlab Quad function and accumulated segment length (ArcL_Specified)
%based on user-defined number of segments (index)
Arc_Length=quad(@(t)sqrt((a.*sin(t)).^2+(b.*cos(t)).^2),lower_angle,upper_angle);
 for index=1:num_segments
 ArcL_Specified(index)=index*(Arc_Length/num_segments);
 %*******************************************************************
**
%% To calculate angle for a given index (number of segment) based on
%% Matlab fminsearch function where
% lowerBound is the total angle for a given index
 if index == 1
    lowerBound = lower_angle;
 else
    lowerBound = theta(index-1); %
 end
 % x is the estimated angle driving the minimisation based on minimal
 % different between the computed arc length for a given angle and the
 % segment length (as defined by num_segments)
 theta(index) = fminsearch(@(x) cf(x, ArcL_Specified(index), lower_angle, a, b),
 lowerBound);
 arcLength(index)
 =quad(@(t)sqrt((a.*sine t)).^2+(b.*cos(t)).^2),lowerBound, theta(index));
 x_t(index)=a.*cos(theta(index));
 y_t(index)=b.*sin(theta(index));
 plot(x_t,y_t,'ko')
 hold on
 end
 function sse=cf(x, ArcL_Specified, lower_angle, a, b)
 Result_Integration = quad(@(t)sqrt((a.*sin(t)).^2+(b.*cos(t)).^2),lower_angle,x);
```

```
sse = (ArcL_Specified-Result_Integration).^2;
return
end
%%%%%%%%%%%%%%%%%%%%%%%%%%%%%%%%%%%%%%%
%%%%%%%%%%%%%%%%%%%%%%%
for index=1:num_segments*2
ArcL_SpecifiedMC(index)=index*(Arc_Length/(num_segments*2));
%% Calculate mid point of segment length, angle for a given length
% lowerBound is used in the calculation of Length (the arclength
% corresponding to the angle for a given index)
if index == 1
    lowerBoundMC = lower_angle;
else
    lowerBoundMC = thetaMC(index-1);
end
% x is the estimated angle driving the minimisation based on minimal
% diff between the computed arc length for a given angle and the
% segment length (as defined by num_segments)
thetaMC(index) = fminsearch(@(x) cfMC(x, ArcL_SpecifiedMC(index),
lower_angle, a, b), lowerBoundMC);
arcLengthMC(index)
=quad(@(t)sqrt((a.*sin(t))^2+(b.*cos(t)).^2),lowerBoundMC,thetaMC(index));
x_tMC(index)=a.*cos(thetaMC(index));
y_tMC(index)=b.*sin(thetaMC(index));
end
for i=1:num_segments;
  %thetaM(i)=thetaMC(2*i-1);
    x_R(i)=x_tMC(2*i-1);
    y_R(i)=y_tMC(2*i-1);
  plot(x_R,y_R,'xr')
    hold on
end
K=a*b./((((b.*x_R)./a).^2+((a.*y_R)./b).^2).^(3/2));
%K(3)=1.2*K(3);% Sensor 2 20% error
%K(4)=0.9*K(4); % sensor 4 -10% error
Mean_radii=1./K;
%%%%%%%%%%%%%%%%%%%%%%%%%%%%%%%%%%%%%%%%
%%%%%%%%%%%
%to include Gaussian Error of +/-20% of curvature, K=1.2*K
%for i=1:length(K);
%Mean_radii(i)=1/K(i);
%end
%%%%%%%%%%%%%%%%%%%%%%%%%%%%%%%%%%%%%%%
%%%%%%%%%%%%%
function sseMC=cfMC(x, ArcL_SpecifiedMC, lower_angle, a, b)
Result_Integration = quad(@(t)sqrt((a.*sin(t)).^2+(b.*cos(t)).^2),lower_angle,x);
sseMC = (ArcL_SpecifiedMC-Result_Integration).^2;
return
end
% finding coordinate of each segment given the segment length and radius
n=1;
Segment_Length=ArcL_Specified(1);
O(:,n)=[a-Mean_radii(1);0];
 C(:,n)= [a;0];
 final_C(:,n)= O(:,n)+[cos(Segment_Length/Mean_radii(1))-
 sin(Segment_Length/Mean_radii(1));...
   sin(Segment_Length/Mean_radii(1))
   cos(Segment_Length/Mean_radii(1))]*(C(:,n)-O(:,n));
for n=1:num_segments-1;
  O(:,n+1)=final_C(:,n)-(Mean_radii(n+1)./Mean_radii(n))*(final_C(:,n)-O(:,n));
   final_C(:,n+1)= O(:,n+1)+[cos(Segment_Length./Mean_radii(n+1)) -
   sin(Segment_Length./Mean_radii(n+1)); sin(Segment_Length./Mean_radii(n+1))
   cos(Segment_Length./Mean_radii(n+1))]*(final_C(:,n)-O(:,n+1));
```

```
    end
Coordinate_C= [C final_C];
for z=1:num_segments+1;
plot(Coordinate_C(1,z),Coordinate_C(2,z),'ko'),xlabel('X
(mm)','fontsize',14,'fontweight','b'),ylabel('y(mm)','fontsize',14,'fontweight','b')
hold on
end
%plot(O(1,:), O(2,:),'xk')
%hold off
% to calculate to area enclosed by all the coordinates
% first find the approximation of the area of the quadrant of ellipse
% to reconstruct the curve of the circular points
m=1;
 for i=1:1:1000;
 P(:,i)=O(:,m)+[cos((i./1000).*Segment_Length/Mean_radii(m)) -
sin((i./1000).*Segment_Length/Mean_radii(m));sin((i./1000).*Segment_Length/Mea
n_radii(m))cos((i./1000).*Segment_Length/Mean_radii(m))]*(C(:,m)-O(:,m));
 plot(P(1,i),P(2,i),'r--');
 hold on
 end
 for m=1:num_segments-1;
    for i=1:1:1000;
 P2(:,i)=O(:,m+1)+[cos((i./1000).*Segment_Length./Mean_radii(m+1)) -
sin((i./1000).*Segment_Length./Mean_radii(m+1));
 sin((i./1000).*Segment_Length./Mean_radii(m+1))
 cos((i./1000).*Segment_Length./Mean_radii(m+1))]*((final_C(:,m)-O(:,m+1)));
  plot(P2(1,i),P2(2,i),'r--');
    end
 end
 hold off
 end
```

## REFERENCES

The following is a detailed list of references mentioned in the description. The entire disclosure of all references is herein incorporated by reference.

Adler, A. et al., 2009. GREIT: a unified approach to 2D linear EIT reconstruction of lung images. Physiological measurement, 30(6), pp.S35-55. Available at: http://www.ncbi.nlm.nih.gov/pubmed/19491438.

Adler, A., Guardo, R. & Berthiaume, Y., 1996. Impedance imaging of lung ventilation: do we need to account for chest expansion? Biomedical Engineering, IEEE Transactions, 43(4), pp.414-420.

Agrons, G.A. et al., 2005. Lung Disease in Premature Neonates: Radiologic-Pathologic Correlation1. Radiographics, 25(4), p.1047. Available at: http://radiographics.rsna.com/content/25/4/1047.abstract [Accessed March 29, 2011].

Babaeizadeh, S., Brooks, D.H. & Isaacson, D., 2004. Electrical impedance tomography using a 3-D boundary element inverse solution. In Signals, Systems and Computers, 2004. Conference Record of the Thirty-Eighth Asilomar Conference on. IEEE, pp. 1595-1599. Available at: http://ieeexplore.ieee.org/xpls/abs_all.jsp?arnumber=1399425 [Accessed March 29, 2011].

Badler, N.I. & Glassner, A.S., 1997. 3D object modeling. SIGGRAPH 97 Introduction to Computer Graphics Course Notes. Available at: http://gamma.cs.unc.edu/courses/graphics-s09/LECTURES/3DModels_SurveyPaper.pdf [Accessed December 10, 2012].

Bagshaw, A.P. et al., 2003. Electrical impedance tomography of human brain function using reconstruction algorithms based on the finite element method. NeuroImage, 20(2), pp.752-64. Available at: http://www.ncbi.nlm.nih.gov/pubmed/14568449 [Accessed July 18, 2011].

Barber, D.C. & Brown, B.H., 1988. Errors in reconstruction of resistivity images using a linear reconstruction technique. Clinical physics and physiological measurement, 9 Suppl A(4), pp. 101-4. Available at: http://www.ncbi.nlm.nih.gov/pubmed/3240636.

Bayford, R.H. et al., 2008. Development of a neonate lung reconstruction algorithm using a wavelet AMG and estimated boundary form. Physiological Measurement, 29(6), p.S125. Available at: http://iopscience.iop.org/0967-3334/29/6/S11 [Accessed March 1, 2011].

Bayford, R.H. et al., 2007. Reconstruction algorithms to monitor neonate lung function. In H. Scharfetter & R. Merwa, eds. 13th International Conference on Electrical Bioimpedance and the 8th Conference on Electrical Impedance Tomography. Springer, pp. 352-355. Available at: http://www.springerlink.com/index/T62628M404R63007.pdf [Accessed March 1, 2011].

Bayford, R.H., 2006. Bioimpedance tomography (electrical impedance tomography). Annual Review of Biomedical Engineering, 8(1), pp.63-91. Available at: http://www.annualreviews.org/doi/full/10.1146/annurev.bioeng.8.061505.095 716 [Accessed July 16, 2010].

Bayford, R.H. et al., 2001. Solving the forward problem in electrical impedance tomography for the human head using IDEAS (integrated design engineering analysis software), a finite element modelling tool. Physiological Measurement, 22(1), p.55. Available at: http://iopscience.iop.org/0967-3334/22/1/308 [Accessed March 1, 2011].

Baysal, U. & Eyüboğlu, B.M., 2000. Tissue resistivity estimation in the presence of positional and geometrical uncertainties. *Physics in medicine and biology,* 45(8), pp.2373-88. Available at: http://www.ncbi.nlm.nih.gov/pubmed/10958201.

Borsic, A. et al., 2001. Realistic 2D human thorax modelling for EIT. Physiological measurement, 22(1), pp.77-83. Available at: http://www.ncbi.nlm.nih.gov/pubmed/11236892.

Borsic, A., 2002. Regularisation methods for imaging from electrical measurements. Oxford Brookes University. Available at: http://engineering.dartmouth.edu/~andrea_borsic/documents/AndreaBorsic-DoctoralThesis.pdf [Accessed March 29, 2011].

Bouhemad, B. et al., 2007. Clinical review: Bedside lung ultrasound in critical care practice. Critical care (London, England), 11(1), p.205. Available at: http://www.pubmedcentral.nih.gov/articlerender.fcgi?artid=2151891&tool=pmcentrez&rendertype=abstract [Accessed July 30, 2012].

Braude, I. et al., 2007. Contour-based surface reconstruction using MPU implicit models. Graphical models, 69(2), pp.139-157. Available at: http://www.sciencedirect.com/science/article/pii/S1524070306000683 [Accessed December 8, 2012].

Breckon, W.R. & Pidcock, M.K., 1987. Mathematical aspects of impedance imaging. Clinical physics and physiological measurement : an official journal of the Hospital Physicists' Association, Deutsche Gesellschaft für Medizinische Physik and the European Federation of Organisations for Medical Physics, 8 Suppl A, pp.77-84. Available at: http://www.ncbi.nlm.nih.gov/pubmed/3240650.

Breckon, W.R. & Pidcock, M.K., 1988. Data errors and reconstruction algorithms in electrical impedance tomography. Clinical physics and physiological measurement: an official journal of the Hospital Physicists' Association, Deutsche Gesellschaft für Medizinische Physik and the European Federation of Organisations for Medical Physics, 9 Suppl A, pp. 105-9. Available at: http://www.ncbi.nlm.nih.gov/pubmed/3240637.

Brown, B.H. et al., 2002a. Neonatal lungs-can absolute lung resistivity be determined non-invasively? Medical and Biological Engineering and Computing, 40(4), pp.388-394. Available at: http://www.spnngerlink.com/index/WIG010673L036672.pdf [Accessed March 9, 2011].

Brown, B.H. et al., 2002b. Neonatal lungs: maturational changes in lung resistivity spectra. Medical and Biological Engineering and Computing, 40(5), pp.506-511. Available at: http://www.springerlink.com/index/27137067M8787132.pdf [Accessed March 9, 2011].

Brown B and Seagar A 1987 The Sheffield data collection system Clinical Physics and Physiological Measurements 8 91-97

Brown, B.H., Barber, D.C. & Seagar, A.D., 1985. Applied potential tomography: possible clinical applications. Clinical Physics and Physiological Measurement, 6(2), p.109. Available at: http://iopscience.iop.org/0143-0815/6/2/002 [Accessed March 1, 2011].

Brown, B.H. & Barber, D.C., 1988. Possibilities and problems of real-time imaging of tissue resistivity. Clinical Physics and Physiological Measurement, 9(Suppl. A), p.121. Available at: http://iopscience.iop.org/0143-0815/9/4A/020 [Accessed March 1, 2011].

Caiulo, V.A. et al., 2011. Usefulness of lung ultrasound in a newborn with pulmonary atelectasis. La Pediatria medica e chirurgica: Medical and surgical pediatrics, 33(5-6), pp.253-5. Available at: http://www.ncbi.nlm.nih.gov/pubmed/22428435.

Cherepenin, V.A. et al., 2002. Preliminary static EIT images of the thorax in health and disease. Physiological measurement, 23(1), pp.33-41. Available at: http://www.ncbi.nlm.nih.gov/pubmed/11876240.

Coates, A. et al., 1997. Measurement of lung volumes by plethysmography. European Respiratory Journal, 10(6), pp.1415-1427. Available at: http://www.ersj.org.uk/content/10/6/1415.full.pdf [Accessed March 29, 2011].

Crelin, E.S., 1973. Lung and pleura. In Functional Anatomy of the Newborn. Yale University Press,Ltd. London. Available at: http://books.google.co.uk/books?id=wBHqbPtG1_oC&pg=PA39&lpg=PA39 &dq=functional+anatomy+lungs+of+newborn&source=bl&ots=4WGhNw-s6x&sig=JrF3GSONEXltgcyMKarcv58bduM&hl=en&sa=X&ei=CGrOT8Xk HMnC0QWGvayTBw&ved=0CEsQ6AEwAA#v=onepage&q&f=false.

Cuardo, R. et al., 1990. Rationale For A Rigid Electrode Frame In Electrical Impedance Tomography. Annual International Conference of the IEEE Engineering in Medicine and Biology Society, 12(1), pp.126-127. Available at: http://ieeexplore.ieee.org/xpls/abs_all.jsp?arnumber=691003 [Accessed March 29, 2011].

Dai, T., Gómez-Laberge, C. & Adler, A., 2008. Reconstruction of conductivity changes and electrode movements based on EIT temporal sequences. Physiological measurement, 29(6), pp.S77-88. Available at: http://www.ncbi.nlm.nih.gov/pubmed/18544802.

Division, B.A.M.E., 2007. Obstetric and Newborn Care II-Chapter 10: The Premature Infant. , 2009(July/07).

Downie, J. & Marshall, J., 2007. Pediatric Neuroimaging Ethics. Cambridge Quarterly of Healthcare Ethics, 16(2), pp. 147-160. Available at: http://www.neuroethics.ca/downloads/Paediatric Neuroimaging Ethics.pdf.

Dunnet, D., 1969. A technique of finte strain analysis using elliptical particles. Tectonophysics, 7, pp.117-136.

Fenner, A. et al., 1973. Periodic Breathing in Premature and Neonatal Babies: Incidence, Breathing Pattern, Respiratory Gas Tensions, Response to Changes in the Composition of Ambient Air. Pediatric Research, 7(4), pp.174-183. Available at: http://journals.lww.com/pedresearch/Fulltext/1973/04000/Periodic_Breathing _in_Premature_and_Neonatal.20.aspx.

Fraser, J., Walls, M. & McGuire, W., 2004. Respiratory complications of preterm birth. BMJ (Clinical research ed.), 329(7472), pp.962-5. Available at: http://www.bmj.com[Accessed November 8, 2010].

Frerichs, I. et al., 2001. Non-invasive radiation-free monitoring of regional lung ventilation in critically ill infants. Intensive Care Medicine, 27(8), pp.1385-1394. Available at: http://www.springerlink.com/openurl.asp?genre=article&id=doi:10.1007/s001 340101021 [Accessed June 28, 2010].

Ganong, W.F., 2005. Respiration: Pulmonary Function. In Singapore: The McGrow-Hill Companies, Inc., pp. 647-697.

Gersing, E., Hofmann, B. & Osypka, M., 1996. Influence of changing peripheral geometry on electrical impedance tomography measurements. Medical and Biological Engineering and Computing, 34(5), pp.359-361. Available at:

http://www.springerlink.com/index/957RH75G08474M70.pdf [Accessed March 29, 2011].

Gillman, L.M. & Kirkpatrick, A.W., 2012. Portable bedside ultrasound: the visual stethoscope of the 21st century. Scandinavian journal of trauma, resuscitation and emergency medicine, 20(1), p.18. Available at: http://www.pubmedcentral.nih.gov/articlerender.fcgi?artid=3352312&tool=p mcentrez&rendertype=abstract [Accessed July 30, 2012].

Gisser, D.G., Isaacson, D. & Newell, J.C., 1988. Theory and performance of an adaptive current tomography system. Clinical Physics and Physiological Measurement, 9 Suppl A(4), pp.35-41. Available at: http://iopscience.iop.org/0143-0815/9/4A/007 [Accessed March 29, 2011].

Grimnes, S. & Martinsen, Ø.G., 2000. Bioimpedance and bioelectricity basics, Academic press. Available at: http://books.google.com/books?hl=en&amp;lr=&amp;id=v3EuUjoqwkkC&a mp;oi=fnd&amp;pg=PP2&amp;dq=Bioimpedance+and+bioelectricity+basics &amp;ots=OWLcP8qi2i&amp;sig=tk2w6CQf4kulhCpDVqi85nvbrug [Accessed March 14, 2011].

Grychtol, B. et al., 2012. Impact of model shape mismatch on reconstruction quality in electrical impedance tomography. IEEE transactions on medical imaging, 31(9), pp.1754-60. Available at: http://www.ncbi.nlm.nih.gov/pubmed/22645263.

Hampshire, A.R. et al., 1995. Multifrequency and parametric EIT images of neonatal lungs. Physiological measurement, 16(3 Suppl A), pp.A175-89. Available at: http://www.ncbi.nlm.nih.gov/pubmed/8528116.

Hartwig, V. et al., 2009. Numerical Estimation of Peak/Average SAR Ratio for Different Thorax Models. In O. Dossel & W. C. Schlegel, eds. IFMBE, World Congress on Medical Physics and Biomedical Engineering. pp. 311-314

Heimler, R. et al., 1992. Effect of positioning on the breathing pattern of preterm infants. Archives of Disease in Childhood, 67(3), pp.312-314.

Holder, D.S., 2005a. Brief Introduction to Bioimpedance. In David S. Holder, ed. Electrical Impedance Tomography-Methods, History and Applications. Institute of Physics Publishing, pp. 411-422.

Holder, D.S., 2005b. Introduction to Biomedical Electrical Impedance Tomography. In David S Holder, ed. Electrical Impedance Tomography- Methods, History and Applications. IOP Publishing Ltd, pp. 423-450.

Ider, Y.Z. et al., 1992. Determination of the boundary of an object inserted into a water-filled cylinder. Clinical Physics and Physiological Measurement, 13(Suppl A), pp.151-154. Available at: http://iopscience.iop.org/0143-0815/13/A/029 [Accessed March 29, 2011].

Isaacson, D., Cheney, M. & Newell, J.C., 1999. Electrical impedance tomography. SIAM Review, 41(1), pp.85-101. Available at: http://www.ncbi.nlm.nih.gov/pubmed/1587116.

Kao, T.J. et al., 2006. A 3D reconstruction algorithm for EIT using a handheld probe for breast cancer detection. Physiological measurement, 27(5), p.S1. Available at: http://iopscience.iop.Org/0967-3334/27/5/S01 [Accessed March 29, 2011].

Kiber, M.A., 1991. Determination of object boundary shape for electrical impedance tomography. University of Sheffield, Dept. of Medical Physics and Clinical Engineering. Available at: http://scholar.google.com/scholar?hl=en&btnG=Search&q=intitle:Determinati on+of+object+boundary+shape+for+Electrical+Impedance+Tomography#0 [Accessed March 29, 2011].

Khor, J. M., et al., 2009. Development of a Sensor Network for Dynamic Boundary Measurement in Neonatal EIT, IFMBE Proceedings 25/II, pp.386-389

Kolehmainen, V., Lassas, M. & Ola, P., 2008. Electrical impedance tomography problem with inaccurately known boundary and contact impedances. Medical Imaging, IEEE Transactions on, 27(10), pp.1404-1414. Available at: http://ieeexplore.ieee.org/xpls/abs_all.jsp?arnumber=4471919 [Accessed March 9, 2011].

**44**

Li, J., 1994. A method of reducing the error caused by boundary shape and electrode positions in electrical impedance tomography. Physiological Measurement, 15(2A), pp.A169-74. Available at: http://iopscience.iop.org/0967-3334/15/2A/022 [Accessed March 29, 2011].

Lionheart, W.R.B., 1998. Boundary shape and electrical impedance tomography. Inverse problems, 14(1), pp.139-147. Available at: http://iopscience.iop.org/0266-5611/14/1/012 [Accessed March 29, 2011].

Maki, D.D., 1999. Recent Advances in Pulmonary Imaging. Chest, 116(5), pp.1388-1402. Available at: http://www.chestjournal.org/cgi/doi/10.1378/chest.116.5.1388 [Accessed December 15, 2010].

Marven, S.S. et al., 1996. Reproducibility of electrical impedance tomographic spectroscopy (EITS) parametric images of neonatal lungs. Physiological measurement, 17 Suppl 4, pp.A205-12. Available at: http://www.ncbi.nlm.nih.gov/pubmed/9001619.

Murphy, D. et al., 1987. Impedance imaging in the newborn. Clinical physics and physiological measurement, 8 Suppl A(4), pp.131-40. Available at: http://www.ncbi.nlm.nih.gov/pubmed/3568562.

Nissinen, A. et al., 2009. Compensation of errors due to discretization, domain truncation and unknown contact impedances in electrical impedance tomography. Measurement Science and Technology, 20(10), p.105504. Available at: http://stacks.iop.org/0957-0233/20/i=10/a=105504?key=crossref.02b35dbd0327dcae484cbb4e8a9e280f [Accessed May 18, 2011].

Otten, D.M., Onik, G. & Rubinsky, B., 2004. Distributed network imaging and electrical impedance tomography of minimally invasive surgery. Technology in cancer research & treatment, 3(2), pp.125-34. Available at: http://www.ncbi.nlm.nih.gov/pubmed/15059018.

Rahal, M., et al., 2009. A comparison study of electrodes for neonate electrical impedance tomography. Physiol. Meas., 30 (2009), S73-S84

Riu, P.J., Rosell, J. & Pallás-Areny, R., 1992. In vivo static imaging for the real and the reactive parts in electrical impedance tomography using multifrequency techniques. IEEE, 5(3), pp.1706-1707. Available at: http://ieeexplore.ieee.org/xpls/abs_all.jsp?arnumber=590101 [Accessed April 11, 2011].

Seidel, H.M. et al., 2009. Chest and Lungs. In Mosby's Guide to Physical Examination, 7th Edition. Evolve, pp. 332-377. Available at: http://www.coursewareobjects.com/objects/evolve/E2/bookj3ages/seidel/images/Seidel_Chap13_MAIN.pdf.

Seydnejad, S.R. & Fahimi, H., 1994. A new method for finding the exact electrode location and body perimeter used for EIT. Proceedings of 16th Annual International Conference of the IEEE Engineering in Medicine and Biology Society, pp.541-542. Available at: http://ieeexplore.ieee.org/lpdocs/epic03/wrapper.htm?arnumber=411913.

Smallwood, R.H. et al., 1999. A comparison of neonatal and adult lung impedances derived from EIT images. Physiological measurement, 20(4), pp.401-13. Available at: http://www.ncbi.nlm.nih.gov/pubmed/10593233.

Soleimani, M., Abascal, J. & Lionheart, W.R.B., 2004. Simultaneous reconstruction of the boundary shape and conductivity in 3D electrical impedance tomography. In Proceedings of the XII International Conference on Electrical Bio-Impedance and Electrical Impedance Tomography, 20-24 June, 2004, Gdansk, Poland. University of Bath, pp. 475-478. Available at: http://opus.bath.ac.uk/12191/ [Accessed May 18, 2011].

Somersalo, E., et al., 1992, Existence and Uniqueness for Electrode Models for Electric Current Computed Tomography, SIAM J. Appl. Math., 52(4), 1023-1040

Starck, J.R. et al., 1999. An inexpensive sensor for measuring surface geometry. Medical engineering & physics, 21(10), pp.725-9. Available at: http://www.ncbi.nlm.nih.gov/pubmed/10717552.

Stocks, J. et al., 2001. Plethysmographic measurements of lung volume and airway resistance. European Respiratory Journal, 17(2), pp.302-312.

Taktak, A. et al., 1995. Practical factors in neonatal lung imaging using electrical impedance tomography. Medical and Biological Engineering and Computing, 33(2), pp.202-205. Available at: http://www.springerlink.com/index/V1Q3611344R37310.pdf [Accessed March 14, 2011].

Taktak, A. et al., 1996. Feasibility of neonatal lung imaging using electrical impedance tomography. Early human development, 44(2), pp.131-138. Available at: http://linkinghub.elsevier.com/retrieve/pii/0378378295017003 [Accessed March 14, 2011].

Tang, M. et al., 2002. Effects of incompatible boundary information in EIT on the convergence behavior of an iterative algorithm. IEEE transactions on medical imaging, 21(6), pp.620-8. Available at: http://www.ncbi.nlm.nih.gov/pubmed/12166858.

Tizzard, A. et al., 2005. Generating accurate finite element meshes for the forward model of the human head in EIT. Physiological Measurement, 26(2), p.S251. Available at: http://iopscience.iop.org/0967-3334/26/2/024 [Accessed March 29, 2011].

Tizzard, A. & Bayford, R.H., 2007. Improving the finite element forward model of the human head by warping using elastic deformation. Physiological Measurement, 28(7), pp.S163-S182.

Zhu, Q.S. et al., 1994. Development of a real-time adaptive current tomograph. Physiological measurement, 15 Suppl 2, pp.A37-43. Available at: http://www.ncbi.nlm.nih.gov/pubmed/8087048.

Zlochiver, S. et al., 2007. A portable bio-impedance system for monitoring lung resistivity. Medical engineering & physics, 29(1), pp.93-100. Available at: http://www.ncbi.nlm.nih.gov/pubmed/16546432 [Accessed July 16, 2010].

**Claims**

1. An apparatus for use in estimating the shape of a body part of a subject, which apparatus comprises:

   a string of sensors for positioning adjacent the body part so that the string of sensors substantially conforms with the shape of the body part, or at least a part of it, the string of sensors comprising:

   (a) at least one bend sensor having an electrical resistance that changes with bending, the at least one bend sensor being either:

   (i) a plurality of separate and distinct bend sensors arranged end to end; or
   (ii) one or a few bend sensors divided lengthwise into different sensing portions to provide an effect similar to the plurality of separate and distinct bend sensors;

   the length of each bend sensor or each sensing portion such that it bends approximately into a circular arc when positioned on the body part, but not so long that each bend sensor or sensing portion adopts a non-circular shape when conforming to the shape of the body part and not so short that the bend sensor or sensing portion is too stiff to conform to the shape of the body part; and
   (b) a single stretch sensor for joining the ends of the at least one bend sensor together;

   the arrangement being such that, in use, the at least one bend sensor lies adjacent a first region of the body part and the single stretch sensor lies adjacent a second region of the body part, wherein the magnitude of a curvature of the first region of the body part is greater than the magnitude of a curvature of the second region of the body part.

2. An apparatus as claimed in claim 1, wherein in use said string of sensors comprises a closed loop of sensors that, in use, circumscribes said body part
   or wherein said string of sensors has a length which is insufficient to circumscribe the body part, but which substantially conforms to a part of the shape of the body when positioned thereadjacent.

3. An apparatus as claimed in claim 1 or 2, wherein the single stretch sensor is arranged in the string of sensors so that, in use, it lies adjacent a substantially flat region of the body part;

and/or wherein said plurality of bend sensors or sensing portions numbers between 7 and 12, and preferably 8.

4. An apparatus as claimed in any of claims 1 to 3, wherein each bend sensor comprises a device that measures strain when bent, such as a conductive ink bend sensor;
and/or further comprising an array of openings around the perimeter thereof, each opening for permitting attachment of an electrode to the subject for the purpose of obtaining an EIT image of the body part.

5. An apparatus as claimed in any preceding claim, further comprising a plurality of said strings of sensors each string of sensors adjacent one another so as to provide shape estimates in different planes of the body part, whereby a three dimensional estimate of the shape of said body part may be estimated;
and optionally wherein each string of sensors is approximately the same size so as to accommodate a body part of substantially similar cross section with length along a region where the shape is to be estimated, such as the thorax of the subject;
and optionally wherein said plurality of strings of sensors comprises strings of sensors of various lengths, arranged so as to provide a shape for accommodating a body part, for example a non-similar cross section with length, such as a breast or head.

6. An apparatus as claimed in claim 5, further comprising one or more inter-string bend and/or stretch sensor connecting at least one string of sensors to at least one other string of sensors.

7. A computer-implemented method of generating an estimated shape of a body part using an apparatus as claimed in any of claims 1 to 6, which method comprises the steps of:

(a) reading at least one bend sensor output from each bend sensor or sensing portion, wherein each bend sensor output represents a curvature value of the curvature of the respective bend sensor or sensing portion;
(b) determining an angle subtended by the or each bend sensor or sensing portion using the at least one bend sensor output and a known length of the or each bend sensor or sensing portion;
(c) determining an angle subtended by the single stretch sensor using an estimated curvature for the single stretch sensor, which estimated curvature is determined based on a curvature value measured for a bend sensor adjacent the single stretch sensor;
(d) adding all angles determined in in steps (b) and (c) and comparing to a predetermined angle value; and
(e) adjusting each angle determined in steps (b) and (c) if the result of comparison in step (d) is that the sum of the angles is greater than or less than said predetermined angle value; and
(f) using said adjusted angles to generate an estimated shape of the body part.

8. A method according to claim 7, wherein said apparatus circumscribes the body part and said predetermined angle value is $+2\pi$ radians considered in an anti-clockwise direction and $-2\pi$ considered in a clockwise direction;
and/or wherein said step of adjusting comprises either multiplying all estimated included angles by the ratio of their actual sum and $-2\pi$ or $+2\pi$ radians, or by subtracting from each subtended angle the mean angular error being the difference of the sum of the angles and $-2\pi$ or $+2\pi$, divided by the total number of bend sensors or sensing portions, and the single stretch sensor.

9. A method according to claim 7 or 8, wherein step (f) comprises:

(a) using the adjusted angle values to define a curve with $i$ points, the gradient of the $i$th point in the curve given by

$$\left[\cos\left(\sum_{1}^{i}\theta_i\right) \quad \sin\left(\sum_{1}^{i}\theta_i\right)\right]$$

where $i$ is the number of bend sensors or sensing portions, and the single stretch sensor, and $\theta_i$ is the adjusted angle value for the $i$th bend sensor, sensing portion or single stretch sensor; and
(b) interpolating a spline through the $i$ points; and
(c) integrating the curve over its length, where the length is given by the sum of the lengths of each bend sensor or sensing portion and the measured length of the single stretch sensor; and
(d) multiplying the integrated curve by the length to provide an estimated shape of the body part.

**10.** A method according to claim 7 or 8, further comprising the step of using the adjusted angles to calculate respective adjusted curvature values for each bend sensor, or sensing portion, curvature value in step (a), and wherein step (f) comprises generating an estimated shape of the body part by adding arcs end-to-end in a co-ordinate space, each arc corresponding to a respective one of the adjusted curvature values;

and optionally wherein the first arc is generated by setting an initial point in the co-ordinate space at the end of a vector having a length equal to the radius of the corresponding adjusted curvature value, performing a plane rotation on the vector so that end of the vector sweeps through an arc to an end point, the arc having a length equal to the length of the corresponding bend sensor or sensing portion, and fitting the first arc between the initial point and the end point along the arc; and

repeating this process for the next bend sensor or sensing portion in the string, but with the initial point set to the end point of the previous vector in the co-ordinate space;

whereby arcs are added end-to-end so as to generated said estimated shape; and optionally wherein the process of adding arcs end-to-end proceeds unidirectionally around the string of sensors from a start point to an end point, the end point being close to the start point.

**11.** A method according to claim 10, wherein the process of adding arcs end-to-end proceeds bidirectionally around the string of sensors in opposite senses from a start point toward an end point or points, whereby respective portions of the estimated shape are generated separately;

and optionally wherein said end point or points in the co-ordinate space corresponds to the ends of the single stretch sensor in the string of sensors.

**12.** A method according to any of claims 7 to 11, wherein the apparatus comprises a plurality of strings of sensors of known separation between each string, wherein the method further comprises the steps of generating a two dimensional estimated shape for each string of sensors, stacking the two dimensional estimated shapes above or below one another in co-ordinate space, and blending the stacked two-dimensional estimated shapes into a three dimensional estimated shape, an optionally wherein the step of blending said two dimensional estimated shapes comprises contour stitching or a interpolating a spline, such as a cubic spline.

**13.** A computer-readable storing medium comprising executable instructions that when executed perform any of the method of any of claims 7 to 12.

**14.** A computer-implemented method of generating an EIT image of a body part of a subject, which method comprises the steps of:

(i) placing an apparatus as claimed in any of claims 1 to 6 onto a body part of a subject;
(ii) generating an estimated shape of the body part of a patient using a method as claimed in any of claims 7 to 12;
(iii) using the estimated shape in a forward model of the body part of the subject; and
(iv) generating an EIT image by solving the forward model, and displaying the EIT image;

and optionally further comprising the step of repeating steps (ii) to (iv) substantially continuously whereby EIT images are generated in real-time using real-time shape estimates of the body part of the subject.

**15.** A method of manufacturing an apparatus as claimed in any of claims 1 to 6 for use in estimating the shape of a body part of a subject, which method comprises the steps of:

(a) dividing into a first number of equal length segments a 2D shape that approximates the shape of the body part to be estimated;
(b) fitting one or more circular arcs to each segment of said first number of segments to generate an estimated shape that approximates said 2D shape;
(c) determining an error between said estimated shape and said 2D shape;
(d) repeating steps (a) to (c) with a second number of segments different to said first until a plurality of errors is generated;
(e) comparing one or more of said plurality of errors to identify the fewest number of segments required to reduce said error below a predetermined threshold; and
(f) constructing the apparatus with a number of bend sensors or sensing portions equal to the fewest number of segments in step (e).

**Patentansprüche**

1.  Vorrichtung zur Verwendung beim Schätzen der Form eines Körperteils eines Patienten, wobei die Vorrichtung umfasst:

    eine Reihe von Sensoren zum Positionieren benachbart zu dem Körperteil, so dass die Reihe von Sensoren im Wesentlichen mit der Form des Körperteils oder mindestens eines Teils davon übereinstimmt, wobei die Reihe von Sensoren umfasst:

    (a) mindestens einen Biegesensor der einen elektrischen Widerstand aufweist, der sich mit dem Biegen ändert, wobei der mindestens eine Biegesensor eine der beiden ist:

    (i) eine Vielzahl von getrennten und unterschiedlichen Biegesensoren, die in Aneinanderreihung aneinander angeordnet sind; oder
    (ii) ein oder mehrere Biegesensoren, die in Längsrichtung in verschiedene Sensorabschnitte unterteilt sind, um eine Wirkung zu erzielen, die ähnlich der Vielzahl von getrennten und unterschiedlichen Biegesensoren ist;

    die Länge jedes Biegesensors oder jedes Abtastabschnitts, so dass er sich bei Positionierung auf dem Körperteil etwa in einen Kreisbogen biegt, jedoch nicht so lang, dass jeder Biegesensor oder Abtastabschnitt eine nicht-kreisförmige Form annimmt, wenn er sich der Form des Körperteils anpasst, und nicht so kurz, dass der Biegesensor oder Abtastabschnitt zu steif ist, um sich der Form des Körperteils anzupassen; und
    (b) einen einzelnen Dehnungssensor zum Verbinden der Enden des mindestens einen Biegesensors miteinander;

    wobei die Anordnung so ist, dass im Gebrauch der mindestens eine Biegesensor angrenzend an einen ersten Bereich des Körperteils und der einzelne Dehnungssensor angrenzend an einen zweiten Bereich des Körperteils liegt, wobei die Stärke einer Krümmung des ersten Bereichs des Körperteils größer ist als die Stärke einer Krümmung des zweiten Bereichs des Körperteils.

2.  Vorrichtung nach Anspruch 1, wobei bei Verwendung die Reihe von Sensoren eine geschlossene Schleife von Sensoren umfasst, die bei Verwendung den Körperteil umschreibt, oder
    wobei die Reihe von Sensoren eine Länge aufweist, die nicht ausreicht, um den Körperteil zu umschreiben, die aber im Wesentlichen einem Teil der Form des Körpers entspricht, wenn sie dort benachbart angeordnet ist.

3.  Vorrichtung nach Anspruch 1 oder 2, wobei der einzelne Dehnungssensor in der Reihe von Sensoren so angeordnet ist, dass er bei Verwendung angrenzend an einen im Wesentlichen flachen Bereich des Körperteils liegt; und/oder
    wobei die Vielzahl von Biegesensoren oder Sensorabschnitten eine Anzahl zwischen 7 und 12 und vorzugsweise 8 aufweist.

4.  Vorrichtung nach einem der Ansprüche 1 bis 3, wobei jeder Biegesensor eine Einrichtung umfasst, die die Belastung beim Biegen misst, wie beispielsweise einen Biegesensor mit leitfähiger Tinte; und/oder
    ferner eine Anordnung von Öffnungen um seinen Umfang herum umfasst, wobei jede Öffnung eine Anbringung einer Elektrode an dem Patienten ermöglicht, um so ein EIT-Bild des Körperteils zu erhalten.

5.  Vorrichtung nach einem der vorangegangenen Ansprüche, ferner umfassend eine Vielzahl von Reihen von Sensoren, wobei die Reihen von Sensoren jeweils aneinander angrenzen, um Formschätzungen in verschiedenen Ebenen des Körperteils zu bieten, wobei eine dreidimensionale Schätzung der Form des Körperteils vorgenommen werden kann; und
    wobei wahlweise jede Reihe von Sensoren ungefähr die gleiche Größe hat, um ein Körperteil aufzunehmen mit im Wesentlichen ähnlichem Querschnitt mit einer Länge entlang einem Bereich, in dem die Form geschätzt werden soll, wie beispielsweise dem Thorax des Patienten; und
    wobei wahlweise die Vielzahl von Reihen von Sensoren Reihen von Sensoren verschiedener Längen umfasst, die so angeordnet sind, dass sie eine Form zur Aufnahme eines Körperteils bilden, zum Beispiel einen nicht ähnlichen Querschnitt mit einer Länge, wie beispielsweise Brust oder Kopf.

6.  Vorrichtung nach Anspruch 5, ferner umfassend einen oder mehrere dazwischenliegende Biege- und/oder Dehnungssensoren, die zumindest eine Reihe von Sensoren mit zumindest einer anderen Reihe von Sensoren verbinden.

7. Computerimplementiertes Verfahren zum Erzeugen einer geschätzten Form eines Körperteils unter Verwendung einer Vorrichtung nach einem der Ansprüche 1 bis 6, wobei das Verfahren die folgenden Schritte umfasst:

(a) Lesen mindestens einer Ausgabe eines Biegesensors von jedem Biegesensor oder Abtastabschnitt, wobei die Ausgabe jedes Biegesensors einen Krümmungswert der Krümmung des jeweiligen Biegesensors oder Abtastabschnitts darstellt;
(b) Bestimmen eines Winkels, der von dem oder von jedem Biegesensor oder Abtastabschnitt geschnitten wird, unter Verwendung der mindestens einen Ausgabe des Biegesensors und einer bekannten Länge des oder jedes Biegesensors oder Abtastabschnitts;
(c) Bestimmen eines Winkels, der durch den einzelnen Dehnungssensor geschnitten wird, unter Verwendung einer geschätzten Krümmung für den einzelnen Dehnungssensor, wobei die geschätzte Krümmung auf Grundlage eines Krümmungswerts bestimmt wird, der für einen Krümmungssensor neben dem einzelnen Dehnungssensor gemessen wird;
(d) Addieren aller in den Schritten (b) und (c) bestimmten Winkel und Vergleichen mit einem vorbestimmten Winkelwert; und
(e) Anpassen eines jeden in den Schritten (b) und (c) bestimmten Winkels, wenn das Ergebnis des Vergleichs in Schritt (d) darin besteht, dass die Summe der Winkel größer oder kleiner als der vorgegebene Winkelwert ist; und
(f) Verwenden der angepassten Winkel, um eine geschätzte Form des Körperteils zu erzeugen.

8. Verfahren nach Anspruch 7, wobei die Vorrichtung den Körperteil umschreibt und der vorgegebene Winkelwert $+2\pi$ Bogenmaß ist, in einer Richtung gegen den Uhrzeigersinn betrachtet, und $-2\pi$ in einer Richtung im Uhrzeigersinn betrachtet; und/oder
wobei der Schritt des Anpassens entweder das Multiplizieren aller geschätzten beinhalteten Winkel mit dem Verhältnis ihrer tatsächlichen Summe und $-2\pi$ oder $+2\pi$ Bogenmaß oder das Subtrahieren von jedem geschnittenen Winkel umfasst, wobei der mittlere Winkelfehler die Differenz aus der Summe der Winkel und $-2\pi$ oder $+2\pi$, dividiert durch die Gesamtzahl der Biegesensoren oder Abtastabschnitte, und des einzelnen Dehnungssensors ist.

9. Verfahren nach Anspruch 7 oder 8, wobei Schritt (f) umfasst:

(a) Verwenden der angepassten Winkelwerte, um eine Kurve mit $i$ Punkten zu definieren, wobei die Steigung des $i$-ten Punktes in der Kurve gegeben ist durch

$$\left[ \cos\left( \sum_{1}^{i} \theta_i \right) \quad \sin\left( \sum_{1}^{i} \theta_i \right) \right]$$

wobei $i$ die Anzahl der Biegesensoren oder Abtastabschnitte und des einzelnen Dehnungssensors ist, und $\theta_i$ der angepasste Winkelwert für den $i$-ten Biegesensor, Abtastabschnitt oder einzelnen Dehnungssensor ist; und
(b) Interpolieren eines Spline durch die $i$ Punkte; und
(c) Integrieren der Kurve über ihre Länge, wobei die Länge durch die Summe der Längen jedes Biegesensors oder Abtastabschnitts und der gemessenen Länge des einzelnen Dehnungssensors gegeben ist; und
(d) Multiplizieren der integrierten Kurve mit der Länge, um eine geschätzte Form des Körperteils bereitzustellen.

10. Verfahren nach Anspruch 7 oder 8, ferner umfassend den Schritt des Verwendens der angepassten Winkel zum Berechnen der jeweiligen angepassten Krümmungswerte für jeden Biegesensor oder Abtastabschnitt, Krümmungswert in Schritt (a), und wobei Schritt (f) das Erzeugen einer geschätzten Form des Körperteils durch Hinzufügen von Bögen in Aneinanderreihung in einem Koordinatenraum umfasst, wobei jeder Bogen einem jeweiligen der eingestellten Krümmungswerte entspricht; und
wobei wahlweise der erste Bogen erzeugt wird, indem ein Anfangspunkt im Koordinatenraum am Ende eines Vektors mit einer Länge gleich dem Radius des entsprechenden eingestellten Krümmungswertes gesetzt wird, eine Ebenendrehung an dem Vektor durchgeführt wird, so dass das Ende des Vektors durch einen Bogen zu einem Endpunkt verläuft, wobei der Bogen eine Länge gleich der Länge des entsprechenden Biegesensors oder Abtastabschnitts aufweist, und der erste Bogen zwischen dem Anfangspunkt und dem Endpunkt entlang des Bogens eingepasst wird; und

Wiederholen dieses Prozesses für den nächsten Biegesensor oder Abtastabschnitt in der Reihe, jedoch mit dem Anfangspunkt auf den Endpunkt des vorherigen Vektors im Koordinatenraum gesetzt;

wodurch Bögen aneinandergereiht werden, um die geschätzte Form zu erzeugen; und

wobei wahlweise der Prozess des Hinzufügens von Bögen in Aneinanderreihung unidirektional um die Reihe von Sensoren von einem Anfangspunkt zu einem Endpunkt verläuft, wobei der Endpunkt nahe dem Anfangspunkt liegt.

11. Verfahren nach Anspruch 10, wobei der Prozess des Hinzufügens von Bögen in Aneinanderreihung bidirektional um die Reihe von Sensoren in entgegengesetzten Richtungen von einem Startpunkt zu einem oder mehreren Endpunkten verläuft, wodurch entsprechende Abschnitte der geschätzten Form getrennt erzeugt werden; und

wobei wahlweise der oder die Endpunkte im Koordinationsraum den Enden des einzelnen Dehnungssensors in der Reihe von Sensoren entsprechen.

12. Verfahren nach einem der Ansprüche 7 bis 11, wobei die Vorrichtung eine Vielzahl von Reihen von Sensoren mit der bekannten Trennung zwischen jeder Reihe umfasst, wobei das Verfahren des Weiteren Folgendes umfasst: die Schritte des Erzeugens einer zweidimensionalen geschätzten Form für jede Reihe von Sensoren, Stapeln der zweidimensionalen geschätzten Formen übereinander oder untereinander im Koordinatenraum und Vermengen der gestapelten zweidimensionalen geschätzten Formen in eine dreidimensionale geschätzte Form, und wobei wahlweise die Schritt des Vermengens der zweidimensionalen geschätzten Formen Konturheften oder ein Interpolieren eines Splines, wie beispielsweise eines kubischen Splines, umfasst.

13. Computerlesbares Speichermedium, umfassend ausführbare Anweisungen, die bei Ausführung eines der Verfahren nach einem der Ansprüche 7 bis 12 durchführen.

14. Computerimplementiertes Verfahren zum Erzeugen eines EIT-Bildes eines Körperteils eines Patienten, wobei das Verfahren die folgenden Schritte umfasst:

(i) Anbringen einer Vorrichtung nach einem der Ansprüche 1 bis 6 an einem Körperteil einer Person;
(ii) Erzeugen einer geschätzten Form des Körperteils eines Patienten unter Verwendung eines Verfahrens nach einem der Ansprüche 7 bis 12;
(iii) Verwenden der geschätzten Form in einem Vorwärtsmodell des Körperteils des Patienten; und
(iv) Erzeugen eines EIT-Bildes durch Auflösen des Vorwärtsmodells und Anzeigen des EIT-Bildes; und

wahlweise ferner umfassend den Schritt des Wiederholens der Schritte (ii) bis (iv), im Wesentlichen kontinuierlich, wodurch EIT-Bilder in Echtzeit unter Verwendung von Echtzeit-Formschätzungen des Körperteils des Patienten erzeugt werden.

15. Verfahren zur Herstellung einer Vorrichtung nach einem der Ansprüche 1 bis 6 zur Verwendung beim Schätzen der Form eines Körperteils eines Patienten, wobei das Verfahren die folgenden Schritte umfasst:

(a) Teilen einer 2D-Form, die der Form des zu schätzenden Körperteils angenähert ist, in eine erste Anzahl von gleichlangen Segmenten;
(b) Anpassen eines oder mehrerer Kreisbögen an jedes Segment der ersten Anzahl von Segmenten, um eine geschätzte Form zu erzeugen, die der 2D-Form angenähert ist;
(c) Bestimmen eines Fehlers zwischen der geschätzten Form und der 2D-Form;
(d) Wiederholen der Schritte (a) bis (c) mit einer zweiten Anzahl von Segmenten, die sich von der ersten unterscheidet, bis eine Vielzahl von Fehlern erzeugt wird;
(e) Vergleichen eines oder mehrerer der Vielzahl von Fehlern, um die geringste Anzahl von Segmenten zu identifizieren, die erforderlich ist, um den Fehler unter einen vorbestimmten Schwellenwert zu reduzieren; und
(f) Konstruieren der Vorrichtung mit einer Anzahl von Biegesensoren oder Abtastabschnitten, die der geringsten Anzahl von Segmenten in Schritt (e) entspricht.

**Revendications**

1. Appareil à utiliser dans l'estimation de la forme d'une partie corporelle d'un sujet, lequel appareil comprend :

une chaîne de capteurs pour le positionnement adjacent de la partie corporelle de sorte que la chaîne de capteurs se conforme sensiblement à la forme de la partie corporelle, ou au moins une partie de celle-ci, la chaîne de capteurs comprenant :

(a) au moins un capteur de cintrage dont une résistance électrique change avec le cintrage, l'au moins un capteur de cintrage étant soit :

(i) une pluralité de capteurs de cintrage séparés et distincts agencés bout à bout ; soit
(ii) un ou quelques capteurs de cintrage divisés dans le sens de la longueur en différentes portions captrices pour assurer un effet similaire à la pluralité de capteurs de cintrage séparés et distincts ;

la longueur de chaque capteur de cintrage ou de chaque portion captrice est telle qu'elle se cintre approximativement en un arc circulaire lorsqu'elle est positionnée sur la partie corporelle, mais pas aussi longue pour que chaque capteur de cintrage ou portion captrice adopte une forme non circulaire lorsqu'elle se conforme à la forme de la partie corporelle et pas aussi courte pour que le capteur de cintrage ou la portion captrice soit trop raide pour se conformer à la forme de la partie corporelle ; et
(b) un capteur d'étirement unique destiné à joindre les bouts d'au moins un capteur de cintrage ensemble ;

l'agencement étant tel que, en utilisation, l'au moins un capteur de cintrage se trouve adjacent à une première région de la partie corporelle et le capteur d'étirement unique se trouve adjacent à une seconde région de la partie corporelle, dans lequel la grandeur d'une courbure de la première région de la partie corporelle est plus grande que la grandeur d'une courbure de la seconde région de la partie corporelle.

2. Appareil selon la revendication 1, dans lequel en utilisation, ladite chaîne de capteurs comprend une boucle fermée de capteurs qui, en utilisation, circonscrit ladite partie corporelle
ou dans lequel ladite chaîne de capteurs a une longueur qui est insuffisante pour circonscrire la partie corporelle, mais qui se conforme sensiblement à une partie de la forme du corps lorsqu'elle est positionnée adjacente à celle-ci.

3. Appareil selon la revendication 1 ou 2, dans lequel le capteur d'étirement unique est agencé dans la chaîne de capteurs de sorte que, en utilisation, il se trouve adjacent à une région sensiblement plate de la partie corporelle ; et/ou dans lequel ladite pluralité de capteurs de cintrage ou de portions captrices est au nombre compris entre 7 et 12, et de préférence de 8.

4. Appareil selon l'une quelconque des revendications 1 à 3, dans lequel chaque capteur de cintrage comprend un dispositif qui mesure la charge lorsqu'il est cintré, tel qu'un capteur de cintrage à encre conductrice ; et/ou comprenant en outre un réseau d'ouvertures autour de son périmètre, chaque ouverture permettant la fixation d'une électrode au sujet dans le but d'obtenir une image par tomographie d'impédance électrique de la partie corporelle.

5. Appareil selon une quelconque revendication précédente, comprenant en outre une pluralité desdites chaînes de capteurs, chaque chaîne de capteurs étant adjacente à une autre de manière à fournir des estimées de forme dans différents plans de la partie corporelle, moyennant quoi une estimée tridimensionnelle de la forme de ladite partie corporelle peut être estimée ;

et facultativement dans lequel chaque chaîne de capteurs est approximativement de la même taille de manière à accueillir une partie corporelle de section transversale sensiblement similaire avec une longueur suivant une région où la forme doit être estimée, telle que le thorax du sujet ;
et facultativement dans lequel ladite pluralité de chaînes de capteurs comprend des chaînes de capteurs de longueurs variables, agencées de manière à fournir une forme visant à accueillir une partie corporelle, par exemple une section transversale non similaire avec une longueur, telle qu'un sein ou la tête.

6. Appareil selon la revendication 5, comprenant en outre un ou plusieurs capteurs de cintrage et/ou d'étirement inter-chaîne reliant au moins une chaîne de capteurs à au moins une autre chaîne de capteurs.

7. Procédé implémenté par ordinateur de génération d'une forme estimée d'une partie corporelle à l'aide d'un appareil tel que revendiqué dans l'une quelconque des revendications 1 à 6, lequel procédé comprend les étapes de :

(a) lecture d'au moins une sortie de capteur de cintrage provenant de chaque capteur de cintrage ou chaque portion captrice, dans lequel chaque sortie de capteur de cintrage représente une valeur de courbure de la courbure du capteur de cintrage ou de la portion captrice respectif (respective) ;

(b) la détermination d'un angle sous-tendu par le (la) ou chaque capteur de cintrage ou portion captrice à l'aide de l'au moins une sortie de capteur de cintrage et une longueur connue du (de la) ou de chaque capteur de cintrage ou portion captrice ;

(c) la détermination d'un angle sous-tendu par le capteur d'étirement unique à l'aide d'une courbure estimée pour le capteur d'étirement unique, laquelle courbure estimée est déterminée d'après une valeur de courbure mesurée pour un capteur de cintrage adjacent au capteur d'étirement unique ;

(d) l'addition de tous les angles déterminés dans les étapes (b) et (c) et la comparaison à une valeur d'angle prédéterminée ; et

(e) l'ajustement de chaque angle déterminé dans les étapes (b) et (c) si le résultat de la comparaison dans l'étape (d) est que la somme des angles est plus grande ou plus petite que ladite valeur d'angle prédéterminée ; et

(f) l'utilisation desdits angles ajustés pour générer une forme estimée de la partie corporelle.

**8.** Procédé selon la revendication 7, dans lequel ledit appareil circonscrit la partie corporelle et ladite valeur d'angle prédéterminée est de +2π radians considéré dans le sens antihoraire et -2π considéré dans un sens horaire ; et/ou dans lequel ladite étape d'ajustement comprend soit la multiplication de tous les angles inclus estimés par le rapport de leur somme effective et -2π ou +2π radians, ou par soustraction de chaque angle sous-tendu de l'erreur angulaire moyenne qui est la différence de la somme des angles et -2π ou +2π, divisée par le nombre total de capteurs de cintrage ou de portions captrices, et le capteur d'étirement unique.

**9.** Procédé selon la revendication 7 ou 8, dans lequel l'étape (f) comprend :

(a) l'utilisation des valeurs d'angle ajusté pour définir une courbe avec $i$ points, le gradient du $i^{ème}$ point dans la courbe étant donnée par

$$\left[\cos\left(\sum_1^i \theta_i\right) \quad \sin\left(\sum_1^i \theta_i\right)\right]$$

où $i$ est le nombre de capteurs de cintrage ou de portions captrices, et le capteur d'étirement unique, et $\theta_i$ est la valeur d'angle ajustée pour le $i^{ème}$ capteur de cintrage, la $i^{ème}$ portion captrice ou le capteur d'étirement unique ; et

(b) l'interpolation d'une spline à travers les $i$ points ; et

(c) l'intégration de la courbe sur sa longueur, où la longueur est donnée par la somme des longueurs de chaque capteur de cintrage ou portion captrice et la longueur mesurée du capteur d'étirement unique ; et

(d) la multiplication de la courbe intégrée par la longueur pour fournir une forme estimée de la partie corporelle.

**10.** Procédé selon la revendication 7 ou 8, comprenant en outre l'étape d'utilisation des angles ajustés pour calculer des valeurs de courbure ajustées respectives pour chaque capteur de cintrage, ou chaque portion captrice, valeur de courbure dans l'étape (a), et dans lequel l'étape (f) comprend la génération d'une forme estimée de la partie corporelle en additionnant des arcs bout à bout dans un espace de coordonnées, chaque arc correspondant à une valeur respective des valeurs de courbure ajustées ;

et facultativement dans lequel le premier arc est généré en fixant un point initial dans l'espace de coordonnées au bout d'un vecteur ayant une longueur égale au rayon de la valeur de courbure ajustée correspondante, en réalisant une rotation dans le plan sur le vecteur pour que chaque bout du vecteur balaie un arc jusqu'à un point de fin, l'arc ayant une longueur égale à la longueur du capteur de cintrage ou de la portion captrice correspondant(e), et l'ajustement du premier arc entre le point initial et le point de fin le long de l'arc ; et

la répétition de ce processus pour le capteur de cintrage ou la portion captrice suivant(e) dans la chaîne, mais avec le point initial fixé au point de fin du vecteur précédent dans l'espace de coordonnées ; moyennant quoi des arcs sont ajoutés bout à bout de façon à générer ladite forme estimée ; et facultativement dans lequel le processus d'addition d'arcs bout à bout est conduit unidirectionnellement autour de la chaîne de capteurs d'un point de départ à un point de fin, le point de fin étant proche du point de départ.

**11.** Procédé selon la revendication 10, dans lequel le processus d'addition d'arcs bout à bout est conduit bidirectionnellement autour de la chaîne de capteurs dans des sens opposés d'un point de départ vers un point ou des points de fin, moyennant quoi des portions respectives de la forme estimée sont générées séparément ;

et facultativement dans lequel ledit point ou lesdits points de fin dans l'espace de coordonnées correspond (correspondent) aux bouts du capteur d'étirement unique dans la chaîne de capteurs.

12. Procédé selon l'une quelconque des revendications 7 à 11, dans lequel l'appareil comprend une pluralité de chaînes de capteurs de séparation connue entre chaque chaîne, dans lequel le procédé comprend en outre les étapes de génération d'une forme estimée bidimensionnelle pour chaque chaîne de capteurs, l'empilement des formes estimées bidimensionnelles au-dessus ou en dessous les uns des autres dans l'espace de coordonnées, et l'assimilation des formes estimées bidimensionnelles empilées en une forme estimée tridimensionnelle, et facultativement dans lequel l'étape d'assimilation desdites formes estimées bidimensionnelles comprend une piqûre de contour ou une interpolation d'une spline, telle qu'une spline cubique.

13. Support de stockage lisible par ordinateur comprenant des instructions exécutables qui, lorsqu'elles sont exécutées, réalisent l'un quelconque des procédés de l'une quelconque des revendications 7 à 12.

14. Procédé implémenté par ordinateur de génération d'une image de tomographie d'impédance électrique d'une partie corporelle d'un sujet, lequel procédé comprend les étapes de :

(i) placement d'un appareil tel que revendiqué dans l'une quelconque des revendications 1 à 6 sur une partie corporelle d'un sujet ;
(ii) génération d'une forme estimée de la partie corporelle d'un patient à l'aide d'un procédé tel que revendiqué dans l'une quelconque des revendications 7 à 12 ;
(iii) utilisation de la forme estimée dans un modèle prospectif de la partie corporelle du sujet ; et
(iv) génération d'une image de tomographie d'impédance électrique par résolution du modèle prospectif, et affichage de l'image de tomographie d'impédance électrique ;

et facultativement comprenant en outre l'étape de répétition des étapes (ii) à (iv) sensiblement continuellement, moyennant quoi des images de tomographie d'impédance électrique sont générées en temps réel à l'aide d'estimées de forme en temps réel de la partie corporelle du sujet.

15. Procédé de fabrication d'un appareil tel que revendiqué dans l'une quelconque des revendications 1 à 6 à utiliser dans l'estimation de la forme d'une partie corporelle d'un sujet, lequel procédé comprend les étapes de :

(a) division en un premier nombre de segments de longueur égale d'une forme en 2D qui approxime la forme de la partie corporelle à estimer ;
(b) ajustement d'un ou plusieurs arcs circulaires à chaque segment dudit premier nombre de segments pour générer une forme estimée qui approxime ladite forme en 2D ;
(c) détermination d'une erreur entre ladite forme estimée et ladite forme en 2D ;
(d) répétition des étapes (a) à (c) avec un second nombre de segments différent dudit premier jusqu'à ce qu'une pluralité d'erreurs soit générée ;
(e) comparaison d'un ou plusieurs de ladite pluralité d'erreurs pour identifier le plus petit nombre de segments requis pour réduire ladite erreur en dessous d'un seuil prédéterminé ; et
(f) construction de l'appareil avec un nombre de capteurs de cintrage ou de portions captrices égal au plus petit nombre de segments dans l'étape (e).

Voxel resistivities ρ

Electrode combinations

$$V1 = \rho_a \times S_{a1} + \rho_b \times S_{b1} + \rho_c \times S_{c1} + \rho_d \times S_{d1}$$
$$V2 = \ldots\ldots$$

## Fig. 1(a)

Voxel resistivities ρ

Electrode combination voltage

## Fig. 1(b)

Fig. 1(c)

Fig. 3(a)

Fig. 3(b)

Fig. 3(c)

Fig. 2

Constant current drive

Record potential difference

(1) (2) (3)

(4) (5) (6)

ELECTRODE POSITION

Fig. 2A

EP 3 035 846 B1

Fig. 4

Fig. 5

Fig. 6

Abrams Gentile Bend Sensors Mean Resistance vs. Radius (mm) with Standard Deviation Bar

Resistance ($\Omega$)

Radius (mm)

Sensor 1
$y = 105468x^{-0.4992}$
$R^2 = 0.9646$

Sensor 2
$y = 83572x^{-0.4329}$
$R^2 = 0.7999$

Key: ▲ Sensor 1
✳ Sensor 2
—— Power (Sensor 1)
- - - - Power (Sensor 2)

EP 3 035 846 B1

Fig. 7

Abrams Gentile Bend Sensors Mean Resistance vs. Curvature (1/radius) with Standard Deviation Bar

Resistance (Ω)

Curvature (1/mm)

70

| Sensor 1 | Sensor 2 | Key: |
|---|---|---|
| $y = 278250x + 9617$ | $y = 280139x + 9511$ | ▲ Sensor 1 |
| $R^2 = 0.979$ | $R^2 = 0.891$ | ✗ Sensor 2 |

Key: ▲ Sensor 1
✗ Sensor 2
---- Linear (Sensor 1)
—— Linear (Sensor 2)

EP 3 035 846 B1

# Flexpoint Bend Sensors Mean Resistance vs. Radius (mm) with Standard Deviation Bar

**Fig. 8**

Resistance (Ω) — Radius (mm)

80

Sensor 1
$y = 2E + 06x^{-1.3978}$
$R^2 = 0.9714$

Sensor 2
$y = 2E + 06x^{-1.5455}$
$R^2 = 0.944$

*Key:*
▲ Sensor 1
✗ Sensor 2
- - - - Power (Sensor 1)
——— Power (Sensor 2)

EP 3 035 846 B1

EP 3 035 846 B1

**Fig. 9**

### Flexpoint Bend Sensors Mean Resistance vs. Curvature (1/radius) with Standard Deviation Bar

Resistance (Ω)

Curvature (1/mm)

| Sensor 1 | Sensor 2 | Key: |
|---|---|---|
| $y = 5156.9e^{37.323x}$ | $y = 3060.4e^{39.679x}$ | ▲ Sensor 1 |
| $R^2 = 0.9321$ | $R^2 = 0.9244$ | ✕ Sensor 2 |

Key: ▲ Sensor 1
✕ Sensor 2
- - - - Expon. (Sensor 1)
——— Expon. (Sensor 2)

| Percentage (%) of resistance drift after 30s of bend position | | | | |
|---|---|---|---|---|
| | Abrams | | Flexpoint | |
| Radius (mm) | Sensor 1 | Sensor 2 | Sensor 1 | Sensor 2 |
| Flat | 3.60 | 0.15 | 0.91 | 0.28 |
| 65 | 2.22 | 2.70 | 4.57 | 2.28 |
| 50 | 2.91 | 5.52 | 3.56 | 0.48 |
| 45 | 5.57 | 6.68 | 3.71 | 4.64 |
| 40 | 7.44 | 2.37 | 1.93 | 4.73 |
| 35 | 3.40 | 3.60 | 10.59 | 24.67 |
| 30 | 5.53 | 4.79 | 12.44 | 22.18 |
| 25 | 4.53 | 1.40 | 1.20 | 19.22 |
| 20 | 2.80 | 8.47 | 3.70 | 16.60 |
| 15 | 6.76 | 0.51 | 10.69 | 3.63 |

## Fig. 10

| Resistance drift (%) at flat position after all bendings | | | |
|---|---|---|---|
| Abrams | | Flexpoint | |
| Sensor 1 | Sensor 2 | Sensor 1 | Sensor 2 |
| 4.21 | 6.38 | 21.40 | 9.19 |

## Fig. 11

| True Radius (mm) | Calculate radius and Error % | | | |
|---|---|---|---|---|
| | Abrams | | Flexpoint | |
| | Sensor 1 | Error % | Sensor 1 | Error % |
| 65 | 76.3 | 17.4 | 116.3 | 78.9 |
| 50 | 50.5 | 1.0 | 62.9 | 25.8 |
| 45 | 38.9 | 13.6 | 48.5 | 7.9 |
| 40 | 41.0 | 2.5 | 36.0 | 10.1 |
| 35 | 36.2 | 3.4 | 34.4 | 1.8 |
| 30 | 34.6 | 15.3 | 25.5 | 15.0 |
| 25 | 24.2 | 3.2 | 24.3 | 2.7 |
| 20 | 18.3 | 8.5 | 17.3 | 13.6 |
| 15 | 15.5 | 3.3 | 16.9 | 12.6 |

## Fig. 12

EP 3 035 846 B1

Fig. 13

(a) Vernier Caliper

$P_3^{initial} = P_2^{final}$

Bend sensor 2
$s_2$ — 142

$P_2^{initial} = P_1^{final}$

Bend sensor 3

$r_2$

Bend sensor 1
$s_1$

143

$o_2$

$r_1$

141

$\theta = \frac{S}{r}$

$P_1^{initial}$

$o_1$

Fig. 14

65

Fig. 15

Resistance vs. Curvature (1/radius)

Resistance (ohm)

Curvature (1/mm)

142
143
150
141

| Sensor 1 | Sensor 2 | Sensor 3 |
|---|---|---|
| $y = 278695.3 x + 8792$ | $y = 360309 x + 7571$ | $y = 356608.7 x + 7128$ |
| $R^2 = 0.996$ | $R^2 = 0.994$ | $R^2 = 0.975$ |

Key: ◆ Sensor 1
■ Sensor 2
▲ Sensor 3

EP 3 035 846 B1

Fig.16

## Mean resistance vs. stretched sensor length

Mean resistance ($\Omega$)

Stretched sensor length (mm)

30mm
$y = 106.83\,x - 2021.7$
$R^2 = 0.9841$

Key: ◆ 30mm
—— Linear (30mm)

EP 3 035 846 B1

Fig. 17(a)

Fig. 17(b)

Fig. 17(c)

Fig. 18

Fig. 19(a)

Fig. 19(b)

| Bend sensor length | DAQ | | DMM | |
|---|---|---|---|---|
| | Radius (mm) | Radius error (%) | Radius (mm) | Radius error (%) |
| 50mm | 52 | 15.6 | 53 | 17.8 |
| 25mm | 58 | 28.9 | 59 | 31.1 |
| 50mm | 57 | 26.7 | 52 | 15.6 |
| 50mm | 44 | -2.2 | 48 | 6.7 |
| 25mm | 64 | 42.2 | 66 | 46.7 |
| 50mm | 53 | 17.8 | 53 | 17.8 |

## Fig. 20

2-D shape generated from bend and stretch sensors output measured using DMM and DAQ compared with the true shape

## Fig. 21

Key: ------ True shape
— — — Measured from DMM
——— Measured from DAQ

# Error in distance (mm) from generated point to real circle point with and without error correction factor

**Fig. 22**

Sensor error correction factor :
- —◆— 0.81 (221)
- —□— 0.83 (222)
- —△— 0.85 (223)
- —✕— 0.87 (224)
- —✳— 0.89 (225)
- —◯— 0.9  (226)
- —+— 0.91 (227)
- —0— 0.93 (228)
- —o— 0.95 (229)
- —◇— No error correction

EP 3 035 846 B1

2 – dimentional real object shape compared with shapes generated based on bend and stretch sensor outputs measured from DMM and DAQ with a constant bend sensor error correction factor of 0.85

Fig. 23

Point position error generated from sensor outputs
measured via DMM and DAQ, without and with
sensor error correction factor of 0.85

240

Key: —◆— Measured by DAQ without sensor error correction (241)
—■— Measured by DMM without sensor error correction (242)
—▲— Measured br DAQ with a constant sensor error correction factor of 0.85 (243)
—✕— Measured by DMM with a constant sensor error correction factor of 0.85 (244)

Fig. 24

EP 3 035 846 B1

Fig. 25(a)

Fig. 25(b)

### Cross-sectional (top view) of baby doll's chest obtained from laser scan at belt level

**Fig. 26A**

**Fig. 26B**

Key: - - - - Stretch sensor at calculated stretched – length of 20mm
───── Bend sensors with length of 30mm

Fig. 27

# Fig. 28A

Key: - - - - Stretch sensor at calculated stretched – length of 20mm
———— Bend sensors with length of 30mm

# Fig. 28B

Fig. 29

| Sensor # | S1 | S2 | S3 | S4 | S5 | S6 | S7 | S8 |
|---|---|---|---|---|---|---|---|---|
| Radii (mm) | 33.8 | 41.5 | 25.7 | 71.7 | 33.8 | 46.1 | 38.5 | 56.8 |

| | Sensor # Radii (mm) | S1 33.8 | S2 41.5 | S3 25.7 | S4 71.7 | S5 33.8 | S6 46.1 | S7 38.5 | S8 56.8 |
|---|---|---|---|---|---|---|---|---|---|
| Radius error correction factor | Mean | 0.997 | 0.999 | 0.970 | 0.954 | 0.960 | 0.968 | 0.962 | 0.960 |
| | Max | 1.032 | 1.066 | 1.1´7 | 1.125 | 1.092 | 1.109 | 1.079 | 1.105 |
| | Min | 0.967 | 0.960 | 0.825 | 0.858 | 0.874 | 0.881 | 0.901 | 0.895 |

Fig. 30

Fig. 31A

Key: —— Cubic spine interpolation function plot
---- 2D line plot from series of rotations

Fig. 31B

Key: —— Cubic spine interpolation function plot
---- 2D line plot from series of rotations

Key: —— Cubic spine interpolation function plot
---- 2D line plot from series of rotations

## Fig. 31C

## Fig. 31D

## Fig. 32

Fig. 33

| Number of segments | Max. positional error factor (a=1) | Max. positional error (a=50mm) | Error percentage with respect to a (%) |
|---|---|---|---|
| 4 | 0.368 | 18.424 | 36.9 |
| 8 | 0.034 | 1.677 | 3.4 |
| 12 | 0.012 | 0.591 | 1.2 |
| 16 | 0.006 | 0.301 | 0.6 |
| 20 | 0.004 | 0.185 | 0.4 |

Fig. 38

## Number of sensors used for generation of ellipse with various semi-minor / major (b/a) ratios and their maximum positional error factor with respect to a=1

Fig. 34

EP 3 035 846 B1

Different number of segments for generation ellipse with various semi-minor / major (b/a) axis ratios and mean positional error with respect to a=1

Fig. 35

Key:
Number of segments
——— 4
– – – – 8
– · – · – 12
——– 16
·········· 20

EP 3 035 846 B1

Maximum positional error factor vs. b/a ratio of ellipse
with 12 number of segments

Fig. 36

Max positional error (mm) vs. Number of segments
for an ellipse with a=50mm, b=40mm

Fig. 37

## Mean error vs. number of segments of an ellipse with semimajor and semiminoraxis of 45mm and 35mm approximates baby doll's dimension

| Number of segments | 4 | 6 | 8 | 10 | 12 | 14 | 16 | 18 | 20 |
|---|---|---|---|---|---|---|---|---|---|
| Mean error (mm) | 14.37 | 3.51 | 1.17 | 0.57 | 0.35 | 0.24 | 0.18 | 0.14 | 0.11 |
| Segment length (mm) | 63.08 | 42.05 | 31.54 | 25.23 | 21.03 | 18.02 | 15.77 | 14.02 | 12.62 |

Mean error (mm)

Number of equal-length segments

## Fig. 39A

EP 3 035 846 B1

Fig. 39B

Fig. 40A

Fig. 40B

Fig. 40C

Fig. 41

Fig. 42A

Fig. 42B

Fig. 42E

Fig. 42F

Framenumber 5. Slice 1

430

431

-50　　　0　　　50

Full width at half maximum

## Fig. 44(a)

Framenumber 5. Slice 1

433

432

-50　　　0　　　50

Full width at half maximum

## Fig. 44(b)

Framenumber 5. Slice 1

434

435

-50　　　0　　　50

Full width at half maximum

## Fig. 44(c)

Fig. 44(a)

Fig. 44(b)

| Group | No. of infants | Age (weeks) | Mean ± std | | | |
|---|---|---|---|---|---|---|
| | | | Chest circumference (mm) | Chest Width (mm) | Chest depth (mm) | Crown-heel length (mm) |
| A | 14 | 5.00 ± 3.41 | 297 ± 33 | 100 ± 16 | 79 ± 18 | 389 ± 34 |
| B | 27 | 76.87 ± 19.24 | 483 ± 25 | 161 ± 16 | 114 ± 20 | 805 ± 49 |

Fig. 45

| Group | Age (weeks) | Chest circumference (mm) | Number of electrodes on chest circumference | Inter-distance between electrodes (mm) | Number of electrode rings (3D) |
|---|---|---|---|---|---|
| A | 5.00 ± 3.41 | 297 ± 33 | ~8 ≤ n ≤ ~10 | ~20 ≥ d ≥ ~15 | Max ~2 x 10 (H~50mm) |
| B | 76.87 ± 19.24 | 483 ± 25 | ~8 ≤ n ≤ ~12 | ~20 ≥ d ≥ ~25 | Max ~3 x 12 (H~80mm) |

Fig. 46

Fig. 47(a)

Fig. 47(b)

480
485
487
Electrode sites
Typical site for electrodes
484
485
Circumferential bend sensors
484
Axial bend sensors
483
482
487
486
481
Fastening e.g. Velcro
Stretch sensors

Fig. 48

Z - axis
487
Fixed point, $C_{1,1}$
Stretched length, a
$\alpha$
487
Stretched length, b
$\theta$
$C_{2,1}$
$\beta$
Stretched length, L
$C_{2,2}$
X - axis

Fig. 49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61867904

- US 5086785 A

### Non-patent literature cited in the description

- **ADLER, A. et al.** GREIT: a unified approach to 2D linear EIT reconstruction of lung images. *Physiological measurement,* 2009, vol. 30 (6), 35-55, http://www.ncbi.nlm.nih.gov/pubmed/19491438
- **ADLER, A. ; GUARDO, R. ; BERTHIAUME, Y.** Impedance imaging of lung ventilation: do we need to account for chest expansion? Biomedical Engineering. *IEEE Transactions,* 1996, vol. 43 (4), 414-420
- **AGRONS, G.A. et al.** Lung Disease in Premature Neonates: Radiologic-Pathologic Correlation1. *Radiographics,* 2005, vol. 25 (4), 1047, http://radiographics.rsna.com/content/25/4/1047.abstract
- Electrical impedance tomography using a 3-D boundary element inverse solution. **BABAEIZADEH, S. ; BROOKS, D.H. ; ISAACSON, D.** Signals, Systems and Computers, 2004. Conference Record of the Thirty-Eighth Asilomar Conference on. IEEE, 2004, 1595-1599
- **BADLER, N.I. ; GLASSNER, A.S.** 3D object modeling. *SIGGRAPH 97 Introduction to Computer Graphics Course Notes,* 1997, http://gamma.cs.unc.edu/courses/graphics-s09/LECTURES/3DModels_SurveyPaper.pdf
- **BAGSHAW, A.P. et al.** Electrical impedance tomography of human brain function using reconstruction algorithms based on the finite element method. *NeuroImage,* 2003, vol. 20 (2), 752-64, http://www.ncbi.nlm.nih.gov/pubmed/14568449
- **BARBER, D.C. ; BROWN, B.H.** Errors in reconstruction of resistivity images using a linear reconstruction technique. *Clinical physics and physiological measurement,* 1988, vol. 9 (A), 101-4, http://www.ncbi.nlm.nih.gov/pubmed/3240636
- **BAYFORD, R.H. et al.** Development of a neonate lung reconstruction algorithm using a wavelet AMG and estimated boundary form. *Physiological Measurement,* 2008, vol. 29 (6), S125, http://iopscience.iop.org/0967-3334/29/6/S11

- Reconstruction algorithms to monitor neonate lung function. **BAYFORD, R.H. et al.** 13th International Conference on Electrical Bioimpedance and the 8th Conference on Electrical Impedance Tomography. Springer, 2007, 352-355
- **BAYFORD, R.H.** Bioimpedance tomography (electrical impedance tomography). *Annual Review of Biomedical Engineering,* 2006, vol. 8 (1), 63-91, http://www.annualreviews.org/doi/full/10.1146/annurev.bioeng.8.061505.095 716
- **BAYFORD, R.H. et al.** Solving the forward problem in electrical impedance tomography for the human head using IDEAS (integrated design engineering analysis software), a finite element modelling tool. *Physiological Measurement,* 2001, vol. 22 (1), 55, http://iopscience.iop.org/0967-3334/22/1/308
- **BORSIC, A. et al.** Realistic 2D human thorax modelling for EIT. *Physiological measurement,* 2001, vol. 22 (1), 77-83, http://www.ncbi.nlm.nih.gov/pubmed/11236892
- **BORSIC, A.** Regularisation methods for imaging from electrical measurements. Oxford Brookes University, 2002
- **BOUHEMAD, B. et al.** Clinical review: Bedside lung ultrasound in critical care practice. *Critical care (London, England),* 2007, vol. 11 (1), 205, http://www.pubmedcentral.nih.gov/articlerender.fcgi?artid=2151891&tool=p mcentrez&rendertype=abstract
- **BRAUDE, I. et al.** Contour-based surface reconstruction using MPU implicit models. *Graphical models,* 2007, vol. 69 (2), 139-157, http://www.sciencedirect.com/science/article/pii/S1524070306000683
- **BRECKON, W.R. ; PIDCOCK, M.K.** Mathematical aspects of impedance imaging. *Clinical physics and physiological measurement : an official journal of the Hospital Physicists' Association, Deutsche Gesellschaft für Medizinische Physik and the European Federation of Organisations for Medical Physics,* 1987, vol. 8 (A), 77-84, http://www.ncbi.nlm.nih.gov/pubmed/3240650

- **BRECKON, W.R. ; PIDCOCK, M.K.** Data errors and reconstruction algorithms in electrical impedance tomography. *Clinical physics and physiological measurement: an official journal of the Hospital Physicists' Association, Deutsche Gesellschaft für Medizinische Physik and the European Federation of Organisations for Medical Physics,* 1988, vol. 9 (A), 105-9, http://www.ncbi.nlm.nih.gov/pubmed/3240637
- **BROWN, B.H. et al.** Neonatal lungs-can absolute lung resistivity be determined non-invasively?. *Medical and Biological Engineering and Computing,* 2002, vol. 40 (4), 388-394, http://www.spnngerlink.com/index/WlG010673L036672.pdf
- **BROWN, B.H. et al.** Neonatal lungs: maturational changes in lung resistivity spectra. *Medical and Biological Engineering and Computing,* 2002, vol. 40 (5), 506-511, http://www.springerlink.com/index/27137067M8787132.pdf
- **BROWN B ; SEAGAR A.** The Sheffield data collection system. *Clinical Physics and Physiological Measurements,* 1987, vol. 8, 91-97
- **BROWN, B.H. ; BARBER, D.C. ; SEAGAR, A.D.** Applied potential tomography: possible clinical applications. *Clinical Physics and Physiological Measurement,* 1985, vol. 6 (2), 109, http://iopscience.iop.org/0143-0815/6/2/002
- **BROWN, B.H. ; BARBER, D.C.** Possibilities and problems of real-time imaging of tissue resistivity. *Clinical Physics and Physiological Measurement,* 1988, vol. 9 (A), 121, http://iopscience.iop.org/0143-0815/9/4A/020
- **CAIULO, V.A. et al.** Usefulness of lung ultrasound in a newborn with pulmonary atelectasis. *La Pediatria medica e chirurgica: Medical and surgical pediatrics,* 2011, vol. 33 (5-6), 253-5, http://www.ncbi.nlm.nih.gov/pubmed/22428435
- **CHEREPENIN, V.A. et al.** Preliminary static EIT images of the thorax in health and disease. *Physiological measurement,* 2002, vol. 23 (1), 33-41, http://www.ncbi.nlm.nih.gov/pubmed/11876240
- **COATES, A. et al.** Measurement of lung volumes by plethysmography. *European Respiratory Journal,* 1997, vol. 10 (6), 1415-1427, http://www.ersj.org.uk/content/10/6/1415.full.pdf
- Lung and pleura. **CRELIN, E.S.** Functional Anatomy of the Newborn. Yale University Press,Ltd, 1973
- **CUARDO, R. et al.** Rationale For A Rigid Electrode Frame In Electrical Impedance Tomography. *Annual International Conference of the IEEE Engineering in Medicine and Biology Society,* 1990, vol. 12 (1), 126-127, http://ieeexplore.ieee.org/xpls/abs_all.jsp?arnumber=691003

- **DAI, T. ; GÓMEZ-LABERGE, C. ; ADLER, A.** Reconstruction of conductivity changes and electrode movements based on EIT temporal sequences. *Physiological measurement,* 2008, vol. 29 (6), S77-88, http://www.ncbi.nlm.nih.gov/pubmed/18544802
- Obstetric and Newborn Care II. **DIVISION, B.A.M.E.** The Premature Infant. 2007
- **DOWNIE, J. ; MARSHALL, J.** Pediatric Neuroimaging Ethics. *Cambridge Quarterly of Healthcare Ethics,* 2007, vol. 16 (2), 147-160, http://www.neuroethics.ca/downloads/Paediatric Neuroimaging Ethics.pdf
- **DUNNET, D.** A technique of finte strain analysis using elliptical particles. *Tectonophysics,* 1969, vol. 7, 117-136
- **FENNER, A. et al.** Periodic Breathing in Premature and Neonatal Babies: Incidence, Breathing Pattern, Respiratory Gas Tensions, Response to Changes in the Composition of Ambient Air. *Pediatric Research,* 1973, vol. 7 (4), 174-183, http://journals.lww.com/pedresearch/Fulltext/1973/04000/Periodic_Breathing _in_Premature_and_Neonatal.20.aspx
- **FRASER, J. ; WALLS, M. ; MCGUIRE, W.** Respiratory complications of preterm birth. *BMJ (Clinical research ed.),* 2004, vol. 329 (7472), 962-5, http://www.bmj.com
- **FRERICHS, I. et al.** Non-invasive radiation-free monitoring of regional lung ventilation in critically ill infants. *Intensive Care Medicine,* 2001, vol. 27 (8), 1385-1394, http://www.springerlink.com/openurl.asp?genre=article&id=doi:10.1007/s001 340101021
- **GANONG, W.F.** Respiration: Pulmonary Function. The McGrow-Hill Companies, Inc, 2005, 647-697
- **GERSING, E. ; HOFMANN, B. ; OSYPKA, M.** Influence of changing peripheral geometry on electrical impedance tomography measurements. *Medical and Biological Engineering and Computing,* 1996, vol. 34 (5), 359-361, http://www.springerlink.com/index/957RH75G08474M70.pdf
- **GILLMAN, L.M. ; KIRKPATRICK, A.W.** Portable bedside ultrasound: the visual stethoscope of the 21st century. *Scandinavian journal of trauma, resuscitation and emergency medicine,* 30 July 2012, vol. 20 (1), 18, http://www.pubmedcentral.nih.gov/articlerender.fcgi?artid=3352312&tool=p mcentrez&rendertype=abstract
- **GISSER, D.G. ; ISAACSON, D. ; NEWELL, J.C.** Theory and performance of an adaptive current tomography system. *Clinical Physics and Physiological Measurement,* 1988, vol. 9 (A), 35-41, http://iopscience.iop.org/0143-0815/9/4A/007
- **GRIMNES, S. ; MARTINSEN, Ø.G.** Bioimpedance and bioelectricity basics. Academic press, 2000

- **GRYCHTOL, B. et al.** Impact of model shape mismatch on reconstruction quality in electrical impedance tomography. *IEEE transactions on medical imaging,* 2012, vol. 31 (9), 1754-60, http://www.ncbi.nlm.nih.gov/pubmed/22645263
- **HAMPSHIRE, A.R. et al.** Multifrequency and parametric EIT images of neonatal lungs. *Physiological measurement,* 1995, vol. 16 (A), A175-89, http://www.ncbi.nlm.nih.gov/pubmed/8528116
- Numerical Estimation of Peak/Average SAR Ratio for Different Thorax Models. **HARTWIG, V. et al.** IFMBE, World Congress on Medical Physics and Biomedical Engineering. 2009, 311-314
- **HEIMLER, R. et al.** Effect of positioning on the breathing pattern of preterm infants. *Archives of Disease in Childhood,* 1992, vol. 67 (3), 312-314
- Brief Introduction to Bioimpedance. **HOLDER, D.S.** Electrical Impedance Tomography- Methods, History and Applications. Institute of Physics Publishing, 2005, 411-422
- Introduction to Biomedical Electrical Impedance Tomography. **HOLDER, D.S.** Electrical Impedance Tomography- Methods, History and Applications. IOP Publishing Ltd, 2005, 423-450
- **IDER, Y.Z. et al.** Determination of the boundary of an object inserted into a water-filled cylinder. *Clinical Physics and Physiological Measurement,* 1992, vol. 13 (A), 151-154, http://iopscience.iop.org/0143-0815/13/A/029
- **ISAACSON, D. ; CHENEY, M. ; NEWELL, J.C.** Electrical impedance tomography. *SIAM Review,* 1999, vol. 41 (1), 85-101, http://www.ncbi.nlm.nih.gov/pubmed/1587116
- **KAO, T.J. et al.** A 3D reconstruction algorithm for EIT using a handheld probe for breast cancer detection. *Physiological measurement,* 2006, vol. 27 (5), S1, http://iopscience.iop.Org/0967-3334/27/5/S01
- Determination of object boundary shape for electrical impedance tomography. **KIBER, M.A.** Dept. of Medical Physics and Clinical Engineering. University of Sheffield, 1991
- **KHOR, J. M. et al.** Development of a Sensor Network for Dynamic Boundary Measurement in Neonatal EIT. *IFMBE Proceedings 25/II,* 2009, 386-389
- **KOLEHMAINEN, V. ; LASSAS, M. ; OLA, P.** Electrical impedance tomography problem with inaccurately known boundary and contact impedances. *Medical Imaging, IEEE Transactions on,* 2008, vol. 27 (10), 1404-1414, http://ieeexplore.ieee.org/xpls/abs_all.jsp?arnumber=4471919
- **LI, J.** A method of reducing the error caused by boundary shape and electrode positions in electrical impedance tomography. *Physiological Measurement,* 1994, vol. 15 (2A), A169-74, http://iopscience.iop.org/0967-3334/15/2A/022
- **LIONHEART, W.R.B.** Boundary shape and electrical impedance tomography. *Inverse problems,* 1998, vol. 14 (1), 139-147, http://iopscience.iop.org/0266-5611/14/1/012
- **MAKI, D.D.** Recent Advances in Pulmonary Imaging. *Chest,* 1999, vol. 116 (5), 1388-1402, http://www.chestjournal.org/cgi/doi/10.1378/chest.116.5.1388
- **MARVEN, S.S. et al.** Reproducibility of electrical impedance tomographic spectroscopy (EITS) parametric images of neonatal lungs. *Physiological measurement,* 1996, vol. 17 (4), A205-12, http://www.ncbi.nlm.nih.gov/pubmed/9001619
- **MURPHY, D. et al.** Impedance imaging in the newborn. *Clinical physics and physiological measurement,* 1987, vol. 8 (A), 131-40, http://www.ncbi.nlm.nih.gov/pubmed/3568562
- **NISSINEN, A. et al.** Compensation of errors due to discretization, domain truncation and unknown contact impedances in electrical impedance tomography. *Measurement Science and Technology,* 2009, vol. 20 (10), 105504, http://stacks.iop.org/0957-0233/20/i=10/a=105504?key=crossref.02b35dbd0327dcae484cbb4e8a9e280f
- **OTTEN, D.M. ; ONIK, G. ; RUBINSKY, B.** Distributed network imaging and electrical impedance tomography of minimally invasive surgery. *Technology in cancer research & treatment,* 2004, vol. 3 (2), 125-34, http://www.ncbi.nlm.nih.gov/pubmed/15059018
- **RAHAL, M. et al.** A comparison study of electrodes for neonate electrical impedance tomography. *Physiol. Meas.,* 2009, vol. 30 (2009), S73-S84
- **RIU, P.J. ; ROSELL, J. ; PALLÁS-ARENY, R.** In vivo static imaging for the real and the reactive parts in electrical impedance tomography using multifrequency techniques. IEEE, 1992, vol. 5, 1706-1707
- Chest and Lungs. **SEIDEL, H.M. et al.** Mosby's Guide to Physical Examination. 2009, 332-377
- **SEYDNEJAD, S.R. ; FAHIMI, H.** A new method for finding the exact electrode location and body perimeter used for EIT. *Proceedings of 16th Annual International Conference of the IEEE Engineering in Medicine and Biology Society,* 1994, 541-542, http://ieeexplore.ieee.org/lpdocs/epic03/wrapper.htm?arnumber=411913
- **SMALLWOOD, R.H. et al.** A comparison of neonatal and adult lung impedances derived from EIT images. *Physiological measurement,* 1999, vol. 20 (4), 401-13, http://www.ncbi.nlm.nih.gov/pubmed/10593233

- Simultaneous reconstruction of the boundary shape and conductivity in 3D electrical impedance tomography. **SOLEIMANI, M. ; ABASCAL, J. ; LIONHEART, W.R.B.** Proceedings of the XII International Conference on Electrical Bio-Impedance and Electrical Impedance Tomography. University of Bath, 20 June 2004, 475-478
- **SOMERSALO, E. et al.** Existence and Uniqueness for Electrode Models for Electric Current Computed Tomography. *SIAM J. Appl. Math.,* 1992, vol. 52 (4), 1023-1040
- **STARCK, J.R. et al.** An inexpensive sensor for measuring surface geometry. *Medical engineering & physics,* 1999, vol. 21 (10), 725-9, http://www.ncbi.nlm.nih.gov/pubmed/10717552
- **STOCKS, J. et al.** Plethysmographic measurements of lung volume and airway resistance. *European Respiratory Journal,* 2001, vol. 17 (2), 302-312
- **TAKTAK, A. et al.** Practical factors in neonatal lung imaging using electrical impedance tomography. *Medical and Biological Engineering and Computing,* 1995, vol. 33 (2), 202-205, http://www.springerlink.com/index/V1Q3611344R37310.pdf
- **TAKTAK, A. et al.** Feasibility of neonatal lung imaging using electrical impedance tomography. *Early human development,* 1996, vol. 44 (2), 131-138, http://linkinghub.elsevier.com/retrieve/pii/0378378295017003
- **TANG, M. et al.** Effects of incompatible boundary information in EIT on the convergence behavior of an iterative algorithm. *IEEE transactions on medical imaging,* 2002, vol. 21 (6), 620-8, http://www.ncbi.nlm.nih.gov/pubmed/12166858
- **TIZZARD, A. et al.** Generating accurate finite element meshes for the forward model of the human head in EIT. *Physiological Measurement,* 2005, vol. 26 (2), S251, http://iopscience.iop.org/0967-3334/26/2/024
- **TIZZARD, A. ; BAYFORD, R.H.** Improving the finite element forward model of the human head by warping using elastic deformation. *Physiological Measurement,* 2007, vol. 28 (7), S163-S182
- **ZHU, Q.S. et al.** Development of a real-time adaptive current tomograph. *Physiological measurement,* 1994, vol. 15 (2), A37-43, http://www.ncbi.nlm.nih.gov/pubmed/8087048
- **ZLOCHIVER, S. et al.** A portable bio-impedance system for monitoring lung resistivity. *Medical engineering & physics,* 2007, vol. 29 (1), 93-100, http://www.ncbi.nlm.nih.gov/pubmed/16546432